# EUROPEAN PATENT SPECIFICATION

(11) **EP 3 388 522 B1**
(45) Date of publication and mention of the grant of the patent: **12.08.2020**
(21) Application number: 16873339.2
(22) Date of filing: 07.12.2016
(51) Int. Cl.: C12N 15/861, C07K 14/47, C07K 14/535, C12N 5/0784, C12N 15/113, A61K 48/00, C12N 15/62, A61P 35/00, A61P 39/00, A61K 31/7105, C12N 15/85, A61K 39/00

(54) **ANTITUMOR COMPOSITION COMPRISING GM-CSF GENE, FLT3L-TRAIL FUSION GENE, SHRNA INHIBITING TGF- EXPRESSION, AND SHRNA INHIBITING HSP EXPRESSION**
ANTITUMORZUSAMMENSETZUNG MIT GM-CSF-GEN, FLT3L-TRAIL-FUSIONSGEN, SHRNA-HEMMENDER TGF-EXPRESSION UND SHRNA-HEMMENDER HSP-EXPRESSION
COMPOSITION ANTITUMORALE CONTENANT UN GÈNE GM-CSF, UN GÈNE HYBRIDE FLT3L-TRAIL, UN ARNSH INHIBANT L'EXPRESSION DE TGF- ET UN ARNSH INHIBANT L'EXPRESSION DE HSP

(30) Priority: 08.12.2015 KR 20150173858
(43) Date of publication of application: 17.10.2018
(73) Proprietor: University-Industry Foundation, Yonsei University, Seoul 03722 (KR)
(72) Inventor: SONG, Jae Jin, Seodaemun-gu Seoul 03722 (KR); HAN, Zhe Zhu, Seodaemun-gu Seoul 03722 (KR); KANG, Dong Xu, Seodaemun-gu Seoul 03722 (KR); XU, Rong, Seodaemun-gu Seoul 03722 (KR); CHOI, Hye Jin, Seodaemun-Gu Seoul 03743 (KR)
(74) Representative: Schiweck Weinzierl Koch Patentanwälte Partnerschaft mbB
(86) International application number: PCT/KR2016/014325
(87) International publication number: WO 2017/099474

(56) References cited:
- WO-A1-2015/152609
- KR-A- 20120 099 503
- KR-A- 20130 123 244
- KR-A- 20150 113 906
- KR-A- 20150 113 906
- US-A1- 2006 292 166
- US-A1- 2006 292 166
- US-A1- 2014 351 961

<110> Industry-Academic Cooperation Foundation, Yonsei University
<120> Composition including GM-CSF gene; Flt3L-TRAIL fusion gene; shRNA downregulating TGF-beta; and shRNA downregulating HSP for treatment of malignant tumor
<130> X16U18C0265
<150> KR 10-2015-0173858
   <151> 2015-12-08
<160> 9
<170> KopatentIn 2.0
<210> 1
   <211> 432
   <212> DNA
   <213> Mus musculus
<400> 1
   atgtacagga tgcaactcct gtcttgcatt gcactaagtc ttgcacttgt cacgaattcg      60
   cccacccgct cacccatcac tgtcacccgg ccttggaagc atgtagaggc catcaaagaa     120
   gccctgaacc tcctggatga catgcctgtc acgttgaatg aagaggtaga agtcgtctct     180
   aacgagttct ccttcaagaa gctaacatgt gtgcagaccc gcctgaagat attcgagcag     240
   ggtctacggg gcaatttcac caaactcaag ggcgccttga acatgacagc cagctactac     300
   cagacatact gccccccaac tccggaaacg gactgtgaaa cacaagttac cacctatgcg     360
   gatttcatag acagccttaa aacctttctg actgatatcc cctttgaatg caaaaaacca     420
   ggccaaaaat ga                                                         432
<210> 2
   <211> 435
   <212> DNA
   <213> Homo sapiens
<400> 2
   atgtggctgc agagcctgct gctcttgggc actgtggcct gcagcatctc tgcacccgcc      60
   cgctcgccca gccccagcac gcagccctgg gagcatgtga atgccatcca ggaggcccgg     120
   cgtctcctga acctgagtag agacactgct gctgagatga atgaaacagt agaagtcatc     180
   tcagaaatgt ttgacctcca ggagccgacc tgcctacaga cccgcctgga gctgtacaag     240
   cagggcctgc ggggcagcct caccaagctc aagggcccct tgaccatgat ggccagccac     300
   tacaagcagc actgccctcc aaccccggaa acttcctgtg caacccagat tatcaccttt     360
   gaaagtttca aagagaacct gaaggacttt ctgcttgtca tcccctttga ctgctgggag     420
   ccagtccagg agtga                                                      435
<210> 3
   <211> 1221
   <212> DNA
   <213> Artificial Sequence
<220>
   <223> Flt3L.TRAIL fusion gene
<400> 3
   atgacagtgc tggcgccagc ctggagccca acaacctatc tcctcctgct gctgctgctg 60
   agctcgggac tcagtgggac ccaggactgc tccttccaac acagccccat ctcctccgac 120
   ttcgctgtca aatccgtgga gctgtctgac tacctgcttc aagattaccc agtcaccgtg 180
   gcctccaacc tgcaggacga ggagctctgc gggggcctct ggcggctggt cctggcacag 240
   cgctggatgg agcggctcaa gactgtcgct gggtccaaga tgcaaggctt gctggagcgc 300
   gtgaacacgg agatacactt tgtcaccaaa tgtgcctttc agcccccccc cagctgtctt 360
   cgcttcgtcc agaccaacat ctcccgcctc ctgcaggaga cctccgagca gctggtggcg 420
   ctgaagccct ggatcactcg ccagaacttc tcccggtgcc tggagctgca gtgtcagccc 480
   gactcctcaa ccctgccacc cccatggagt ccccggcccc tggaggccac agccccgaca 540
   gccccggcta gcagaatgaa gcagatcgag gacaaaattg aggaaatcct gtccaaaatt 600
   taccacatcg agaacgagat cgcccggatt aagaaactca ttggcgagag ggaattcacc 660
   tctgaggaaa ccatttctac agttcaagaa aagcaacaaa atatttctcc cctagtgaga 720
   gaaagaggtc ctcagagagt agcagctcac ataactggga ccagaggaag aagcaacaca 780
   ttgtcttctc caaactccaa gaatgaaaag gctctgggcc gcaaaataaa ctcctgggaa 840
   tcatcaagga gtgggcattc attcctgagc aacttgcact tgaggaatgg tgaactggtc 900
   atccatgaaa aagggtttta ctacatctat tcccaaacat actttcgatt tcaggaggaa 960
   ataaaagaaa acacaaagaa cgacaaacaa atggtccaat atatttacaa atacacaagt 1020
   tatcctgacc ctatattgtt gatgaaaagt gctagaaata gttgttggtc taaagatgca 1080
   gaatatggac tctattccat ctatcaaggg ggaatatttg agcttaagga aaatgacaga 1140
   atttttgttt ctgtaacaaa tgagcacttg atagacatgg accatgaagc cagttttttc 1200
   ggggcctttt tagttggcta a 1221
<210> 4
   <211> 54
   <212> DNA
   <213> Artificial Sequence
<220>
   <223> mouse shTGF-beta1
<400> 4
   ccctctacaa ccaacacaac ccgggtctcc ccgggttgtg ttggttgtag aggg 54

## Description

### [Technical Field]

The present invention relates to an antitumor composition comprising a GM-CSF gene, a Flt3L-TRAIL fusion gene, shRNA inhibiting TGF-β expression, and shRNA inhibiting HSP expression.

### [Background Art]

A granulocyte-macrophage stimulating factor (GM-CSF) acts in various ways, and first serves to gather antigen-presenting cells such as natural killer cells or dendritic cells. In addition, GM-CSF has been known to stimulate dendritic cells around a tumor and thus increase the expression of a costimulatory molecule, so that CD4+ and CD8+ T cells reinforce immune responses and to be involved in regulation of expression of molecules consisting of MHC class II in a primary monocyte, in addition to the promotion of dendritic cell differentiation [J. Immunol. 171: 2374 by Hornell et al., 2003. Regulation of the class II MHC pathway in primary human monocytes by granulocyte-macrophage colony-stimulating factor]. In addition, it has been known that, when GM-CSF is expressed in a tumor, APCs are collected around the tumor, and anticancer immune responses are strongly induced through effective treatment of tumor antigens [Cancer Immunol. Immunother. 53: 17 by Pan et al., 2004 In situ recruitment of antigen-presenting cells by intratumoral GM-CSF gene delivery.].

By coexpressing Flt3L and TRAIL, Flt3L-TRAIL has dual functions, for example, potent stimulation of the proliferation of dendritic cells from DC progenitor cells to DCs (Flt3L), and induction of apoptosis of cancer cells (TRAIL). As DCs acquire antigens derived from an apoptotic tumor body, attractive vaccination against tumors can be achieved. This approach can trigger a wide-range list of CD4⁺ and CD8⁺ T cell responses without a need to identify tumor-specific antigenic epitopes. Therefore, it is applicable to all cancer patients regardless of an HLA haplotype [Mol. Ther. 3: 368 by Wu et al., 2001. Regression of human mammary adenocarcinoma by systemic administration of a recombinant gene encoding the hFlex-TRAIL fusion protein.; Mol. Ther. 9: 674 by Wu and Hui, 2004. Induction of potent TRAIL-mediated tumoricidal activity by hFLEX/furin/TRAIL recombinant DNA construct].

There are the patents on shRNA inhibiting TGF-β1 or TGF-β2 and HSP27 expression received by the inventors [Korean Patent Nos. 1286053, 1420564, and 1374585].

It has been well known that the immunological action of HSP27 increases tumor necrosis and a memory response, which are mediated by CD8⁺ T cells, by increasing proteosomic activity due to silencing of HSP27.

In this regard, there has not been a study on the application of shRNA simultaneously inhibiting TGF-β and HSP expression to antitumor treatment by preparing an organic complex using both GM-CSF and Flt3L-TRAIL.

### [Disclosure]

### [Technical Problem]

Therefore, the inventors had attempted to develop an anti-tumor gene composition having maximized anti-tumor activity by simultaneously increasing a tumor-specific cytotoxic action and immune activity and tumor immunogenicity, and thus confirmed that, when the gene composition is transduced into target cells using a gene delivery system for coexpression of shRNA inhibiting TGF-β expression (shTGF-β) and shRNA inhibiting HSP expression (shHSP), compared to when each gene is expressed, or even when GM-CSF or Flt3L-TRAIL is expressed, an antitumor effect is more highly improved, and also confirmed that, when the gene composition is transduced into a target cell using a gene delivery system for coexpressing a GM-CSF gene, a Flt3L-TRAIL fusion gene, shTGF-β and shHSP, compared to when each gene is expressed, and particularly, when shTGF-β and shHSP are expressed, a higher antitumor effect is exhibited. Therefore, the present invention was completed.

### [Technical Solution]

For this reason, the present invention is directed to providing a GM-CSF, Flt3L-TRAIL, shTGF-β and shHSP-coexpressing gene delivery system which includes a GM-CSF gene, a Flt3L-TRAIL fusion gene, shRNA inhibiting TGF-β expression (shTGF-β) and shRNA inhibiting HSP expression (shHSP), and an antitumor composition including the same.

In addition, the present invention is directed to providing a shTGF-β and shHSP-coexpressing gene delivery system, which includes shRNA inhibiting TGF-β expression (shTGF-β) and shRNA inhibiting HSP expression (shHSP), and an antitumor composition including the same.

As a means for solving the above problems, the present invention provides a GM-CSF, Flt3L-TRAIL, shTGF-β and shHSP-coexpressing gene delivery system which includes a GM-CSF gene, a Flt3L-TRAIL fusion gene, shRNA inhibiting TGF-β expression (shTGF-β) and shRNA inhibiting HSP expression (shHSP).

As another means for solving the above problems, the present invention provides an antitumor composition which includes a GM-CSF gene, a Flt3L-TRAIL fusion gene, shRNA inhibiting TGF-β expression (shTGF-β) and shRNA inhibiting HSP expression (shHSP).

As still another means for solving the above problems, the present invention provides a shTGF-β and shHSP-coexpressing gene delivery system, which includes shRNA inhibiting TGF-β expression (shTGF-β) and shRNA inhibiting HSP expression (shHSP).

As yet another means for solving the above problems, the present invention provides an antitumor composition, which includes shRNA inhibiting TGF-β expression (shTGF-β) and shRNA inhibiting HSP expression (shHSP).

### [Advantageous Effects]

In the present invention, as TGF-β expression is inhibited using shRNA-mediated RNA interference acting on a tumor-associated gene of TGF-β, which is a protein causing the onset of a disease, to restrict a factor inducing immune tolerance and induce an immune boosting response induced by GM-CSF, an antitumor effect is enhanced, Flt3L-TRAIL is expressed, and also TGF-β and HSP expression is simultaneously inhibited, resulting in considerable enhancement in an antitumor effect in a cancer disease animal model. Binding of a total of four individual genes including these fusion genes, rather than a random combination of genes simply having an antitumor function, was made for these genes to be closely and organically associated with each other.

### [Description of Drawings]

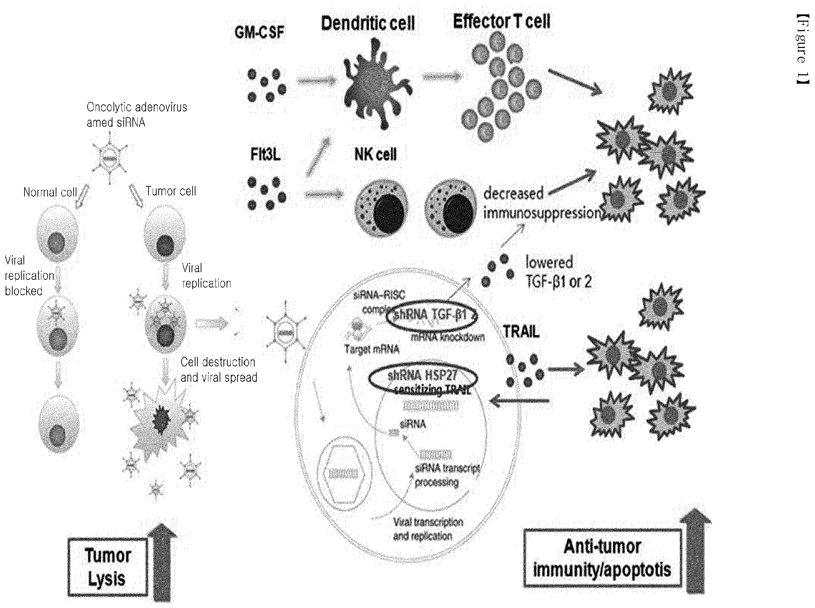

FIG. 1 illustrates individual modes of the action of four genes which are included in a recombinant adenovirus vector according to the present invention;
FIG. 2 illustrates that four genes of the present invention have an organic and cooperative relation, rather than separate actions thereof, to exhibit an anticancer action;
FIG. 3 shows two types of recombinant adenovirus vectors and four types of recombinant adenovirus vectors (YSC-02 and YSC-01) according to the present invention;
FIG. 4A shows that Flt3L-TRAIL is inserted into an Adlox vector with SalI/BamHI;
FIG. 4B shows that Flt3L-TRAIL is inserted into a pIRES vector with Xbal and MfeI;
FIG. 4C shows that Flt3L-TRAIL is inserted into a pVAX1-3484-CMVp-ΔE1B(-E1R) shuttle vector with PmeI;
FIG. 4D shows an oncolytic adenovirus expressing human Flt3L-TRAIL, which is prepared by homologous recombination of dl324-BstBI as a backbone and pVAX1-3484-CMVp-ΔE1B-Flt3L-TRAIL as a shuttle vector;
FIG. 5A shows an oncolytic adenovirus expressing human shTGF-β, which is prepared by homologous recombination of dl324-BstBI-U6-shTGF-β1 as a backbone and pVAX1-3484-CMVp-ΔE1B as a shuttle vector;
FIG. 5B shows an oncolytic adenovirus expressing human shTGF-β, which is prepared by homologous recombination of dl324-BstBI-U6-shTGF-β2 as a backbone and pVAX1-3484-CMVp-ΔE1B as a shuttle vector;
FIG. 6 shows an oncolytic adenovirus expressing human shHSP27, which is prepared by homologous recombination of dl324-BstBI-H1-shHSP27 as a backbone and pVAX1-3484-CMVp-ΔE1B as a shuttle vector;
FIG. 7 shows an effect of reducing HSP25 expression by transfecting a BNL-HSP25 cell line with HSP25 shRNA to confirm an HSP25 shRNA effect from pSP72-H1-mshHSP25-1, -2 or -3;
FIG. 8 shows an oncolytic adenovirus expressing murine shHSP25, which is prepared by homologous recombination of dl324-IX as a backbone and pSP72-shHSP25-2 as a shuttle vector;
FIG. 9 shows an oncolytic adenovirus expressing murine shHSP25, which is prepared by homologous recombination of dl324-IX as a backbone and pSP72-shHSP25-3 as a shuttle vector;
FIG. 10 shows an effect of reducing expression from each adenovirus that expresses HSP25 shRNA of FIG. 8 or 9;
FIG. 11 shows insertion of a Flt3L-TRAIL gene into pIRES-GM-CSF, as confirmed with a restriction enzyme [Lane 1: negative DNA; Lane 2, 3: Flt3L-TRAIL inserted];
FIG. 12A shows insertion of human GM-CSF-Flt3L-TRAIL genes into pVAX1-3484-CMVp-ΔE1B-E1R, as confirmed with a restriction enzyme;
FIG. 12B shows insertion of murine GM-CSF-Flt3L-TRAIL genes into pVAX1-3484-CMVp-ΔE1B-E1R, as confirmed by a restriction enzyme [A subcloned product was cleaved with PmeI to confirm the insert and an increased vector size];
FIG. 13 shows homologous recombination of GM-CSF and Flt3L-TRAIL gene-inserted, tumor-selective replication-competent adenovirus DNA, as confirmed with a restriction enzyme, in which FIG. 13A shows human GM-CSF [all of Lanes 1,2,3 and 4 are positive], and FIG. 13B shows murine GM-CSF [Lanes 1 and 4 are positive];

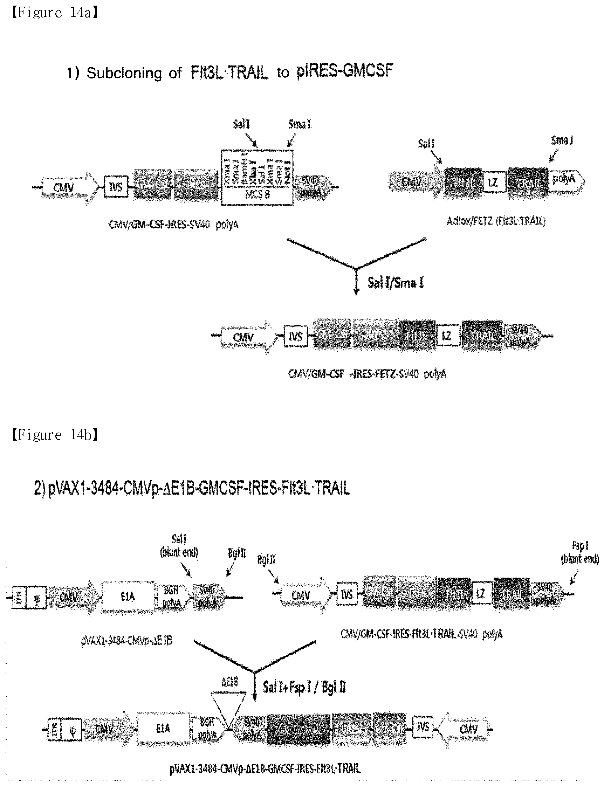

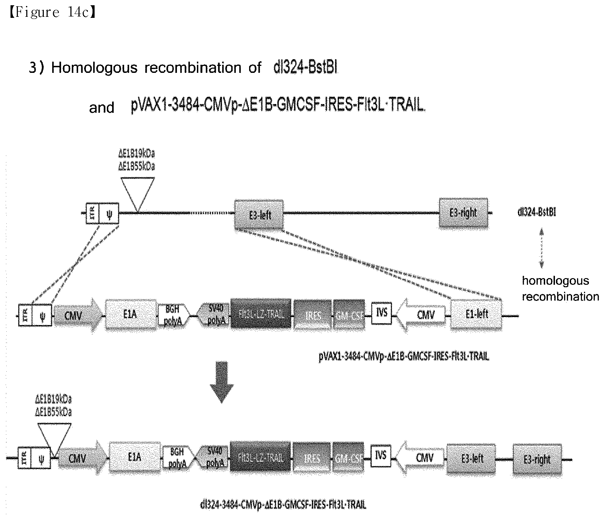

FIG. 14 shows a process of subcloning GM-CSF and Flt3L-TRAIL genes, and a process of homologous recombination for manufacturing a shuttle vector and producing a replicable adenovirus;
FIG. 15A shows an ELISA result confirming that GM-CSF and TRAIL proteins are expressed in human GM-CSF and human Flt3L-TRAIL gene-inserted, tumor-selective replication-competent adenoviruses;
FIG. 15B shows an ELISA result confirming that GM-CSF and TRAIL proteins are expressed in murine GM-CSF and human Flt3L-TRAIL gene-inserted, tumor-selective replication-competent adenoviruses;
FIG. 16A shows apoptosis induced by TRAIL in GM-CSF and Flt3L-TRAIL gene-inserted, tumor-selective replication-competent adenoviruses, as confirmed by poly(ADP-ribose)polymerase (PARP) cleavage;
FIG. 16B shows that Flt3L is expressed in GM-CSF and Flt3L-TRAIL gene-inserted, tumor-selective replication-competent adenoviruses;
FIG. 17 shows a process of manufacturing a pSP72ΔE3-U6-shTGFβ2-H1-shHSP27 or pSP72ΔE3-H1-shHSP27-U6-shTGFβ1 shuttle vector;
FIG. 18 shows homologous recombination of dl324-IX as a backbone and pSP72-ΔE3-U6-shTGFβ2-H1-shHSP27 as a shuttle vector;
FIG. 19 shows homologous recombination of dl324-IX as a backbone and pSP72-ΔE3-H1-shHSP27-U6-shTGFβ1 as a shuttle vector;
FIG. 20 shows homologous recombination of dl324-BstBI as a backbone and pSP72-ΔE3-H1-shHSP27-U6-shTGFβ1 as a shuttle vector;
FIG. 21 shows homologous recombination of dl324-BstBI as a backbone and pSP72-ΔE3-U6-shTGFβ2-H1-shHSP27 as a shuttle vector;
FIG. 22 shows homologous recombination of dl324-BstBI-H1-shHSP27-U6-shTGFβ1 as a backbone and pVAX1-3484-CMVp-ΔE1B as a shuttle vector;
FIG. 23 shows homologous recombination of dl324-BstBI-U6-shTGFβ2-H1-shHSP27 as a backbone and pVAX1-3484-CMVp-ΔE1B as a shuttle vector;
FIG. 24A shows clones in which a H1-mshHSP25 gene is inserted into pSP72ΔE3-U6-mshTGF-β1 ;
FIG. 24B shows homologous recombination of dl324-IX as a backbone and pSP72-H1-shHSP25-U6-mshTGFβ1 as a shuttle vector;
FIG. 25A shows homologous recombination of dl324-BstBI as a backbone and pSP72-H1-shHSP25-U6-mshTGFβ1 as a shuttle vector;
FIG. 25B shows homologous recombination of dl324-BstBI-H1-shHSP25-U6-mshTGFβ1 as a backbone and pVAX1-3484-CMVp-ΔE1B as a shuttle vector;
FIG. 26 shows homologous recombination of dl324-BstBI-H1-shTGF-β1 as a backbone and pVAX1-3484-CMVp-ΔE1B-GMCSF-IRES-Flt3L-TRAIL as a shuttle vector to confirm the manufacture of an oncolytic adenovirus coexpressing human GM-CSF, Flt3L-TRAIL and shTGF-β1;
FIG. 27 shows homologous recombination of dl324-BstBI-U6-mshTGFβ1 as a backbone and pVAX1-3484-CMVp-ΔE1B-mGMCSF-IRES-Flt3L-TRAIL as a shuttle vector to confirm the manufacture of an oncolytic adenovirus coexpressing murine GM-CSF, Flt3L-TRAIL and mTGFβ1;
FIG. 28 shows homologous recombination of dl324-BstBI-mshHSP27 as a backbone and pVAX1-3484-CMVp-ΔE1B-GMCSF-IRES-Flt3L-TRAIL as a shuttle vector to confirm the manufacture of an oncolytic adenovirus coexpressing human GM-CSF, Flt3L-TRAIL and shHSP27;
FIG. 29 shows homologous recombination of dl324-BstBI-shHSP25 (mouse-derived) as a backbone and pVAX1-3484-CMVp-ΔE1B-mGMCSF-IRES-Flt3L-TRAIL as a shuttle vector to confirm the manufacture of an oncolytic adenovirus coexpressing murine GM-CSF, Flt3L-TRAIL and shHSP25;
FIG. 30 shows homologous recombination of dl324-BstBI-shHSP27-shTGFβ1 as a backbone and pVAX1-3484-CMVp-ΔE1B-hGMCSF as a shuttle vector to confirm the manufacture of an oncolytic adenovirus coexpressing human GM-CSF, shTGFβ1 and shHSP27;
FIG. 31 shows homologous recombination of dl324-BstBI-U6-shTGFβ2-H1-shHSP27 as a backbone and pVAX1-3484-CMVp-ΔE1B-GM-CSF as a shuttle vector to confirm the manufacture of an oncolytic adenovirus coexpressing human GM-CSF, shTGFβ2 and shHSP27;
FIG. 32 shows homologous recombination of dl324-BstBI-H1-mshHSP25-U6-mshTGFβ1 as a backbone and pVAX1-3484-CMVp-ΔE1B-mGM-CSF as a shuttle vector to confirm the manufacture of an oncolytic adenovirus coexpressing murine GMCSF, shTGFβ1 and shHSP25;
FIG. 33 shows homologous recombination of dl324-BstBI-H1-shHSP27-U6-shTGF-β1 as a backbone and pVAX1-3484-CMVp-ΔE1B-Flt3L-TRAIL as a shuttle vector to confirm the manufacture of an oncolytic adenovirus coexpressing Flt3L-TRAIL, shHSP27 and shTGFβ1;
FIG. 34 shows homologous recombination of dl324-BstBI-U6-shTGFβ2-H1-shHSP27 as a backbone and pVAX1-3484-CMVp-ΔE1B-Flt3L-TRAIL as a shuttle vector to confirm the manufacture of an oncolytic adenovirus coexpressing Flt3L-TRAIL, shHSP27 and shTGFβ2;
FIG. 35 shows homologous recombination of dl324-BstBI-H1-mshHSP25-U6-mshTGF-β1 as a backbone and pVAX1-3484-CMVp-ΔE1B-Flt3L-TRAIL as a shuttle vector to confirm the manufacture of an oncolytic adenovirus coexpressing Flt3L-TRAIL, shHSP25 and shTGFβ1;
FIG. 36 shows homologous recombination of dl324-BstBI-ΔE3-U6-shTGFβ2-H1-shHSP27 and pVAX1-3484-CMV-ΔE1B-GM-CSF-IRES-Flt3L-TRAIL as a shuttle vector to confirm the manufacture of a GM-CSF, Flt3L-TRAIL, shTGF-β2 and shHSP27-loaded oncolytic adenovirus (YSC-01);
FIG. 37 shows homologous recombination of dl324-BstBI-ΔE3-H1-shHSP27-U6-shTGF-β1 and pVAX1-3484-CMV-ΔE1B-GM-CSF-IRES-Flt3L-TRAIL as a shuttle vector to confirm the manufacture of a GM-CSF, Flt3L-TRAIL, shTGF-β1 and shHSP27-loaded oncolytic adenovirus (YSC-02);
FIG. 38 shows homologous recombination of dl324-BstBI-ΔE3-U6-shTGFβ2-H1-shHSP27 as a backbone and pVAX1-3484-CMV-ΔE1B-GM-CSF-IRES-Flt3L-TRAIL as a shuttle vector;
FIG. 39 shows homologous recombination of dl324-BstBI-ΔE3-H1-shHSP27-U6-shTGFβ1 as a backbone and pVAX1-3484-CMV-ΔE1B-GM-CSF-IRES-Flt3LTRAIL as a shuttle vector;
FIG. 40 shows homologous recombination (mYSC-02) of dl324-BstBI-ΔE3-H1-shHSP25-U6-mshTGFβ1 as a backbone and pVAX1-3484-CMV-ΔE1B-GM-CSF-IRES-Flt3L-TRAIL as a shuttle vector;
FIG. 41 shows that genes loaded in a virus are normally expressed when murine hepatocellular carcinoma cell line-derived BNL-CAR-E1B55K-HSP25 cells are infected with mYSC-02;
FIG. 42 shows an antitumor effect caused by a tumor-selective replication-competent virus expressing each or all of shTGF-β1 and shHSP27, as confirmed by a nude mouse animal test;
FIG. 43 shows the difference in survival potentials according to the isotype of TGF-β, as confirmed through a clonogenic assay performed for various cancer cell lines;
FIG. 44A shows effects on cell survival signals and SAPK-associated signals when intracellular TGF-β1 levels are decreased in U251N, A549, Huh7 and A375 cancer cell lines;
FIG. 44B shows effects on cell survival signals and SAPK-associated signals when intracellular TGF-β1 levels are decreased in MiaPaCa-2, HPAC, Aspc-1 and Capan-1 cancer cell lines;
FIG. 45A shows that ROS productivity when TGF-β1 or TGF-β2 is decreased in an MDA-MB231-Her2 cancer cell line is measured by fluorescence intensity generated by DCF-DA oxidation;
FIG. 45B shows effects on cell survival signals and SAPK-related signals when TGF-β1 or TGF-β2 is decreased in an MDA-MB231-Her2 cancer cell line;
FIG. 45C shows that ROS productivity when TGF-β1 or TGF-β2 is decreased in A549, A375, Hun7 and U251N cancer cell lines is measured by fluorescence intensity generated by DCF-DA oxidation;
FIG. 46 shows effects on cell survival signals and SAPK-related signals when TGF-β1 or TGF-β2 and HSP27 are decreased in various cancer cell lines;
FIG. 47 shows a decrease in various marker signals associated with tumor progression when HSP27 is decreased in various cancer cell lines;
FIG. 48 shows various marker signals associated with tumor progression are more clearly decreased when TGF-β1 or TGF-β2 and HSP27 are simultaneously decreased, compared to when HSP27 is only decreased in various cancer cell lines;
FIG. 49 shows a survival rate is decreased when TGF-β1 or TGF-β2 and HSP27 are simultaneously decreased, compared to when HSP27 is only decreased in various cancer cell lines, as confirmed by a clonogenic assay;
FIG. 50 shows that the increase in TRAIL receptors and decrease in TRAIL resistance-associated CDK9 are exhibited when TGF-β1 or TGF-β2 and HSP27 are simultaneously decreased, compared to when HSP27 is only decreased in various cancer cell lines;
FIG. 51A shows effects on cell survival signals and SAPK-associated signals when TGF-β1 and HSP27 are simultaneously decreased, and TRAIL expression is induced in breast cancer, glioma and melanoma cell lines;
FIG. 51B shows effects on cell survival signals and SAPK-associated signals when TGF-β1 and HSP27 are simultaneously decreased, and TRAIL expression is induced in pancreatic cancer and hepatocellular carcinoma cell lines;
FIG. 52 shows that genes introduced through base sequence analyses for viral DNAs of YSC-01 and YSC-02 are normally inserted [GM-CSF has InvivoGen pORF-GM-CSF-derived entire amino acid residues, Flt3L has amino acid residues 1 to 181, and TRAIL has amino acid residues 95 to 281];
FIG. 52A shows that a GM-CSF base sequence is inserted into YSC-01;
FIG. 52B shows that a Flt3L base sequence (corresponding to amino acids 1 to 181) is identified in Flt3L-TRAIL of YSC-01;
FIG. 52C shows that a TRAIL base sequence (corresponding to amino acids 95 to 281) is identified in Flt3L-TRAIL of YSC-01 [the right TCT on the third row is TCC, but the amino acids are the same as serine];
FIG. 52D shows that a shTGF-β2 base sequence is identified in shTGF-β2 of YSC-01;
FIG. 52E shows that a shHSP27 base sequence is identified in shHSP27 of YSC-01;
FIG. 52F shows that a GM-CSF base sequence is inserted into YSC-02;
FIG. 52G shows that a Flt3L base sequence (corresponding to amino acids 1 to 181) is identified in Flt3L-TRAIL of YSC-02;
FIG. 52H shows that a TRAIL base sequence (corresponding to amino acids 95 to 281) is identified in Flt3L-TRAIL of YSC-02 [the right TCT on the third row is TCC, but the amino acids are the same as serine];
FIG. 52I shows that a shHSP27 base sequence is identified in shHSP27 of YSC-02;
FIG. 52J shows that a shTGF-β1 base sequence is identified in shTGF-β1 of YSC-02;
FIG. 53 shows that four genes introduced when pancreatic cancer cell lines are infected with YSC-01 and YSC-02 at different MOIs are normally expressed or degraded in expression by shRNA;
FIG. 53A shows GM-CSF and TRAIL secretion by YSC-01 infection;
FIG. 53B shows inhibition of TGF-β2 mRNA expression by YSC-01 infection, and also shows PARP cleavage, Flt3L expression and inhibition of HSP27 expression;
FIG. 53C shows GM-CSF and TRAIL secretion by YSC-02 infection;FIG. 53D shows inhibition of TGF-β2 mRNA expression by YSC-02 infection, and also shows PARP cleavage, Flt3L expression and inhibition of HSP27 expression;
FIG. 54A shows that survival potential is further degraded in several types of cancer cell lines including a p53 mutant type, compared to an oncolytic adenovirus expressing GM-CSF and Flt3L-TRAIL, by YSC-01 or YSC-02, as confirmed by a clonogenic assay;
FIG. 54B shows that survival potential is further degraded in a p53 wild type cancer cell line, compared to an oncolytic adenovirus expressing GM-CSF and Flt3L-TRAIL, by YSC-01 or YSC-02, as confirmed by a clonogenic assay (left), and that there is no significant difference in survival potential of normal cells, compared to that of YSC-01 or YSC-02, an oncolytic adenovirus expressing GM-CSF and Flt3L-TRAIL, or a control oncolytic adenovirus, as confirmed by a clonogenic assay (right);
FIG. 55A shows tumor selectivity, induction of a decrease in survival rate and an increase in oncolytic potential by confirming survival signals and TRAIL-related signals according to an increase in MOI of YSC-02 in normal cells and several types of cancer cell lines including a p53 mutant type;
FIG. 55B shows tumor selectivity, induction of a decrease in survival rate and an increase in oncolytic potential by confirming a survival signal and a TRAIL-related signal according to an increase in MOI of YSC-02 in two types of cancer cell lines including a p53 wild type;
FIG. 56 shows that YSC-02 exhibits a relatively higher oncolytic potential in some types of cancer cell lines including normal cells through an oncolytic assay performed on YSC-01 or YSC-02;
FIG. 57A shows that, relative to a tumor selectively replicating virus expressing GM-CSF and Flt3L-TRAIL, YSC-01 or YSC-02, particularly YSC-02, exhibits an antitumor effect in immune-deficient nude mice into which a human pancreatic cancer cell line is transplanted;
FIG. 57B shows that, relative to a tumor selectively replicating virus expressing GM-CSF and Flt3L-TRAIL, YSC-01 or YSC-02, particularly, YSC-02, shows a higher survival rate in immune-deficient nude mice into which a human pancreatic cancer cell line is transplanted;
FIG. 58 shows a mouse-derived hepatocellular carcinoma cell line (BNL-CAR-EIB55K-HSP25) expressing an inserted gene by introducing genes capable of enhancing infectivity and replicability of an adenovirus into a mouse and selecting clones;
FIG. 59 shows antitumor effects induced by adenoviruses (YSC-01 and YSC-02, GX-03) expressing four genes by comparing antitumor effects of all types of viruses used to infect immunocompetent mice to confirm the contribution of immune factors between murine genes and Flt3L-TRAIL having compatibility in a human and a mouse.
FIG. 60 shows that, in various cancer cells lines, an effect of reducing a survival rate when TGF-β1 or HSP27 is only decreased or TGF-β1 and HSP27 are simultaneously decreased or an effect of reducing a survival rate when the cells are infected with YSC-02, compared to GX-03 is highly exhibited in most cancer cell lines, as confirmed by a clonogenic assay;
FIG. 61 shows the comparison in CD4+ T and CD8+ T cell immunity of all types of viruses including YSC-01, 02 and GX-03 used to infect immunocompetent mice to confirm the contribution of immune factors between murine genes and Flt3L-TRAIL having compatibility in a human and a mouse.
FIG. 62 shows the comparison in T regulatory cell immunity of all types of viruses including YSC-01, 02 and GX-03 used to infect immunocompetent mice to confirm the contribution of immune factors between murine genes and Flt3L-TRAIL having compatibility in a human and a mouse.
FIG. 63 shows the comparison in DC immunity of all types of viruses including YSC-01, 02 and GX-03 used to infect immunocompetent mice to confirm the contribution of an immune factors between murine genes and Flt3L-TRAIL having compatibility in a human and a mouse.
FIG. 63A shows comparison in DC activity by PBS, an oncolytic control adenovirus, an oncolytic adenovirus only expressing murine GM-CSF, oncolytic adenovirus only expressing Flt3L-TRAIL;
FIG. 63B shows comparison in DC activity by an oncolytic adenovirus expressing murine GM-CSF and Flt3L-TRAIL, an oncolytic adenovirus expressing shRNA of murine GM-CSF, Flt3L-TRAIL and mouse type HSP25, an oncolytic adenovirus expressing shRNA of mouse type TGF-β1, and mouse type YSC-02; and
FIG. 63C shows comparison in DC activity by mouse type YSC-02 and mouse type GX-03.

### [Best Mode]

The present invention relates to a gene delivery system for coexpressing hTGF-β and shHSP (hereinafter, a two-type gene delivery system), which includes shRNA inhibiting TGF-β expression (shTGF-β) and shRNA inhibiting HSP expression (shHSP).

In addition, the present invention relates to a gene delivery system for coexpressing GM-CSF, Flt3L-TRAIL, shTGF-β and shHSP (hereinafter, a four-type gene delivery system), which includes a GM-CSF gene, a Flt3L-TRAIL gene, shRNA inhibiting TGF-β expression (shTGF-β) and shRNA inhibiting HSP expression (shHSP).

When being introduced into target cells using a gene delivery system coexpressing shRNA inhibiting TGF-β expression (shTGF-β) and shRNA inhibiting HSP expression (shHSP), compared to when each gene is expressed or even when GM-CSF and Flt3L-TRAIL are expressed, it was confirmed that an antitumor effect is further improved.

When being introduced into target cells using a gene delivery system for coexpressing a GM-CSF gene, a Flt3L-TRAIL fusion gene, shTGF-β and shHSP, it was confirmed that an antitumor effect is superior to that when each gene is expressed, and particularly, when shTGF-β and shHSP are expressed.

Particularly, the inventors confirmed that a GM-CSF gene, a Flt3L-TRAIL fusion gene, shRNA inhibiting TGF-β expression (shTGF-β) and shRNA inhibiting HSP27 expression (shHSP27) are very closely related to apoptosis and survival factors and immunological factors, combined in a manner effectively regulating these factors to overcome barriers in current cancer treatment, for example, cross-talk between different signaling networks in a cell, cross-talk between tumor cells and immune cells and intratumor heterogeneity, resulting in very effective and ultimate regulation of cancer cells within a wide range of cancer types.

The term "GM-CSF" used herein includes all homologues of GM-CSF inducing an immune reinforcing response as well as GM-CSF exemplified in an example.

A murine GM-CSF gene was obtained from Dr. Gerald C. O'Sullivan (Cork Cancer Research Centre, Mercy University Hospital and Leslie C. Quick Jnr. Laboratory, University College Cork, Cork, Ireland), and a base sequence is the same as disclosed in Cancer Gene Therapy (2006) 13, 1061-10710, and represented by SEQ ID NO: 1.

A human GM-CSF gene was obtained from InvivoGen, its base sequence is the same as disclosed in Gene Bank M11220 and represented by SEQ ID NO: 2.

A human Flt3L-TRAIL gene is prepared by fusing a gene encoding amino acids 1 to 181 of an FMS-like tyrosine kinase 3 ligand (Flt3L) and a gene encoding amino acids 95 to 281 of a tumor necrosis factor-related apoptosis-inducing ligand (TRAIL) with a leucine zipper, and represented by SEQ ID NO: 3 as shown in Gene Bank U03858 (Flt3L) and Gene Bank B032722 or U57059 (TRAIL).

In the case of the Flt3L-TRAIL fusion gene, since such a human-type gene has an activity even in a mouse, the human Flt3L-TRAIL fusion gene was used without separately manufacturing a mouse Flt3L-TRAIL fusion gene.

The term "shRNA inhibiting TGF-β expression (shTGF-β)" used herein refers to shRNA inhibiting TGF-β1 expression (shTGF-β1) or shRNA inhibiting TGF-β2 expression (shTGF-β2).

The "shRNA inhibiting TGF-β1 expression (shTGF-β1)" was disclosed in Korean Unexamined Patent Application No. 2013-0012095 (the sequence disclosed in Korean Unexamined Patent Application No. 2013-0012095 is represented by an RNA sequence. If the shTGF-β1 is represented as DNA, since U of the RNA sequence is substituted with T, the shTGF-β1 represented by an RNA sequence is the same as that represented by a DNA sequence). In the case of murine shRNA, shTGF-β1 is represented by SEQ ID NO: 4, and in the case of human shRNA, shTGF-β1 is represented by SEQ ID NO: 5.
[SEQ ID NO: 4]
   ccctctacaa ccaacacaac ccgggtctcc ccgggttgtg ttggttgtag aggg 54
[SEQ ID NO: 5]
   accagaaata cagcaacaat tcctguctct ccaggaattg ttgctggtat ttctggttt 59

The "shRNA inhibiting TGF-β2 expression (shTGF-β2)" is disclosed in Korean Unexamined Patent Application No. 2013-0088792. In the case of murine shRNA, the shTGF-β2 is represented by SEQ ID NO: 6, and in the case of human shRNA, the shTGF-β2 is represented by SEQ ID NO: 7 (when the shTGF-β2 is represented as RNA, T of the DNA sequence is substituted with U).
[SEQ ID NO: 6]
   ggattgaact gtatcagatc cttaatctct taaggatctg atacagttca atcc 54
[SEQ ID NO: 7]
   ggattgagct atatcagatt ctcaatctct tgagaatctg atatagctca atcc 54

The term "shRNA inhibiting HSP expression (shHSP)" used herein may include shHSP25 corresponding to murine shRNA or shHSP27 corresponding to human shRNA, in which the shHSP25 is represented by SEQ ID NO: 8, and the shHSP27 is disclosed in Korean Unexamined Patent Application No. 2013-0123244 and represented by SEQ ID NO: 9 (when the hHSP is represented as RNA, T of the DNA sequence is substituted with U).
[SEQ ID NO: 8]
   gctac atctc tcggt gcttc a tctc t gaagc accga gagat gtagc
[SEQ ID NO: 9]
   gatccgacga gcatggctac atctcccggt tctcaccggg agatgtagcc atgctcgtct

To prepare a gene delivery system of the present invention, shTGF-β and shHSP; or-a GM-CSF gene; a Flt3L-TRAIL fusion gene, shTGF-β and shHSP may be present in a suitable expression construct. In the expression construct, the genes may be operably linked to a promoter. The term "operatively linked" used herein refers to functional binding between a gene expression regulatory sequence (e.g., a promoter, a signal sequence, or an array of transcription regulatory factor-binding sites) and a different nucleic acid sequence, and therefore, the regulatory sequence regulates the transcription and/or translation of the other nucleic acid sequence. In the present invention, a promoter binding to the target genes of the present invention is preferably functional in an animal cell, and more preferably in a mammalian cell to regulate the transcription of a decorin gene, and includes a promoter derived from a mammalian virus and a promoter derived from the genome of a mammalian cell, for example, a cytomegalo virus (CMV) promoter, an adenovirus late promoter, a vaccinia virus 7.5K promoter, a SV40 promoter, a HSV tk promoter, a RSV promoter, an EF1 alpha promoter, a metallothionein promoter, a β-actin promoter, a human IL-2 gene promoter, a human IFN gene promoter, a human IL-4 gene promoter, a human lymphotoxin gene promoter and a human GM-CSF gene promoter, but the present invention is not limited thereto.

Preferably, the expression construct used in the present invention includes a polyadenylation sequence (e.g., a bovine growth hormone terminator and a SV40-derived polyadenylation sequence).

The gene delivery system of the present invention may be constructed in various forms, for example, (i) a naked recombinant DNA molecule, (ii) a plasmid, (iii) a viral vector, and (iv) a liposome or niosome containing the naked recombinant DNA molecule or plasmid.

A GM-CSF gene; a Flt3L-TRAIL fusion gene, shTGF-β (shTGF-β1 or shTGF-β2) and/or shHSP may be applied to all gene delivery systems which are used in conventional gene treatment, and preferably, to a plasmid, an adenovirus (Lockett LJ, et al., Clin. Cancer Res. 3:2075-2080(1997)), an adeno-associated virus (AAV, Lashford LS., et al., Gene Therapy Technologies, Applications and Regulations Ed. A. Meager, 1999), a retrovirus (Gunzburg WH, et al., Retroviral vectors. Gene Therapy Technologies, Applications and Regulations Ed. A. Meager, 1999), a lentivirus (Wang G. et al., J. Clin. Invest. 104(11):R55-62(1999)), a herpes simplex virus (Chamber R., et al., Proc. Natl. Acad. Sci USA 92:1411-1415(1995)), a vaccinia virus (Puhlmann M. et al., Human Gene Therapy 10:649-657(1999)), a liposome (Methods in Molecular Biology, Vol 199, S.C. Basu and M. Basu (Eds.), Human Press 2002) or a niosome. Most preferably, the gene delivery system of the present invention is constructed by applying a GM-CSF gene; a Flt3L-TRAIL fusion gene; shTGF-β; and/or shHSP to an adenovirus.

### i. Adenovirus

An adenovirus has been widely used as a gene delivery vector due to a medium-sized genome, easy manipulation, a high titer, a wide range of target cells and excellent infectibility. Both ends of the genome include an inverted terminal repeat (ITR) of 100 to 200 bp, which is a cis element essential for DNA replication and packaging. The E1 regions (E1A and E1B) of the genome encode proteins regulating transcription and the transcription of a host cell gene. The E2 regions (E2A and E2B) encode proteins involved in viral DNA replication. Among currently-developed adenovirus vectors, E1 region-deleted replication-deficient adenoviruses are widely used. However, the E3 region is removed from a conventional adenovirus vector to provide a foreign gene insertion site (Thimmappaya, B. et al., Cell, 31:543-551(1982); and Riordan, J. R. et al., Science, 245:1066-1073(1989)).

Therefore, shTGF-β (shTGF-β1 or shTGF-β2) and shHSP; or a GM-CSF gene, a Flt3L-TRAIL fusion gene, shTGF-β (shTGF-β1 or shTGF-β2) and shHSP according to the present invention may be inserted into the deleted E1 regions (the E1A region and/or the E1B region, and preferably the E1B region) or E3 region. In the specification, the term "deletion" used in terms of a viral genome sequence includes partial deletion of the corresponding sequence, as well as complete deletion of the corresponding sequence.

In addition, since the adenovirus may package approximately 105% of a wild-type genome, approximately 2 kb of genetic information may be additionally packaged (Ghosh-Choudhury et al., EMBO J., 6:1733-1739(1987)). Therefore, the above-mentioned foreign sequence inserted into the adenovirus may be additionally bound to the adenovirus genome. Adenoviruses have 42 different serotypes and A to F subgroups. Among these, adenovirus type 5 included in subgroup C is the most suitable starting material to obtain an adenovirus vector of the present invention. Biochemical and genetic information on the adenovirus type 5 is well known. The foreign gene delivered by the adenovirus is replicated in the same manner as an episome and has very low genetic toxicity with respect to a host cell. Accordingly, a gene therapy using the adenovirus gene delivery system of the present invention is considered to be very safe.

### ii. Retrovirus

A retrovirus is widely used as a gene delivery vector because it inserts its own genes into a host genome, being capable of delivering a great amount of foreign genetic substances, and having a wide spectrum of cells that can be infected.

To construct a retroviral vector, a replication-deficient virus is produced by inserting a target nucleotide sequence to be delivered, instead of a retrovirus sequence, into a retroviral genome. To produce a virion, a packaging cell line which includes gag, pol and env genes, but not including a long terminal repeat (LTR) and a Ψ sequence, is constructed (Mann et al., Cell, 33:153-159(1983)). When a recombinant plasmid including a target nucleotide sequence to be delivered, an LTR and a Ψ sequence is introduced into the cell line, the Ψ sequence facilitates production of an RNA transcript of the recombinant plasmid. This transcript is packaged into a virus, and the virus is released into a medium (Nicolas Flt3L-TRAIL fusion and Rubinstein "retroviral vectors," In: Vectors: A survey of molecular cloning vectors and their uses, Rodriguez and Denhardt(eds.), Stoneham: Butterworth, 494-513(1988)). The medium containing the recombinant retroviruses is collected and concentrated, and thus the recombinant retroviruses are used as a gene delivery system.

Gene delivery using a second generation retroviral vector was reported. According to Kasahara et al. (Science, 266:1373-1376 (1994)), a chimeric protein having a new binding characteristic was produced by preparing a Moloney murine leukemia virus (MMLV) mutant and inserting an erythropoietin (EPO) sequence into an envelope site. The gene delivery system of the present invention may also be prepared according to a strategy of constructing such a second generation retroviral vector.

### iii. AAV vector

An adeno-associated virus (AAV) can infect non-dividing cells and has an ability to infect various types of cells, and thus is suitable as a gene delivery system of the present invention. Detailed descriptions on the construction and use of an AAV vector are disclosed in U.S. Patent Nos. 5,139,941 and 4,797,368.

As a gene delivery system, a study on AAVs is disclosed in LaFace et al, Virology, 162:483486(1988), Zhou et al., Exp. Hematol. (NY), 21:928-933(1993), Walsh et al, J. Clin. Invest., 94:1440-1448(1994) and Flotte et al., Gene Therapy, 2:29-37(1995). Recently, an AAV vector has been implemented in a phase I clinical trial as a therapeutic agent for cystic fibrosis.

Typically, an AAV virus is prepared by simultaneously transforming a plasmid including a target gene sequence located beside two AAV terminal repeats (McLaughlin et al., J. Virol., 62:1963-1973(1988); Samulski et al., J. Virol., 63:3822-3828(1989)) and an expression plasmid (McCarty et al., J. Virol., 65:2936-2945 (1991)) including a wild type AAV coding sequence without a terminal repeat.

### iv. Other viral vectors

Other viral vectors may also be used as the gene delivery system of the present invention. Vectors derived from vaccinia viruses (Puhlmann M. et al., Human Gene Therapy 10:649-657(1999); Ridgeway, "Mammalian expression vectors," In: Vectors: A survey of molecular cloning vectors and their uses. Rodriguez and Denhardt, eds. Stoneham: Butterworth, 467-492(1988); Baichwal and Sugden, "Vectors for gene transfer derived from animal DNA viruses: Transient and stable expression of transferred genes," In: Kucherlapati R, ed. Gene transfer. New York: Plenum Press, 117-148 (1986) and Coupar et al., Gene, 68:1-10(1988)), lentiviruses (Wang G. et al., J. Clin. Invest. 104(11):R55-62(1999)) or herpes simplex viruses (Chamber R., et al., Proc. Natl. Acad. Sci USA 92:1411-1415(1995)) may also be used as a delivery system capable of delivering a target nucleotide sequence to be delivered into a cell.

### v. Liposome

Liposomes are automatically formed by phospholipids dispersed in an aqueous phase. Examples of successful delivery of a foreign DNA molecule into cells by a liposome are disclosed in Nicolau and Sene, Biochim. Biophys. Acta, 721:185-190(1982) and Nicolau et al., Methods Enzymol., 149:157-176(1987). Meanwhile, as a reagent that is most widely used in transformation of animal cells using a liposome, there is Lipofectamine (Gibco BRL). Liposomes containing a target nucleotide sequence to be delivered interact with cells through a mechanism such as endocytosis, adsorption to a cell surface or fusion with a plasma cell membrane to deliver a target nucleotide sequence to be delivered into the cells.

According to an exemplary embodiment of the present invention, the gene delivery system of the present invention is a recombinant adenovirus vector.

According to a more exemplary embodiment of the present invention, the recombinant adenovirus vector of the present invention does not have E1B and E3 regions, and the shTGF-β1 or shTGF-β2 is inserted into a site from which the E3 region is deleted, and the shHSP is inserted into a site from which the E3 region is deleted. In addition, in a four genes-introduced recombinant adenovirus vector, the GM-CSF encoding nucleotide and the Flt3L-TRAIL encoding nucleotide are inserted into the site from which the E1B region is deleted, and the shTGF-β1 or shTGF-β2 and shHSP are inserted into the site from which the E3 region is deleted.

The recombinant adenovirus including an active E1A gene may have replicable properties, and cell apoptosis potential may be enhanced when the E1B region is deleted. The "deletion" used in terms of viral genome sequence herein includes partial deletion of a corresponding sequence, as well as complete deletion of the corresponding sequence. The recombinant adenovirus of the present invention may include a non-mutated E1A gene or an active E1A gene which is mutated.

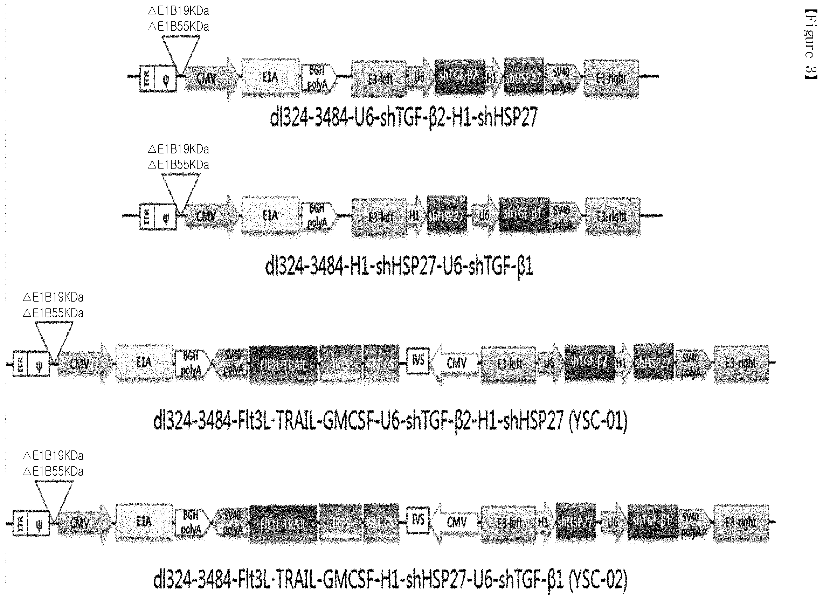

Most preferably, the recombinant adenovirus vector of the present invention includes shTGF-β1 or shTGF-β2 at the site from which the E3 region is deleted in a 5' to 3' direction, and shHSP27 at the site from which the E3 region is deleted in a 5' to 3' direction (FIG. 3).

The recombinant adenovirus vector of the present invention, into which four types of genes are introduced, includes a GM-CSF gene, an internal ribosome entry site (IRES) and Flt3L-TRAIL at the site from which the E1 region is deleted in a 3' to 5' direction, and shTGF-β2, shHSP27 or shHSP27 and shTGF-β1 at the site from which the E3 region is deleted in a 5' to 3' direction (FIG. 3).

In addition, the present invention provides an antitumor composition including a gene delivery system for expressing the shTGF-β and the shHSP; or a gene delivery system for expressing the GM-CSF, Flt3L-TRAIL, shTGF-β and shHSP.

The term "antitumor composition" used herein refers to a pharmaceutical composition to be used in tumor treatment.

Since the gene delivery system included in the antitumor composition of the present invention as an active ingredient is the same as the above-described gene delivery system of the present invention, the detailed description on the gene delivery system is also applied to the composition of the present invention. Therefore, in order to avoid excessive complexity due to unnecessary repetition in the specification, the common description will be omitted.

Since the recombinant adenovirus included in the composition of the present invention exhibits cytolytic efficacy against various tumor cells as described above, the pharmaceutical composition of the present invention may be used in treatment of tumor-related various diseases or disorders, for example, gastric cancer, lung cancer, breast cancer, ovarian cancer, liver cancer, bronchial cancer, nasopharyngeal cancer, laryngeal cancer, pancreatic cancer, bladder cancer, colon cancer, cervical cancer and melanoma. The term "treatment" used herein means (i) prevention of tumor cell formation; (ii) inhibition of tumor-related diseases or disorders according to removal of tumor cells; and (iii) alleviation of tumor-related diseases or disorders according to removal of tumor cells. Accordingly, the term "therapeutically effective amount" used herein refers to an amount sufficient for achieving a pharmaceutical effect.

A pharmaceutically acceptable carrier included in the composition of the present invention is conventionally used in formulation, and may be, but is not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate or mineral oil.

The pharmaceutical composition of the present invention may further include a lubricant, a wetting agent, a sweetening agent, a flavoring agent, an emulsifier, a suspension and a preservative in addition to the above-mentioned components.

The pharmaceutical composition of the present invention may be administered parenterally, for example, intravenously, intraperitoneally, intramuscularly, subcutaneously or locally. The pharmaceutical composition of the present invention may be administered intraperitoneally to treat ovarian cancer, and administered into a portal vein to treat liver cancer through injection, and may be directly injected into a tumor mass to treat breast cancer, directly injected through an enema to treat colon cancer, and directly injected into a catheter to treat bladder cancer.

A suitable dose of the pharmaceutical composition of the present invention may vary depending on factors such as a preparation method, an administration method, a patient's age, weight and sex, severity of disease symptoms, diet, administration time, an administration route, an excretion rate, and response sensitivity, and an effective dose for desired treatment may be easily determined and prescribed by an ordinarily skilled doctor. Generally, the pharmaceutical composition of the present invention includes 1 × 10⁵ to 1 × 10¹⁵ pfu/ml of recombinant adenoviruses, and is typically injected at, 1 × 10⁸ to 1 × 10¹³ pfu every two days for two weeks.

The pharmaceutical composition of the present invention may be prepared by unit-dose packaging or multi-dose packaging after being formulated using a pharmaceutically acceptable carrier and/or excipient according to a method that can be easily implemented by those of ordinary skill in the art. Here, a dosage form of the pharmaceutical composition of the present invention may be a solution in an oil or aqueous medium, a suspension or an emulsion, an extract, a powder, a granule, a tablet or a capsule, and the pharmaceutical composition of the present invention may further include a dispersant or a stabilizer.

The pharmaceutical composition of the present invention may be used independently or in combination with other conventional chemical or radiation therapies, and such combination therapy may be used more effectively in cancer treatment. Chemical therapeutics that can be used together with the composition of the present invention include gemcitabine, sorafenib, cisplatin, carboplatin, procarbazine, mechlorethamine, cyclophosphamide, ifosfamide, melphalan, chlorambucil, bisulfan, nitrosourea, dactinomycin, daunorubicin, doxorubicin, bleomycin, plicomycin, mitomycin, etoposide, tamoxifen, taxol, transplatinum, 5-fluorouracil, vincristin, vinblastin and methotrexate. Radiation therapies that can be used together with the composition of the present invention include X-ray radiation and γ-ray radiation.

According to another exemplary embodiment of the present invention, the composition of the present invention improves tumor apoptosis, immune activity and immunogenicity.

In addition, the present invention provides an antitumor composition including shRNA inhibiting TGF-β expression (shTGF-β) and shRNA inhibiting HSP expression (shHSP).

All descriptions regarding the antitumor composition may also be applied or applied mutatis mutandis to a gene community and/or antitumor composition of the present invention

Particularly, the composition of the present invention is prepared to coexpress shRNA inhibiting TGF-β expression (shTGF-β) and/or shRNA inhibiting HSP expression (shHSP) by introducing the shTGF-β and/or shHSP into one or more gene delivery systems.

In addition, the present invention provides an antitumor composition including a GM-CSF gene; a Flt3L-TRAIL gene, shRNA inhibiting TGF-β expression (shTGF-β) and shRNA inhibiting HSP expression (shHSP).

All descriptions regarding the antitumor composition may also be applied or applied mutatis mutandis to a gene community and/or antitumor composition of the present invention.

Particularly, the composition of the present invention is prepared to coexpress a GM-CSF gene; an Flt3L-TRAIL gene, shRNA inhibiting TGF-β expression (shTGF-β) and/or shRNA inhibiting HSP expression (shHSP) by introducing the GM-CSF gene; the Flt3L-TRAIL gene, shTGF-β and/or shHSP into one or more gene delivery systems.

In addition, the present invention provides an adenovirus into which the gene delivery system is introduced.

In the present invention, as a virus useful for transferring a nucleic acid molecule for RNAi (viral vector), there are an adenovirus, a retrovirus, a lentivirus and an AAV, and among these, an adenovirus is preferable because of the need for temporary induction of expression like in tumors.

In addition, the present invention includes a method for treating a tumor, which includes administering a therapeutically effective amount of gene delivery systems (2 or 4 types) including shRNA inhibiting TGF-β expression (shTGF-β) and shRNA inhibiting HSP expression (shHSP), or a GM-CSF gene; a Flt3L-TRAIL fusion gene; shRNA inhibiting TGF-β expression (shTGF-β) and shRNA inhibiting HSP expression (shHSP) to a subject.

In addition, the present invention includes a method for treating a tumor, which includes administering therapeutically effective amounts of shRNA inhibiting TGF-β expression (shTGF-β) and shRNA inhibiting HSP expression (shHSP) to a subject.

In addition, the present invention includes a method for treating a tumor, which includes administering therapeutically effective amounts of a GM-CSF gene; an Flt3L-TRAIL fusion gene; shRNA inhibiting TGF-β expression (shTGF-β) and shRNA inhibiting HSP expression (shHSP) to a subject.

All descriptions regarding the method for treating a tumor may also be applied or applied mutatis mutandis to a gene community and/or antitumor composition of the present invention.

The term "subject" used herein refers to a mammal which is a target for treatment, observation or experiment, and preferably a human.

The term "therapeutically effective amount" used herein is an amount of an active ingredient or pharmaceutical composition that induces a biological or medical response in a tissue system, animal or human that is considered by a researcher, a veterinarian, a doctor or other clinicians, and includes an amount for inducing the alleviation of symptoms of a disease or disorder to be treated. It will be apparent to those of ordinary skill in the art that the therapeutically effective amount and administration frequency of the active ingredient of the present invention will vary depending on a desired effect. Therefore, the optimal dosage to be administered may be easily determined by those of ordinary skill in the art, and the range of the optimal dosage varies depending on the type of a disease, the severity of a disease, the contents of an active ingredient and other ingredients, which are contained in the composition, the type of a dosage form, a patient's body weight, age, sex and health condition, diet, an administration time, an administration method, and an excretion rate. In the treatment method of the present invention, the composition includes 1 × 10⁵ to 1 × 10¹⁵ pfu/ml of recombinant adenoviruses, and typically, 1 × 10⁸ to 1 × 10¹³ pfu is administered every two days for two weeks.

In the treatment method of the present invention, the antitumor composition of the present invention may be administered orally or parenterally (e.g., intravenously, subcutaneously, intraperitoneally or locally) according to a desired method.

Hereinafter, the present invention will be described in further detail with reference to examples of the present invention, but the scope of the present invention is not limited to the following examples.

### [Examples]

### Reference Example 1: Preparation of GM-CSF gene

A murine GM-CSF gene was obtained from Dr. Gerald C. O'Sullivan (Cork Cancer Research Centre, Mercy University Hospital and Leslie C. Quick Jnr. Laboratory, University College Cork, Cork, Ireland), and its base sequence is disclosed in Cancer Gene Therapy ((2006) 13, 1061-10710) and represented by SEQ ID NO: 1.

A human GM-CSF gene was obtained from InvivoGen, and its base sequence, as shown in Gene Bank M11220, is represented by SEQ ID NO: 2.

The gene sequence used in Korean Patent Application No. 2015-0044507 refers to the base sequence set forth in SEQ ID NO: 2 corresponding to 33 to 467 bp of the 789 bp base sequence actually shown in Gene Bank M11220.

### Preparation Example 1: Construction of adenovirus for expressing GM-CSF (replication-deficient: dl324-GM-CSF, replication-competent: dl324-3484-CMVp-ΔE1B-GM-CSF)

An adenovirus for expressing GM-CSF was constructed in the same manner as in Preparation Example 1 disclosed in Korean Patent No. 2015-0044507 by using the same gene disclosed in Korean Patent No. 2015-0044507.

### Reference Example 2: Preparation of Flt3L-TRAIL fusion gene

A human Flt3L-TRAIL gene was prepared by fusing a gene encoding amino acids 1-181 of FMS-like tyrosine kinase 3 ligand (Flt3L) and a gene encoding amino acids 95-281 of tumor necrosis factor-related apoptosis-inducing ligand (TRAIL) using a leucine zipper, and is represented by SEQ ID NO: 3, as shown in Gene Bank U03858 (Flt3L) and Gene Bank B032722 or U57059 (TRAIL).

In the case of the Flt3L-TRAIL fusion gene, since a human-type gene also has an activity in a mouse, the human Flt3L-TRAIL fusion gene was used without separately constructing a mouse Flt3L-TRAIL fusion gene.

### Preparation Example 2: Construction of Flt3L-TRAIL fusion gene (Flt3L-TRAIL)-loaded oncolytic adenovirus (dl324-3484-CMVp-ΔE1B-Flt3L-TRAIL)

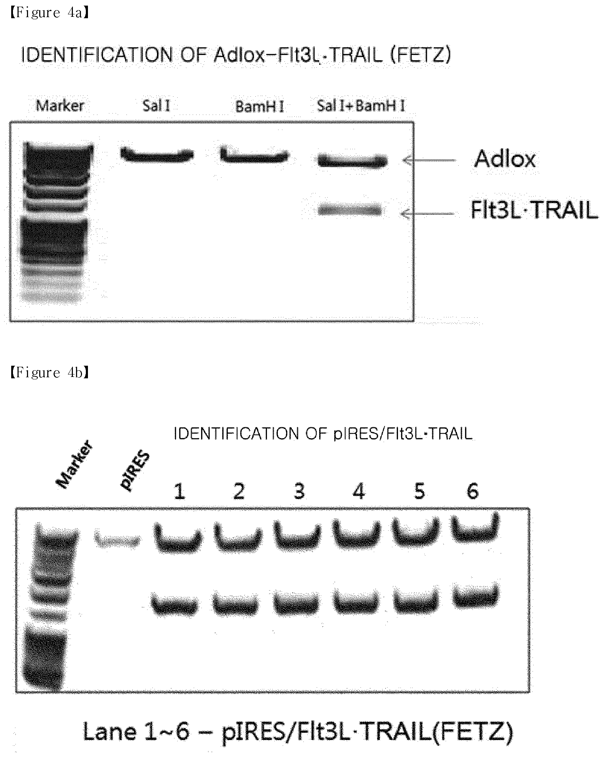

Adlox-FETZ (Flt3L-TRAIL) was prepared by transferring FETZ (Flt3L-TRAIL fusion gene; Flt3L is only formed in a soluble form, and TRAIL only consists of an extracellular domain such as a 95-281 region, both being linked with an isoleucine zipper) prepared by treating pFETZ (Flt3L-TRAIL; Regression of human mammary adenocarcinoma by systemic administration of a recombinant gene encoding the hFlex-TRAIL fusion protein by Xiaofeng Wu et al., Molecular Therapy vol. 3 368-374, 2001) with Sal/BamHI to an Adlox vector cleaved with SalI/BamHI (FIG. 4A).

Adlox-Flt3L-TRAIL was cleaved with BamHI, and pIRES was cleaved with NotI, resulting in blunting.

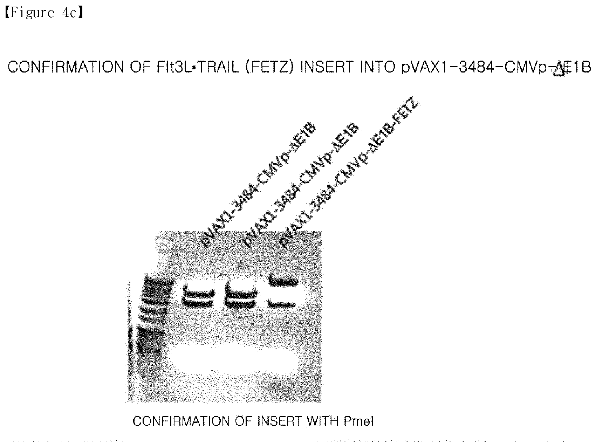

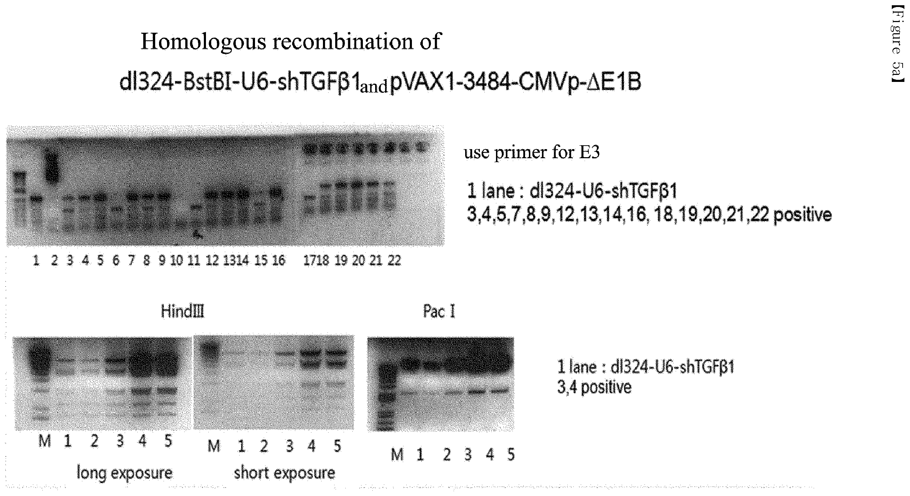

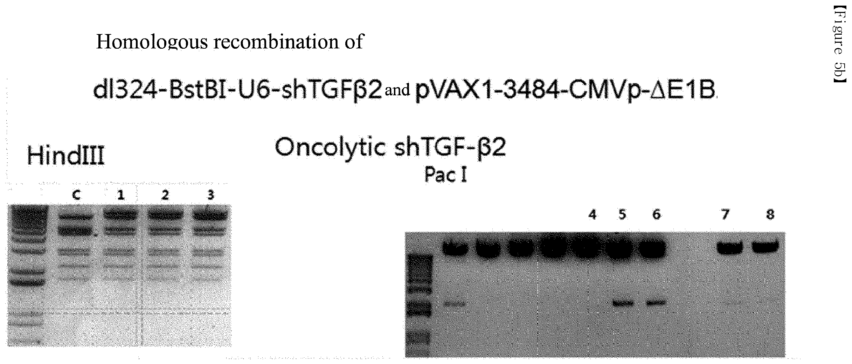

Subsequently, each end was cleaved with SalI and then linked. Flt3L-TRAIL insertion was confirmed by cleaving the previously-obtained product with Xbal and MfeI to identify an insert (FIG. 4B). Afterward, to insert a Flt3L-TRAIL gene into a pVAX1-3484-CMVp-ΔE1B(-E1R) shuttle vector (the same vector pVAX1-3484-CMVp-ΔE1B-E1R shown in FIG. 5 in Korean Patent Application No. 2015-0044507), pVAX1-3484-CMVp-ΔE1B(-E1R) was cleaved with SalI, and pIRES-Flt3L-TRAIL was cleaved with FspI, thereby blunting the vector. Likewise, after cleaving with BglII, Flt3L-TRAIL cut out of pIRES-Flt3L TRAIL was subcloned into a shuttle vector. FIG. 4C shows that the presence of the insert by cleavage with PmeI.

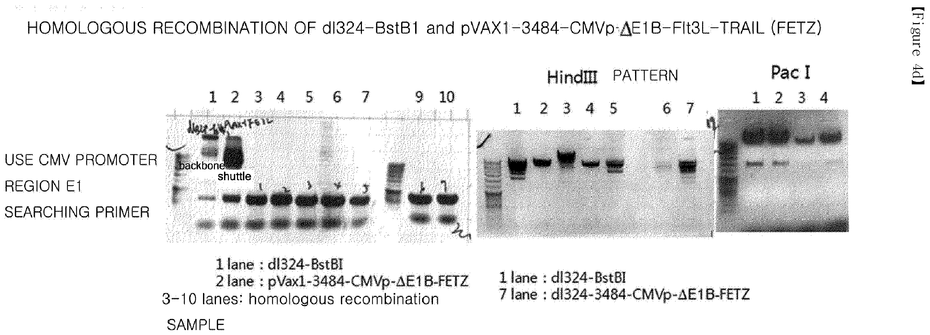

To produce a virus, a viral backbone dl324-BstBI was cleaved with Bsp1191, the constructed pVAX1-3484-CMVp-ΔE1B-Flt3L-TRAIL was linearized using PmeI and used to transform *E. coli* BJ5183, thereby producing homologous recombination DNA. As a result, recombination was confirmed using a HindIII pattern and PacI cleavage (FIG. 4D).

An adenovirus dl324-3484-CMVp-ΔE1B-Flt3L-TRAIL was constructed through the homologous recombination described above.

### Reference Example 3: Preparation of gene of shRNA that inhibits TGF-β1 expression (shTGFβ1)

As disclosed in Korean Unexamined Patent Application Publication No. 2013-0012095, shRNA inhibiting TGF-β1 expression represented by SEQ ID NO: 4 or 5 (hereinafter, referred to as shTGF-β1) was prepared.

In the case of murine shRNA, the shTGF-β1 is represented by SEQ ID NO: 4, and in the case of human shRNA, the shTGF-β1 is represented by SEQ ID NO: 5.

### Reference Example 4: Preparation of gene of shRNA that inhibits TGF-β2 expression (shTGFβ2)

As shown in Korean Unexamined Patent Application Publication No. 2013-0088792, shRNA inhibiting TGF-β2 expression (hereinafter, referred to as shTGF-β2) represented by SEQ ID NO: 6 or 7 was prepared.

In the case of murine shRNA, the shTGF-β2 is represented by SEQ ID NO: 6, and in the case of human shRNA, the shTGF-β2 is represented by SEQ ID NO: 7.

### Preparation Example 3: Construction of shTGF-β-loaded oncolytic adenovirus (dl324-3484-CMVp-ΔE1B-U6-shTGFβ1, dl324-3484-CMVp-ΔE1B-U6-shTGFβ2)

Based on Korean Unexamined Patent Application Publication Nos. 2013-0088792 and 2013-0012095, to produce a virus, a viral backbone dl324-BstBI-U6-shTGF-β (β1 or β2) was cleaved with Bsp1191, pVAX1-3484-CMVp-ΔE1B was linearized with PmeI and used to transform *E. coli* BJ5183, thereby producing homologous recombination DNA. As a result, recombination was confirmed using a HindIII pattern and PacI cleavage (FIGS. 5A and 5B).

An adenovirus dl324-3484-CMVp-ΔE1B-U6-shTGFβ1 or dl324-3484-CMVp-ΔE1B-U6-shTGFβ2 was constructed through the homologous recombination described above.

### Reference Example 5: Preparation of gene of shRNA that inhibits HSP27 expression (shHSP27)

As disclosed in Korean Unexamined Patent Application Publication No. 2013-0123244, shRNA inhibiting HSP27 expression (hereinafter, referred to as shHSP27) represented by SEQ ID NO: 9 was prepared.

### Preparation Example 4: Construction of shHSP27-loaded oncolytic adenovirus (dl324-3484-CMVp-ΔE1B-H1-shHSP27)

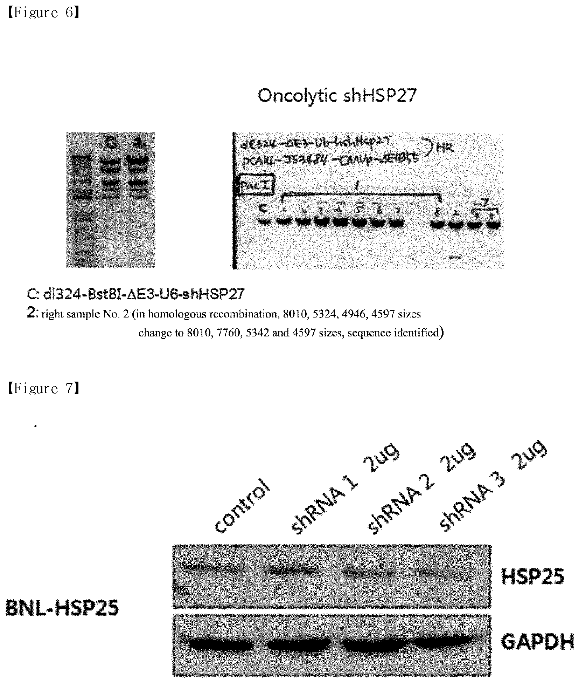

Based on Korean Unexamined Patent Application Publication No. 2013-0123244, to produce a virus, a viral backbone dl324-BstBI-H1-shHSP27 was cleaved with Bsp1191, pVAX1-3484-CMVp-ΔE1B was linearized with PmeI and used to transform *E*. *coli* BJ5183, thereby producing homologous recombination DNA. As a result, recombination was confirmed using a HindIII pattern and PacI cleavage (FIG. 6).

An adenovirus dl324-3484-CMVp-ΔE1B-H1-shHSP27 was constructed through the homologous recombination described above.

### Reference Example 6: Preparation of gene of shRNA that inhibits HSP25 expression (shHSP25)

shRNA inhibiting HSP25 expression (shHSP25) represented by SEQ ID NO: 8 was prepared.

To prepare effective shHSP25 inhibiting murine HSP25 expression, three base sequences for targeting a shRNA candidate were prepared.

Murine target sequence: 5'-gctac atctc tcggt gcttc a-3' (SEQ ID NO: 10)
1. Sense 5'-GATCC gcctc ttcga tcaag ctttc g TCTC c gaaag cttga tcgaa gaggc TTTT A-3' (SEQ ID NO: 11)
   Antisense 5'-AGCTT AAAA gcctc ttcga tcaag ctttc g GAGA c gaaag cttga tcgaa gaggc G-3' (SEQ ID NO: 12)
2. Sense 5'-GATCC gctac atctc tcggt gcttc a TCTC t gaagc accga gagat gtagc TTTT A-3' (SEQ ID NO: 13)
   Antisense 5'-AGCTT AAAA gctac atctc tcggt gcttc a GAGA t gaagc accga gagat gtagc G-3' (SEQ ID NO: 14)
3. Sense 5'-GATCC ggaga tcacc attcc ggtta c TCTC g taacc ggaat ggtga tctcc TTTT A-3' (SEQ ID NO: 15)
   Antisense 5'-AGCTT AAAA ggaga tcacc attcc ggtta c GAGA g taacc ggaat ggtga tctcc G-3' (SEQ ID NO: 16)

For pSP72ΔE3-H1-shHSP25 subcloning, pSP72ΔE3-H1-hshTGFβ2 (refer to Preparation Example 5 disclosed in Korean Patent No. 2015-0044507) was cleaved with BamHI/HindIII, and then linked with three types of annealed shHSP25. To confirm an HSP25 shRNA effect from the pSP72ΔE3-H1-mshHSP25-1, 2 or 3 obtained as described above, a BNL-HSP25 cell line obtained through stable transfection of HSP25 into a BNL murine hepatocellular carcinoma cell line (BNL 1ME A.7R.1) was transfected with the three types of shHSP25 to confirm an effect of reducing HSP25 expression (FIG. 7).

### Preparation Example 5: Construction of shHSP25-loaded oncolytic adenovirus (dl324-3484-CMVp-ΔE1B-H1-shHSP25)

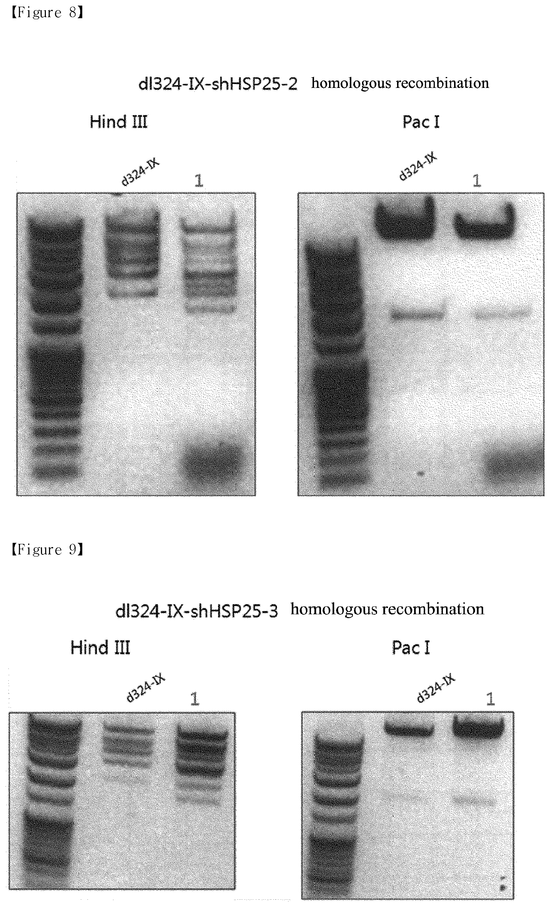

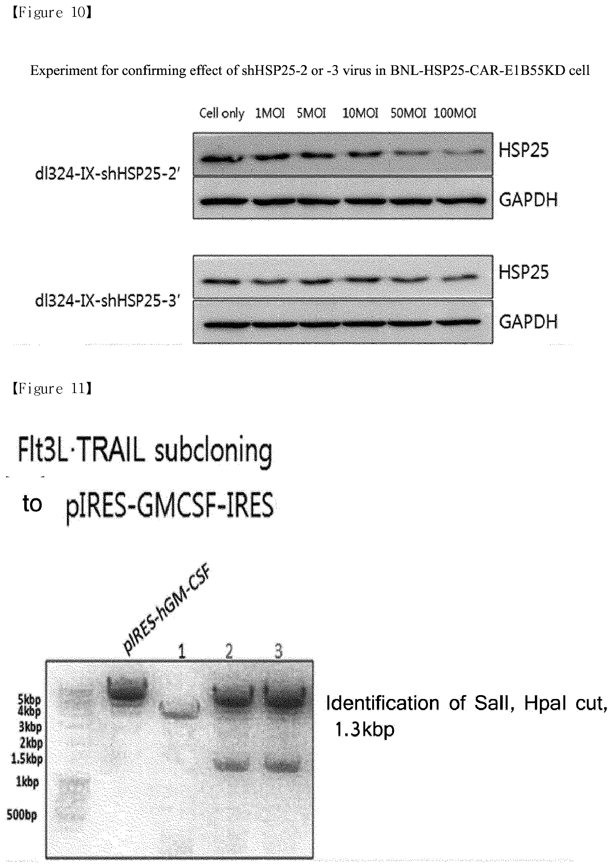

Homologous recombination of the pSP72ΔE3-H1-shHSP25-2 or 3, which exhibits a reducing effect as shown in Reference Example 5, and dl324-IX was performed. For homologous recombination to dl324-IX-shHSP25-2, a pSP72-shHSP25-2 shuttle vector was cleaved with DrdI, backbone DNA dl324-IX was cleaved with SpeI for linearization, and then colonies obtained by transformation of BJ5183 with the resulting fragment were selected using a HindIII pattern and PacI cleavage (FIG. 8). On the other hand, for homologous recombination to dl324-IX-shHSP25-3, a pSP72-shHSP25-3 shuttle vector was cleaved with XmnI, backbone DNA dl324-IX was cleaved with SpeI for linearization, and then colonies obtained by transformation of BJ5183 with the resulting fragment were selected using a HindIII pattern and PacI cleavage (FIG. 9). An effect of reducing expression was confirmed from each adenovirus which expresses HSP25 shRNA from the construct obtained as described above (FIG. 10). Therefore, for subsequent construction of murine shRNA HSP25, a shHSP25-2 sequence [SEQ ID NO: 8] was used.

### Preparation Example 6: Construction of GM-CSF and Flt3L-TRAIL-replicable adenovirus (dl324-3484-CMVp-ΔE1B-GM-CSF-IRES-Flt3L-TRAIL)

A tumor-selective replication-competent adenovirus which simultaneously expresses GM-CSF and Flt3L-TRAIL was constructed.

### 1) Construction of vector simultaneously expressing GM-CSF and Flt3L-TRAIL

### ① Human

A GM-CSF gene was inserted into multi cloning site (MCS) A of pIRES (Clontech), and a Flt3L-TRAILgene was inserted into MCS B.

To subclone a human GM-CSF gene into the MCS A site of pIRES, a primer for PCR was prepared. As a sense strand, 5'-CCG CTCGAG ATGTGGCTGC AGAGCCTGCT G-3' (SEQ ID NO: 17) having an XhoI site at the 5' end was prepared, and as an antisense strand, 5'-CCGACGCGTTCACTCCTGGACTGGCTCCCA-3' (SEQ ID NO: 18) having MluI at the 5' end was prepared. pORF-GMCSF was used as a PCR template to perform PCR for 30 cycles under the following conditions: 2 min at 95 °C (initial denaturation); 1 min at 95°C (denaturation); 1 min at 55 °C (annealing); and 1 min at 72 °C (elongation). In addition, final elongation was performed for 5 minutes at 72 °C.

To subclone a human Flt3L-TRAIL gene into the MCS B site of pIRES, pIRES-hGM-CSF was cleaved with SalI/SmaII, Adlox-Flt3L-TRAIL was also cleaved with SalI/SmaI, and then the cleaved Flt3L-TRAIL was inserted into and ligated with pIRES-GM-CSF, resulting in obtaining pIRES-GMCSF-Flt3L-TRAIL.

Adlox-Flt3L-TRAIL was prepared by transferring hFlex-TRAIL (Flt3L-TRAIL fusion gene: SEQ ID NO: 3) obtained by cleaving pFETZ (Flt3L-TRAIL; Regression of human mammary adenocarcinoma by systemic administration of a recombinant gene encoding the hFlex-TRAIL fusion protein, Molecular Therapy vol. 3 368-374, 2001) with SalI/BamHI to an Adlox vector cleaved with Sal/BamHI.

The inserted Flt3L-TRAIL was cleaved with SalI/HpaI, and the resulting fragment was inserted into Lane 2 and Lane 3 as follows (FIG. 11).

### (2) Mouse

To subclone a murine GM-CSF gene into the MCS A site of pIRES, a primer for PCR was prepared. As a sense strand, 5'-CCG CTCGAG ATGTACAGGATGCAACTCCTGTCT-3'(SEQ ID NO: 19) having an XhoI site at the 5' end was prepared, and as an antisense strand, 5'-CCGACGCGT TCATTTTTGGCCTGGTTTTTTGCA-3' (SEQ ID NO: 20) having MluI at the 5' end was prepared. pCA14-mGM-CSF was used as a PCR template to perform PCR for 30 cycles under the following conditions: 2 min at 95 °C (initial denaturation); 1 min at 95 °C (denaturation); 1 min at 55 °C (annealing); and 1 min at 72 °C (elongation). In addition, final elongation was performed for 5 minutes at 72 °C.

An experimental procedure is the same as described in process ①, except that a human GM-CSF gene is replaced with a murine GM-CSF gene, and a human Flt3L-TRAIL fusion gene is used since it can be applied to a mouse.

### 2) Construction of GM-CSF and Flt3L-TRAIL-inserted shuttle vector

Cloning into an oncolytic shuttle vector was performed as follows.

Both vectors for a human and a mouse were constructed by the same method.

pIRES-GM-CSF-Flt3L-TRAIL was cleaved with FspI (blunting), and then with BglII, thereby obtaining hGM-CSF (or mGM-CSF) and Flt3L-TRAIL. pVAX1-3484-CMVp-ΔE1B-E1R was treated with SalI for blunting, and then with BglII, followed by ligation with the Bgl II and Fsp I fragments.

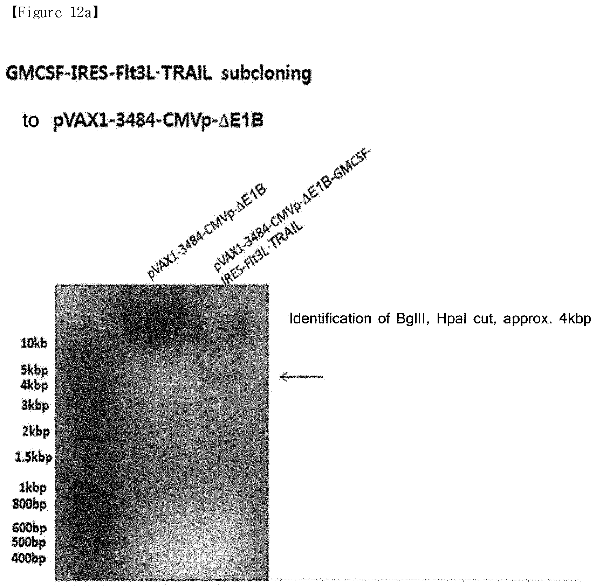

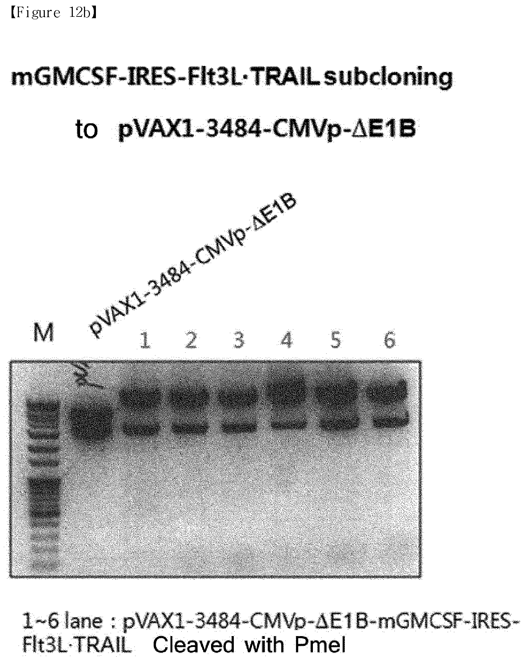

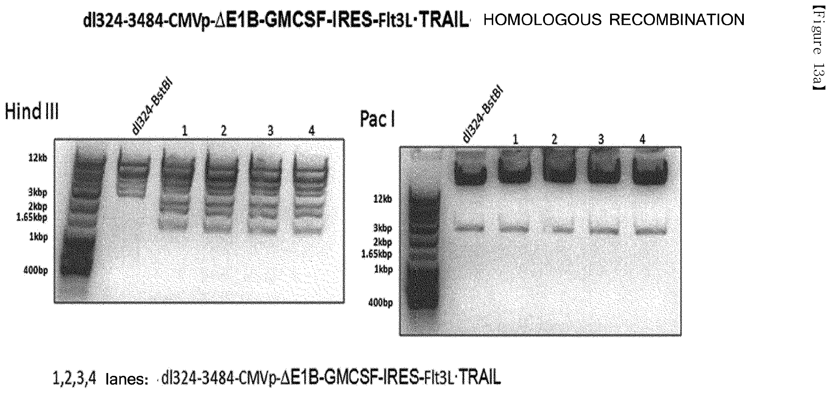

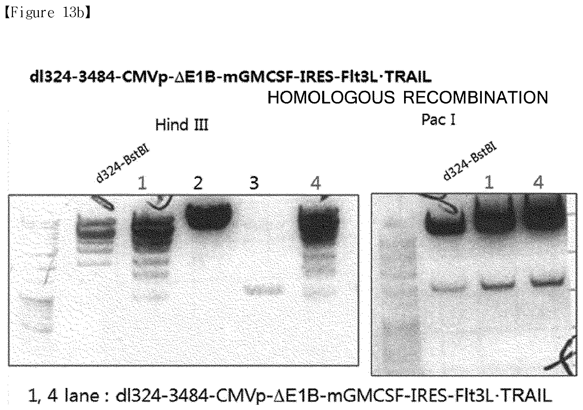

A subcloned product was cleaved with PmeI to confirm DNA fragments with two different sizes, and finally pVAX1-3484-CMVp-ΔE1B-GMCSF (human or mouse)-IRES-Flt3L-TRAIL was obtained (FIG. 12). Afterward, through homologous recombination with dl324-BstBI, dl324-3484-CMVp-ΔE1B-human GMCSF-IRES-Flt3L-TRAIL or dl324-3484-CMVp-ΔE1B-murine GMCSF-IRES-Flt3L-TRAIL was obtained (FIG. 13).

### 3) Construction of tumor-selective replication-competent adenovirus which expresses GM-CSF and Flt3L-TRAIL (the same for both a human and a mouse)

To produce a virus, a viral backbone DNA dl324-BstBI was cleaved with Bsp1191, and pVAX1-3484-CMVp-ΔE1B-GMCSF-IRES-Flt3L-TRAIL was linearized with PmeI and then inserted into *E. coli* BJ5183 for transformation, thereby producing homologous recombinant DNA. As a result, successful recombination was confirmed with a HindIII pattern and PacI cleavage (FIG. 13).

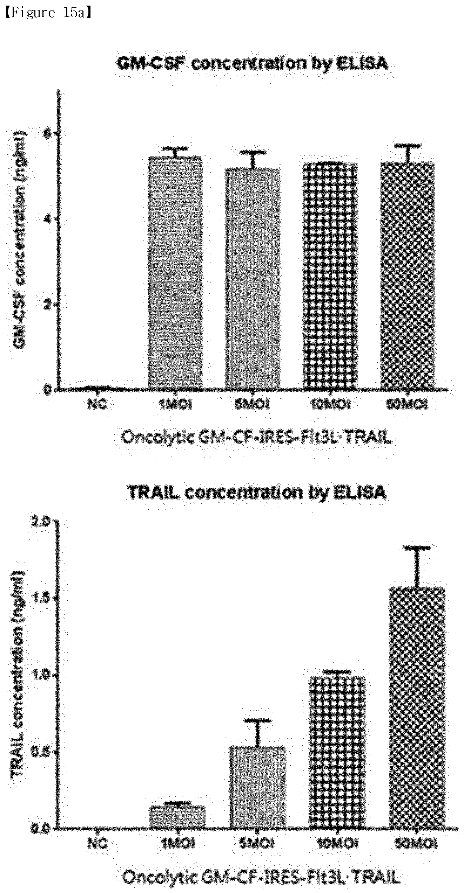

To confirm that human GM-CSF and Flt3L-TRAIL proteins were expressed, GM-CSF and TRAIL which were released into a medium were measured by infecting DU-145 human prostatic cancer cells with a virus at different MOIs, and after 24 hours, culturing the cells in a fresh serum-free medium for a day after changing the medium. As a result, ELISA was performed to confirm that GM-CSF and TRAIL are normally expressed (FIG. 15A).

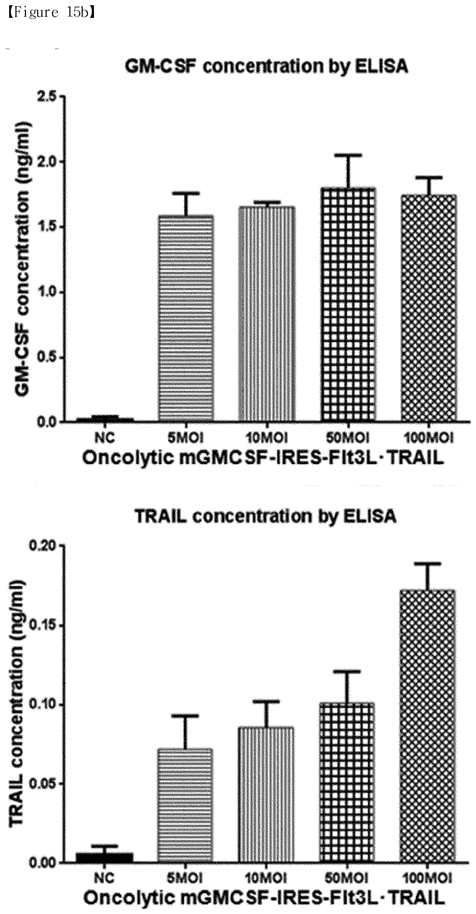

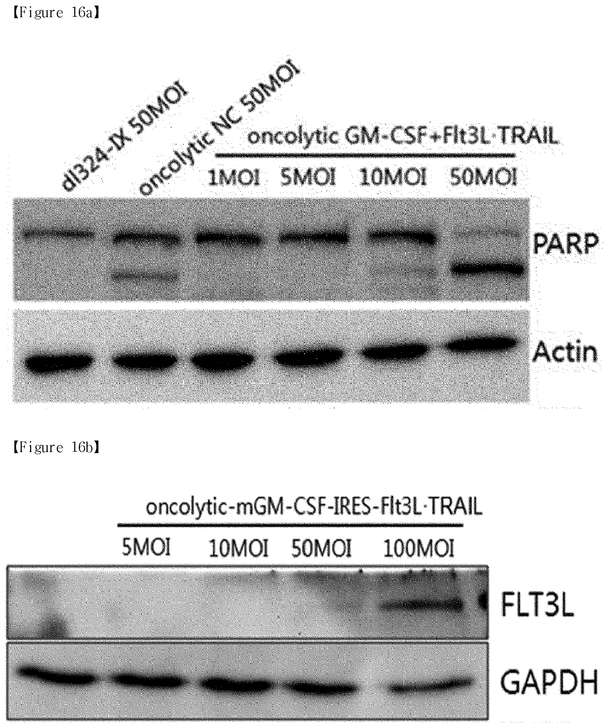

Likewise, to confirm that murine GM-CSF and Flt3L-TRAIL proteins were expressed, GM-CSF and TRAIL which were released into a medium were measured by infecting murine hepatocellular carcinoma cell (BNL)-derived BNL-CAR-E1B55-HSP25 cells with a virus at different MOIs, and after 24 hours, culturing the cells in a fresh serum-free medium for a day after changing the medium. As a result, ELISA was performed to confirm that GM-CSF and TRAIL are normally expressed (FIG. 15B), and western blotting was performed to confirm Flt3L expression (FIG. 16B).

Afterward, western blotting was performed on DU-145 to confirm whether TRAIL of Flt3L-TRAIL present in the virus maintains an apoptotic function. As a result, it can be seen that the apoptotic function is properly exhibited since PARP cleavage is highly shown at an increased MOI (50 MOI) (FIG. 16A).

DNA obtained from a bacterial clone in which homologous recombination had been confirmed was cleaved with PacI, and introduced into a 293A (a subclone of the 293 human embryonic kidney cell line) cell line for transfection, thereby producing an adenovirus. The purified adenovirus obtained by proliferation in the 293 cell line, isolation according to a CsCl concentration gradient by ultracentrifugation and dialysis was analyzed using a standard plaque assay kit developed by Qbiogene (Carlsbad, CA, USA) to estimate a titer. The final virus titer was 1x10¹² to 5x10¹².

### Preparation Example 7: Construction of replication-deficient adenoviruses that simultaneously express shTGFβ and shHSP27 (human)(dl324-H1-shHSP27-U6-shTGFβ1 and dl324-U6-shTGFβ2-HI-shHSP27)

### 1) Construction of pSP72ΔE3-U6-shTGFβ1

BamHI and HindIII cleavage was performed between an U6 promoter and poly A of pSP72ΔE3-U6-sh-negative (the same as the pSP72/ΔE3/si-negative vector described in Example 2 disclosed in Korean Unexamined Patent Application Publication No. 2013-0012095), and then
a top strand (5'-gatcc G*CCAGAAATACAGCAACAATTCCTG* tctctc CAGGAATTGTTGCTGTATTTCTGGT tttttt a- 3', SEQ ID NO: 21) and
a bottom strand (5'-agctt aaaaaa ACCAGAAATACAGCAACAATTCCTG gagaga CAGGAATTGTTGCTGTATTTCTGGT g-3', SEQ ID NO: 22) were ligated through annealing, thereby constructing pSP72ΔE3-U6-shTGFβ1 (refer to Examples 1 and 2 disclosed in Korean Unexamined Patent Application Publication No. 2013-0012095).

### 2) Construction of pSP72ΔE3-U6-shTGFβ2

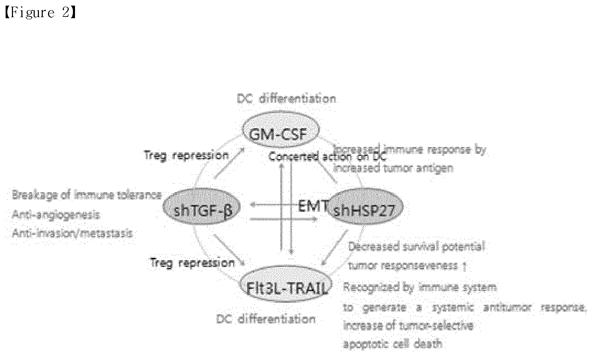

BamHI and HindIII cleavage was performed between a U6 promoter and poly A of pSP72ΔE3-U6-sh-negative, and then
a top strand (5'-gatcc GGATTGAGCTATATCAGATTCTCAA tctc TTGAGAATCTGATATAGCTCAATCC tttt a -3' , SEQ ID NO: 23) and
a bottom strand (5'-agctt aaaa GGATTGAGCTATATCAGATTCTCAA gaga TTGAGAATCTGATATAGCTCAATCC g-3', SEQ ID NO: 24) were ligated through annealing, thereby constructing pSP72ΔE3-U6-shTGFβ2 (refer to Example 2 and FIG. 2 disclosed in Korean Unexamined Patent Application Publication No. 2013-0088792).

### 3) Construction of pSP72ΔE3-H1-shHSP27

pSP72ΔE3-H1-shTGFβ2 of Preparation Example 5 disclosed in Korean Patent Application No. 2015-0044507 was cleaved with BamH/HindIII, and then
a top strand (5'-gatcc gacgagcatggctacatctcccggt tctc accgggagatgtagccatgctcgtc tttttt a-3', SEQ ID NO: 25) and
a bottom strand (5'-agctt aaaa gacgagcatggctacatctcccggt gaga accgggagatgtagccatgctcgtc g-3', SEQ ID NO: 26) were ligated through annealing, thereby constructing pSP72ΔE3-H1-shHSP27 (refer to Examples 1 and 2 disclosed in Korean Unexamined Patent Application Publication No. 2013-0123244).

### 4) Construction of pSP72ΔE3-U6-shTGFβ2-H1-shHSP27 and pSP72ΔE3-H1-shHSP27-U6-shTGFβ1 shuttle vectors

A pSP72ΔE3-U6-shTGFβ2-H1-shHSP27 shuttle vector was constructed by blunting the pSP72ΔE3-U6-shTGFβ2 with HindIII, removing an SV40 poly A site with KpnI treatment, blunting the pSP72ΔE3-H1-shHSP27 with SphI, removing H1 promoter-shHSP27-SV40 poly A by KpnI treatment, and ligating the H1 promoter-shHSP27-SV40 poly A with pSP72ΔE3-U6-shTGFβ2 treated with HindIII (blunt)/KpnI.

On the other hand, a pSP72ΔE3-H1-shHSP27-U6-shTGFβ1 shuttle vector was constructed by blunting pSP72ΔE3-H1-shHSP27 with HindIII, removing a SV40 poly A site by KpnI treatment, blunting the pSP72ΔE3-U6-shTGFβ1 with SphI, removing U6 promoter-shTGF-β1-SV40 poly A by KpnI treatment, and ligating the U6 promoter-shTGF-β1-SV40 poly A with HindIII (blunt)/KpnI-treated pSP72ΔE3-H1-shHSP27.

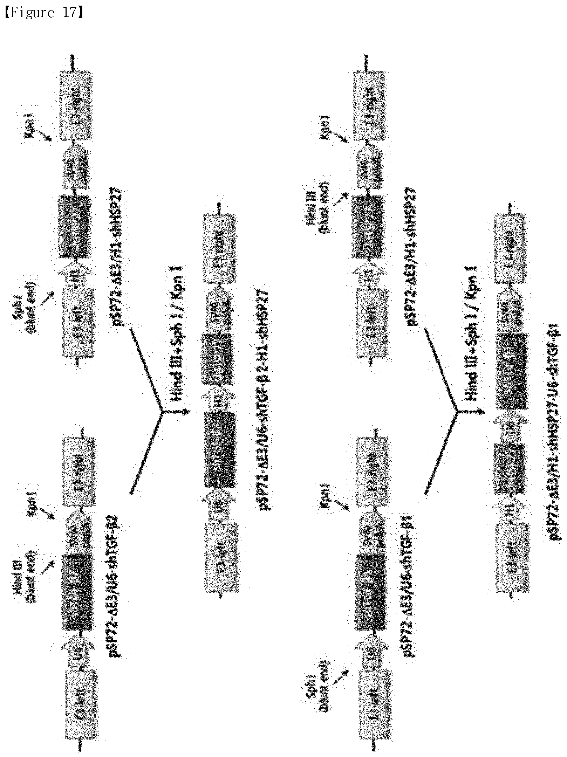

A process of constructing the pSP72ΔE3-U6-shTGFβ2-H1-shHSP27 or pSP72ΔE3-H1-shHSP27-U6-shTGFβ1 shuttle vector is illustrated in FIG. 17.

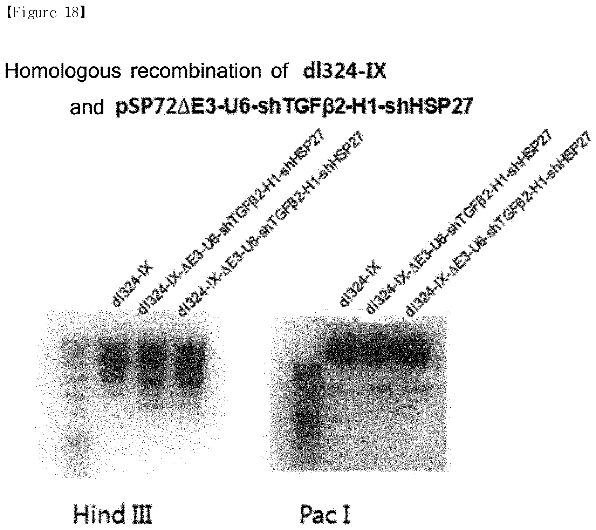

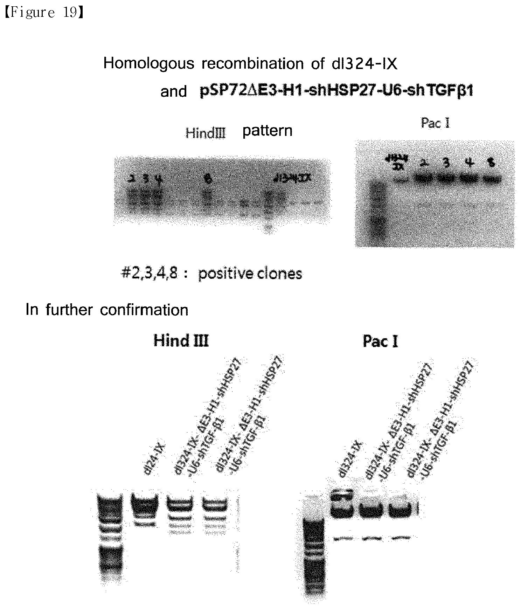

Homologous recombination of the completed pSP72-ΔE3-U6-shTGFβ2-H1-shHSP27 and the dl324-IX backbone was performed. Here, homologous recombination was performed by cleaving pSP72-ΔE3-U6-shTGFβ2-H1-shHSP27 as a shuttle vector with XmnI and cleaving the backbone dl324-IX with SpeI (FIG. 18). In the same manner as described above, homologous recombination of pSP72-ΔE3-H1-shHSP27-U6-shTGFβ1 and the dl324-IX backbone was performed. Here, homologous recombination was performed by cleaving pSP72-ΔE3-H1-shHSP27-U6-shTGFβ1 as a shuttle vector with XmnI and cleaving the backbone dl324-IX with SpeI (FIG. 19).

DNA obtained from a bacterial clone in which homologous recombination had been identified was cleaved with PacI, and introduced into a 293A (a subclone of the 293 human embryonic kidney cell line) cell line for transfection, thereby producing an adenovirus. The purified adenovirus obtained by proliferation in the 293 cell line, isolation according to a CsCl concentration gradient by ultracentrifugation and dialysis was analyzed using a standard plaque assay kit developed by Qbiogene (Carlsbad, CA, USA) to estimate a titer. The final viral titer was 1x10¹⁰ to 1x10¹¹.

### Preparation Example 8: Construction of replication-deficient adenoviruses that simultaneously express shTGFβ and shHSP25 (mouse)(dl324-H1-shHSP25-U6-mshTGFβ1 and dl324-U6-mshTGFβ2-H1-shHSP25)

### 1) Construction of pSP72ΔE3-U6-murine shTGFβ1

A vector was constructed by the same method described in Preparation Example 7 1), except that murine shTGFβ1, instead of human shTGFβ1, was used.

### 2) Construction of pSP72ΔE3-U6-murine shTGFβ2

A vector was constructed by the same method described in Preparation Example 7 2), except that murine shTGFβ2, instead of human shTGFβ2, was used.

### 3) Construction of pSP72ΔE3-H1-shHSP25

A vector was constructed by the same method described in Preparation Example 7 3), except that murine HSP25, instead of human shHSP27, was used.

### 4) pSP72ΔE3-H1-shHSP25-U6-mshTGFβ1 subcloning

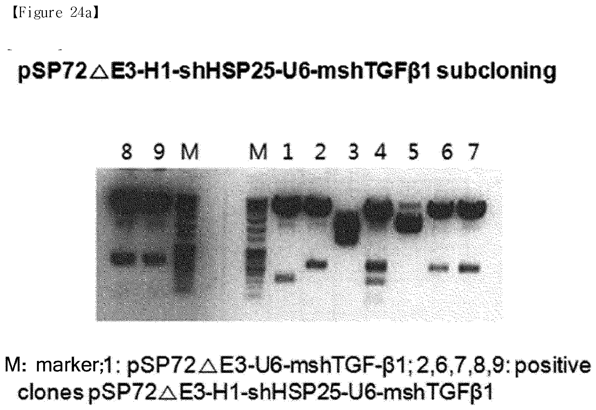

Blunting was performed by cleaving pSP72ΔE3-U6-murine shTGFβ1 with HindIII and cleaving pSP72ΔE3-H1-shHSP25 with SphI. In addition, pSP72ΔE3-H1-mshHSP25-U6-mshTGFβ1 was obtained by cleaving the resulting products with KpnI and ligating the cleaved products (FIG. 24A).

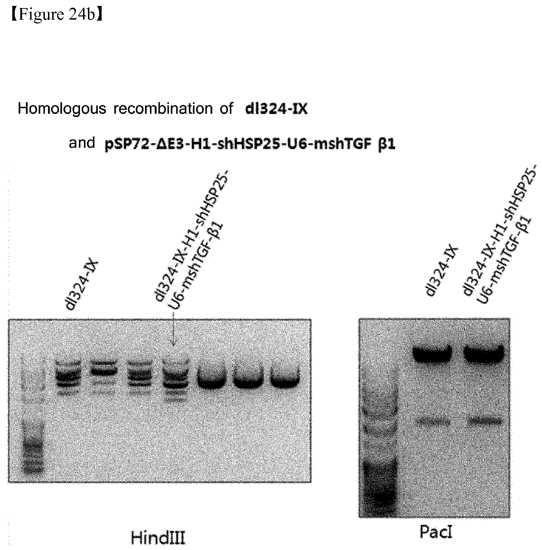

Homologous recombination of the completed pSP72-ΔE3-H1-shHSP25-U6-mshTGFβ1 and the dl324-IX backbone was performed. Here, homologous recombination was performed by cleaving pSP72-ΔE3-H1-mshHSP25-U6-mshTGFβ1 as a shuttle vector with XmnI and cleaving the backbone dl324-IX with SpeI (FIG. 24B).

Through the homologous recombination described above, dl324-H1-shHSP25-U6-mshTGFβ1 was constructed.

By the same method as described above, homologous recombination of pSP72-ΔE3-U6-mTGFβ2-H1-mHSP25 and the dl324-IX backbone was performed. Here, homologous recombination was performed by cleaving pSP72-ΔE3-U6-TGFβ2-H1-HSP25 as a shuttle vector with XmnI and cleaving the backbone dl324-IX with Spel.

Through the homologous recombination described above, dl324-U6-mshTGFβ2-H1-shHSP25 was constructed.

### Preparation Example 9: Construction of oncolytic adenoviruses that simultaneously express shTGFβ and shHSP27 (human) (dl324-3484-CMVp-ΔE1B-H1-shHSP27-U6-shTGFβ1 and dl324-3484-CMVp-ΔE1B-U6-shTGFβ2-H1-shHSP27)

An oncolytic adenovirus was constructed as follows.

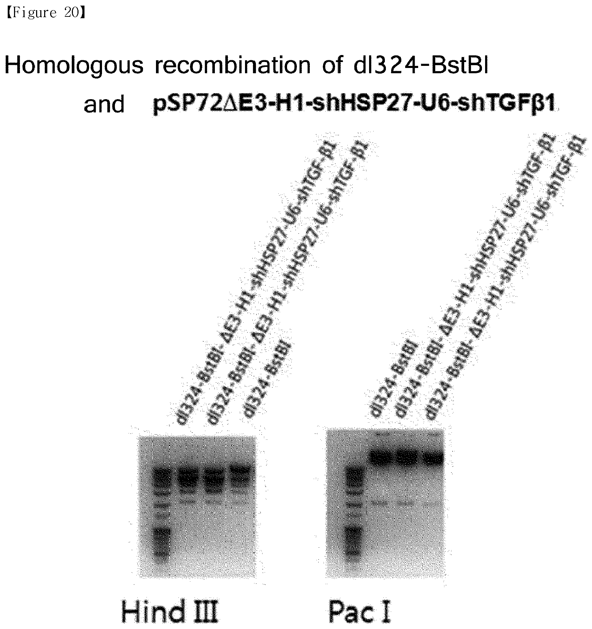

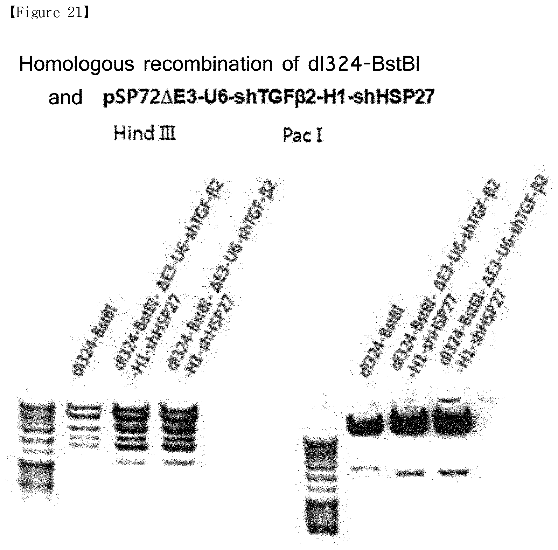

First, homologous recombination of dl324-BstBI and pSP72ΔE3-H1-shHSP27-U6-shTGFβ1 (FIG. 20) and homologous recombination of dl324-BstBI and pSP72ΔE3-U6-shTGFβ2-H1-HSP27 (FIG. 21) were performed.

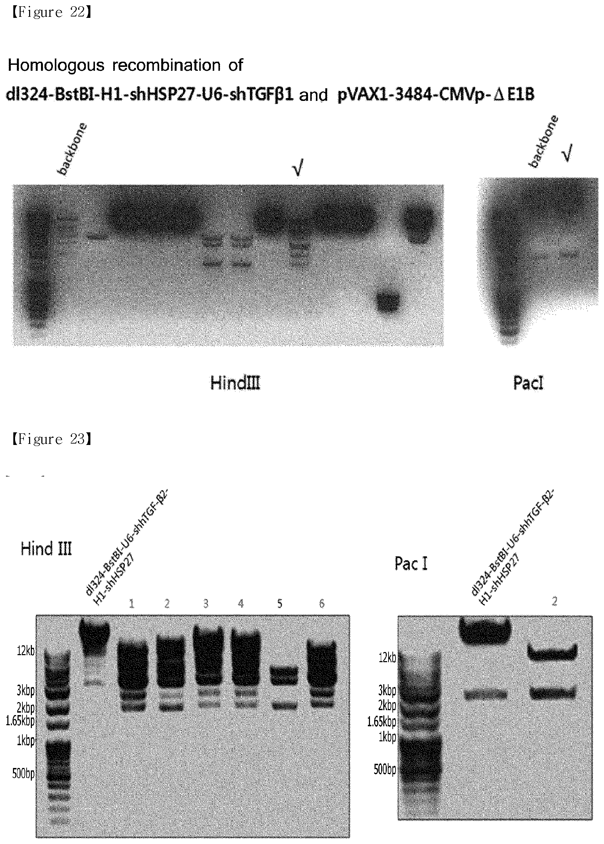

Second, homologous recombination was performed again between dl324-BstBI-H1-shHSP27-U6-shTGFβ1 (FIG. 20) or dl324-BstBI-U6-shTGFβ2-H1-shHSP27 (FIG. 21) and pVAX1-3484-CMVp-ΔE1B as an E1 shuttle vector, thereby constructing dl324-3484-CMVp-ΔE1B-H1-shHSP27-U6-shTGFβ1 (FIG. 22) or dl324-3484-CMVp-ΔE1B-U6-shTGFβ2-H1-shHSP27(FIG. 23). The pVAX1-3484-CMVp-ΔE1B as the E1 shuttle vector was cleaved with PmeI for linearization, and the dl324-BstBI-H1-shHSP27-U6-shTGFβ1 or dl324-BstBI-U6-shTGFβ2-H1-shHSP27 as the backbone DNA was cleaved with Bsp1191 for linearization, and then homologous recombinant DNA was identified from a band of approximately 2 kb through a HindIII pattern and PacI cleavage for colonies obtained by transforming BJ5183 with two types of the linearized DNA.

Through the homologous recombination described above, dl324-3484-CMVp-ΔE1B-H1-shHSP27-U6-shTGFβ1 or dl324-3484-CMVp-ΔE1B-U6-shTGFβ2-H1-shHSP27 was constructed.

### Preparation Example 10: Construction of oncolytic adenovirus that simultaneously expresses shTGFβ1 and shHSP25 (mouse) (dl324-3484-CMVp-ΔE1B-H1-shHSP25-U6-mshTGFβ1)

A process of homologous recombination of dl324-3484-CMVp-ΔE1B-H1-shHSP25-U6-mshTGFβ1 to construct a tumor-selective adenovirus that expresses mouse type shRNA is as follows.

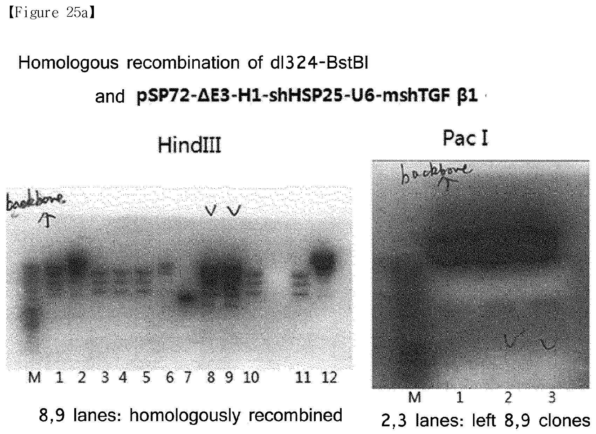

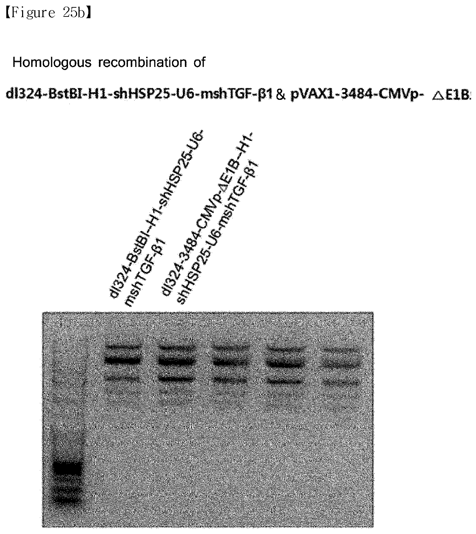

Homologous recombination was performed by cleaving the pSP72ΔE3-H1-shHSP25-U6-mshTGFβ1 obtained in Preparation Example 8 as a shuttle vector with XmnI and cleaving the backbone dl324-BstBI with Spel, thereby constructing dl324-BstBI-H1-shHSP25-U6-mshTGFβ1 (FIG. 25A). Afterward, homologous recombination was performed by cleaving pVAX1-3484-CMVp-ΔE1B as a shuttle vector with PmeI and cleaving the backbone dl324-BstBI-H1-shHSP25-U6-mshTGFβ1 with Bsp1191, thereby constructing dl324-3484-CMVp-ΔE1B-H1-shHSP25-U6-mshTGFβ1 (FIG. 25B).

### Preparation Example 11: Construction of GM-CSF, Flt3L-TRAIL and shTGF-β-loaded oncolytic adenovirus (human) (dl324-3484-CMVp-ΔE1B-GMCSF-IRES-Flt3L-TRAIL-U6-shTGF-β1)

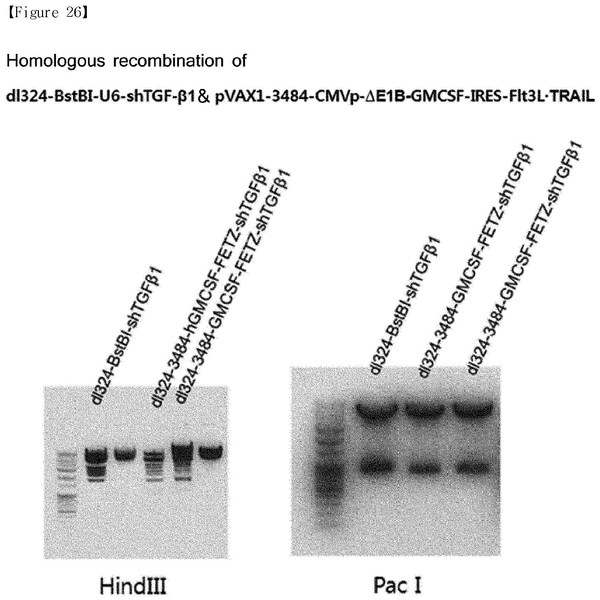

For viral production, a viral backbone dl324-BstBI-U6-shTGF-β1 was obtained through homologous recombination of the pSP72ΔE3-U6-shTGFβ1 described in Preparation Example 7 and dl324-BstBI. The homologous recombination was induced through transformation of *E. coli* BJ5183 by cleaving the dl324-BstBI-U6-shTGF-β1 with Bsp1191 and linearizing the pVAX1-3484-CMVp-ΔE1B-GMCSF-IRES-Flt3L-TRAIL obtained in Preparation Example 6 with PmeI. As a result, the recombination was confirmed with a HindIII pattern and PacI cleavage (FIG. 26).

Through the homologous recombination described above, dl324-3484-CMVp-ΔE1B-GMCSF-IRES-Flt3L-TRAIL-U6-shTGF-β1 was constructed.

### Preparation Example 12: Construction of mGM-CSF, Flt3L-TRAIL and mshTGF-β1-loaded oncolytic adenovirus (mouse) (dl324-3484-CMVp-ΔE1B-mGMCSF-IRES-Flt3L-TRAIL-U6-mshTGF-β1)

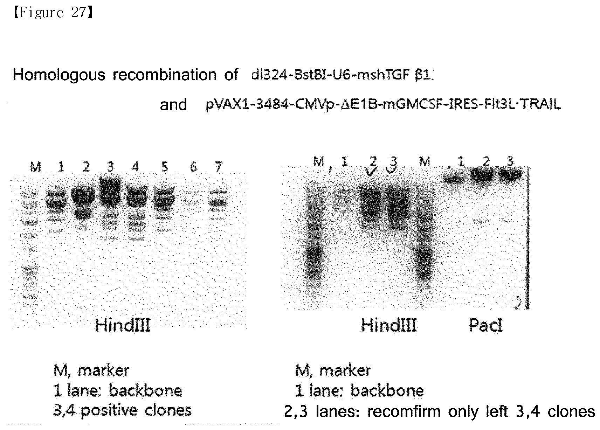

For viral production, a viral backbone dl324-BstBI-U6-mshTGF-β1 was obtained through homologous recombination of the pSP72ΔE3-U6-mshTGFβ1 described in Preparation Example 8 and dl324-BstBI. For homologous recombination of the dl324-BstBI-U6-mshTGF-β1 obtained thereby and the pVAX1-3484-CMVp-ΔE1B-mGMCSF-IRES-Flt3L-TRAIL obtained in Preparation Example 6, the pVAX1-3484-CMVp-ΔE1B-mGMCSF-IRES-Flt3L-TRAIL (FIG. 12B) as a shuttle vector was cleaved with PmeI, and the backbone dl324-BstBI-U6-mshTGFβ1 was cleaved with Bsp1191 (FIG. 27).

Through the homologous recombination described above, dl324-3484-CMVp-ΔE1B-mGMCSF-IRES-Flt3L-TRAIL-U6-mshTGF-β1 was constructed.

### Preparation Example 13: Construction of mGM-CSF, Flt3L-TRAIL and shHSP27-loaded oncolytic adenovirus (human) (dl324-3484-CMVp-ΔE1B-GMCSF-IRES-Flt3L-TRAIL-H1-shHSP27)

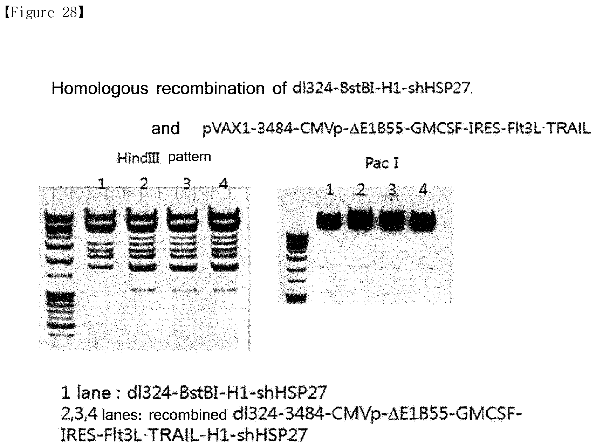

For viral production, a viral backbone dl324-BstBI-H1-shHSP27 was obtained through homologous recombination of the pSP72ΔE3-H1-shHSP27 described in Preparation Example 7 and dl324-BstBI. The homologous recombination was induced through transformation of *E. coli* BJ5183 by cleaving the dl324-BstBI-H1-shHSP27 obtained thereby with Bsp1191 and linearizing the pVAX1-3484-CMVp-ΔE1B-GMCSF-IRES-Flt3L-TRAIL obtained in Preparation Example 6 with PmeI. As a result, successful recombination in Lanes 1, 2 and 3 were confirmed with a HindIII pattern and PacI cleavage (FIG. 28).

Through the homologous recombination described above, dl324-3484-CMVp-ΔE1B-GMCSF-IRES-Flt3L-TRAIL-H1-shHSP27 was constructed.

### Preparation Example 14: Construction of GM-CSF, Flt3L-TRAIL and shHSP25-loaded oncolytic adenovirus (mouse) (dl324-3484-CMVp-ΔE1B-GMCSF-IRES-Flt3L-TRAIL-H1-shHSP25)

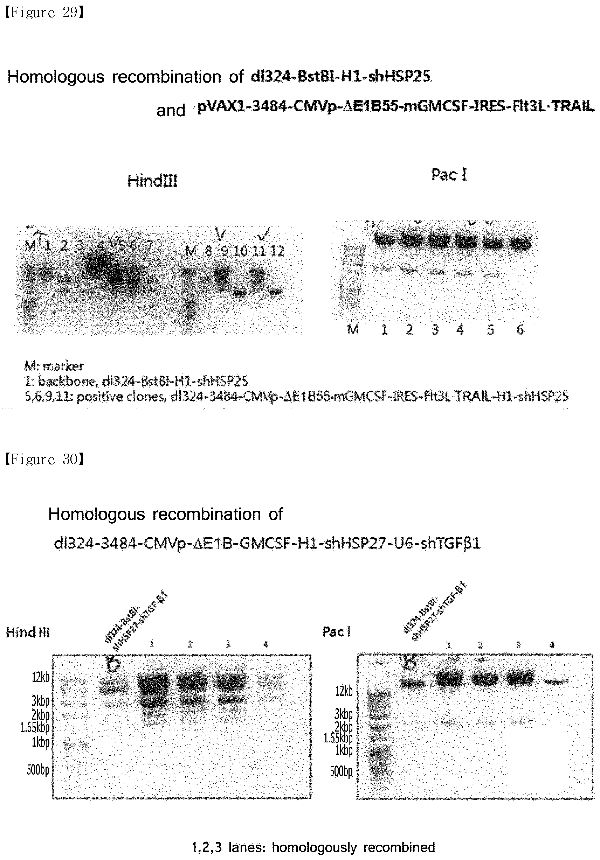

For viral production, a viral backbone dl324-BstBI-H1-shHSP25 was obtained through homologous recombination of the pSP72ΔE3-H1-shHSP25 described in Preparation Example 8 and dl324-BstBI. The dl324-BstBI-H1-shHSP25 obtained thereby was cleaved with Bsp1191, and for homologous recombination with the pVAX1-3484-CMVp-ΔE1B-mGMCSF-IRES-Flt3L-TRAIL obtained in Preparation Example 6, pVAX1-3484-CMVp-ΔE1B-mGMCSF-IRES-Flt3L-TRAIL as a shuttle vector was cleaved with PmeI, and the backbone dl324-BstBI-mshHSP25 was cleaved with Bspl1191 (FIG. 29).

Through the homologous recombination described above, dl324-3484-CMVp-ΔE1B-GMCSF-IRES-Flt3L-TRAIL-H1-shHSP25 was constructed.

### Preparation Example 15: Construction of GM-CSF, shHSP27 and shTGF-β-loaded oncolytic adenoviruses (human) (dl324-3484-CMVp-ΔE1B-GMCSF-H1-shHSP27-U6-shTGFβ1 and dl324-3484-CMVp-ΔE1B-GMCSF-U6-shTGFβ2-H1-shHSP27)

A pVAX1-3484-CMVp-ΔE1B-GMCSF shuttle vector was constructed by subcloning an insert prepared by blunting pCA14-GMCSF with BglII and performing cleavage with SalI into pVAX1-3484-CMVp-ΔE1B which was blunted by cleavage with EcoRI and performing cleavage with SalI (refer to Preparation Example 1 disclosed in Korean Patent No. 2015-0044507).

Homologous recombination to dl324-3484-CMVp-ΔE1B-GMCSF-H1-shHSP27-U6-shTGFβ1 was identified by selecting colonies obtained through transformation of BJ5183 after the pVAX1-3484-CMVp-AE1B-GMCSF as an E1 shuttle vector was linearized by cleavage with PmeI, and the dl324-BstBI-H1-shHSP27-U6-shTGFβ1 obtained in Preparation Example 9 as backbone DNA was linearized by cleavage with Bsp1191 using a HindIII pattern and PacI cleavage (FIG. 30).

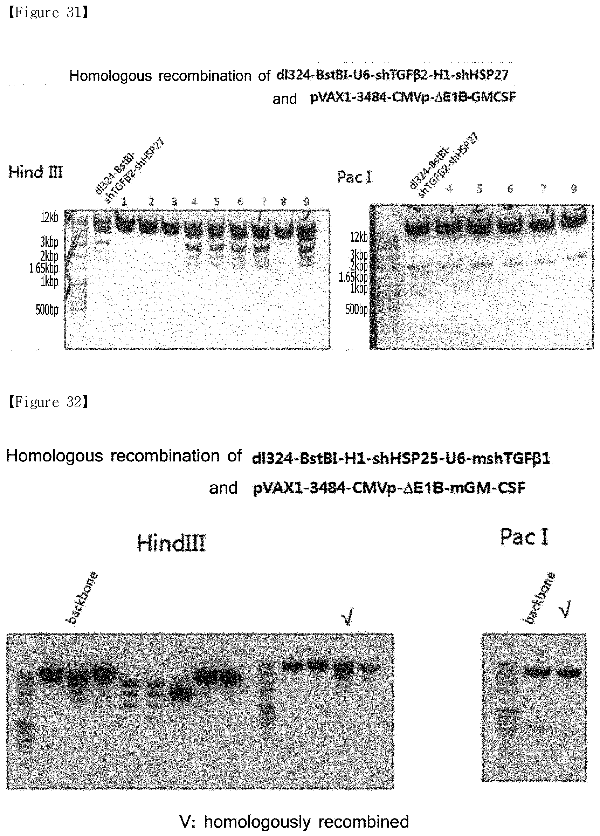

Homologous recombination to dl324-3484-CMVp-ΔE1B-GMCSF-U6-shTGFβ2-H1-shHSP27 was identified by selecting colonies obtained through transformation of BJ5183 after the pVAX1-3484-CMVp-AE1B-hGMCSF as an E1 shuttle vector was linearized by cleavage with PmeI, and the dl324-BstBI-U6-shTGFβ2-H1-shHSP27 obtained in Preparation Example 9 as backbone DNA was linearized by cleavage with Bsp1191 using a HindIII pattern and PacI cleavage (FIG. 31).

### Preparation Example 16: Construction of GM-CSF, shHSP25 and shTGF-β1-loaded oncolytic adenovirus (mouse) (dl324-3484-CMVp-ΔE1B-GMCSF-H1-shHSP25-U6-mshTGFβ1)

Homologous recombination to dl324-3484-CMVp-ΔE1B-mGMCSF-shHSP25-mshTGFβ1 was identified by selecting colonies obtained through transformation of BJ5183 after the pVAX1-3484-CMVp-ΔE1B-mGMCSF (refer to Korean Patent No. 2015-0044507 based on Preparation Example 1) as an E1 shuttle vector was linearized by cleavage with PmeI, and the backbone DNA dl324-BstBI-H1-shHSP25-U6-mshTGFβ1 obtained through homologous recombination of the pSP72-ΔE3-H1-shHSP25-U6-mshTGFβ1 obtained in Preparation Example 8 and dl324-BstBI was linearized by cleavage with Bsp1191 using a HindIII pattern and PacI cleavage (FIG. 32).

Through the homologous recombination described above, dl324-3484-CMVp-ΔE1B-GMCSF-H1-shHSP25-U6-mshTGFβ1 was constructed.

### Preparation Example 17: Construction of Flt3L-TRAIL, shTGF-β and shHSP27-loaded oncolytic adenoviruses (human) (dl324-3484-CMVp-ΔE1B-Flt3L-TRAIL-H1-shHSP27-U6-shTGFβ1, and dl324-3484-CMVp-ΔE1B-Flt3L-TRAIL-U6-shTGFβ2-H1-shHSP27)

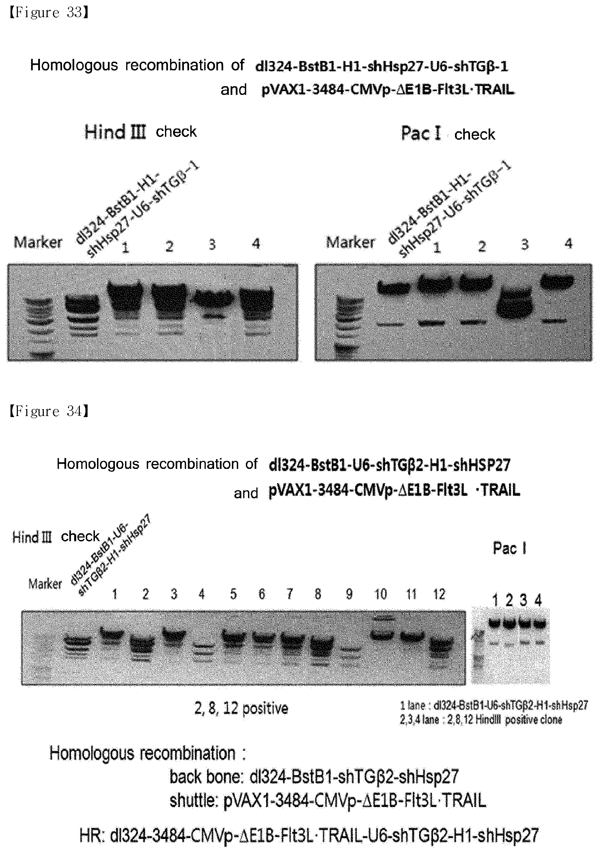

Homologous recombination of the shuttle vector (pVAX1-3484-CMVp-ΔE1B-Flt3L-TRAIL) obtained in Preparation Example 2, and the backbone dl324-BstB1-H1-shHSP27-U6-shTGFβ1 or dl324-BstB1-U6-shTGFβ2-H1-shHSP27, obtained in Preparation Example 9 was performed (FIGS. 33 and 34).

DNA obtained from a bacterial clone in which homologous recombination had been identified was cleaved with PacI, and then introduced into a 293A (a subclone of the 293 human embryonic kidney cell line) cell line for transfection, thereby producing an adenovirus. The purified adenovirus obtained by proliferation in the 293 cell line, isolation according to a CsCl concentration gradient by ultracentrifugation and dialysis was analyzed using a standard plaque assay kit developed by Qbiogene (Carlsbad, CA, USA) to estimate a titer. The final virus titer was 1x10¹² to 5x10¹².

### Preparation Example 18: Construction of Flt3L-TRAIL, shHSP25 and shTGF-β1-loaded oncolytic adenovirus (mouse) (dl324-3484-CMVp-ΔE1B-Flt3L-TRAIL-H1-shHSP25-U6-mshTGFβ1)

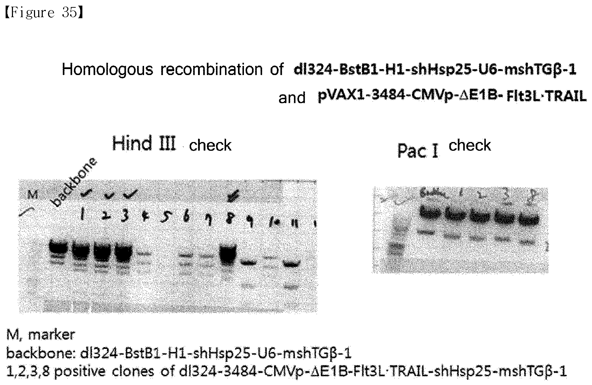

For viral production, homologous recombinant DNA was produced through transformation of *E. coli* BJ5183 by cleaving the backbone DNA dl324-BstBI-H1-shHSP25-U6-mshTGFβ 1(Preparation Example 9) obtained through homologous recombination of the pSP72-ΔE3-H1-shHSP25-U6-mshTGFβ1 (Preparation Example 8) as a viral backbone and dl324-BstBI with Bsp1191, and linearizing the pVAX1-3484-CMVp-ΔE1B-Flt3L-TRAIL obtained in Preparation Example 2 using PmeI. As a result, recombination was confirmed using a HindIII pattern and PacI cleavage (FIG. 35).

Through the homologous recombination described above, dl324-3484-CMVp-ΔE1B-Flt3L-TRAIL-H1-shHSP25-U6-mshTGFβ1 was constructed.

### Preparation Example 19: Construction of GM-CSF, Flt3L-TRAIL, shTGF-β and shHSP27-loaded oncolytic adenoviruses (human) (dl324-3484-CMVp-ΔE1B-GMCSF-IRES-Flt3L-TRAIL-H1-shHSP27-U6-shTGFβ1, and dl324-3484-CMVp-ΔE1B-GMCSF-IRES-Flt3L-TRAIL-U6-shTGFβ2-H1-shHSP27)

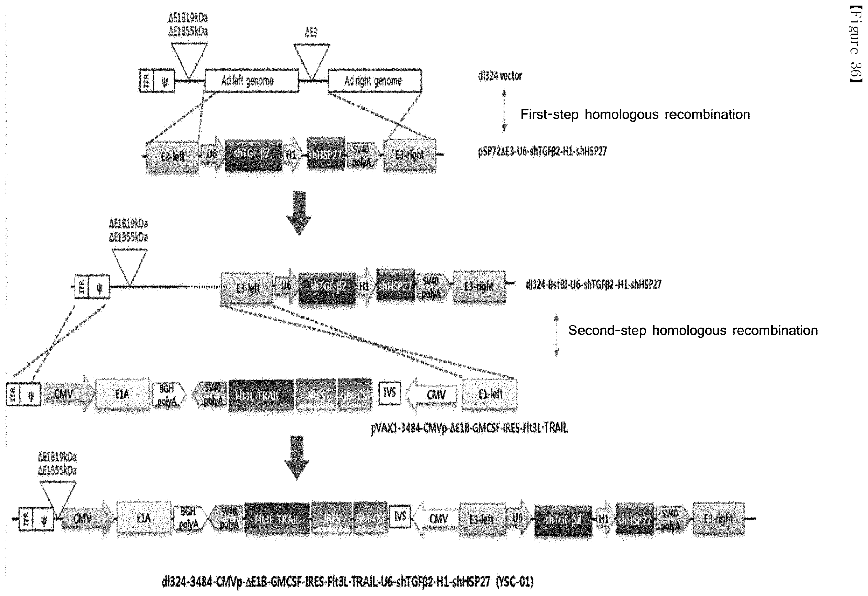

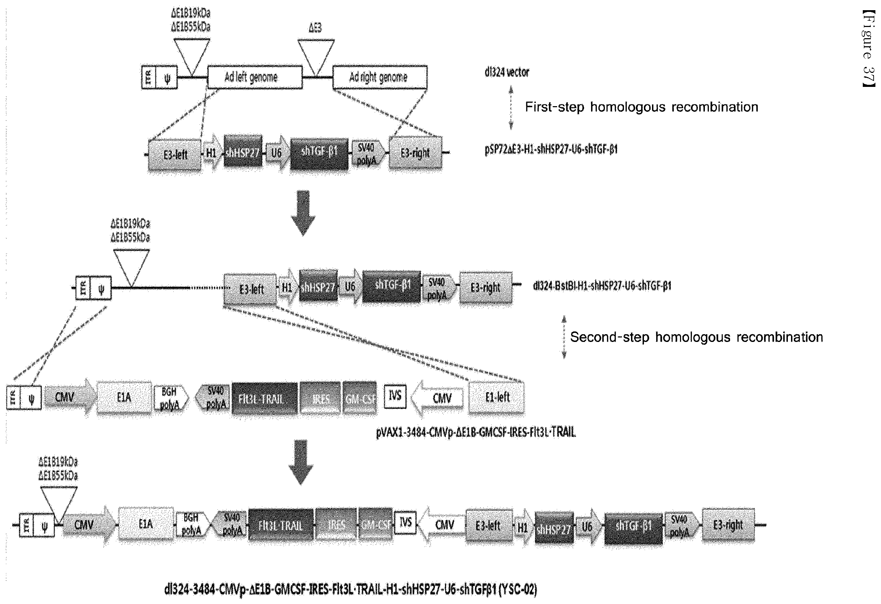

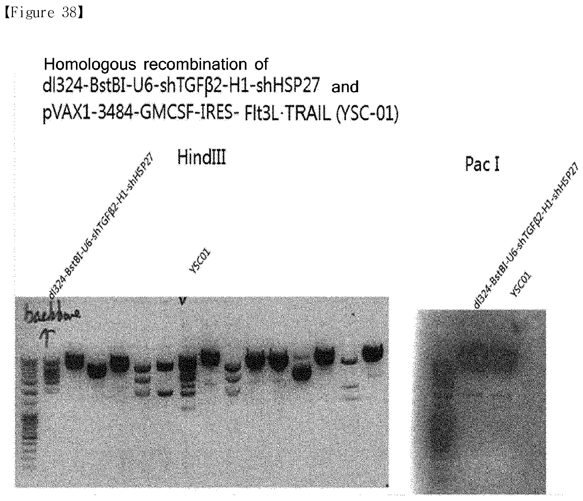

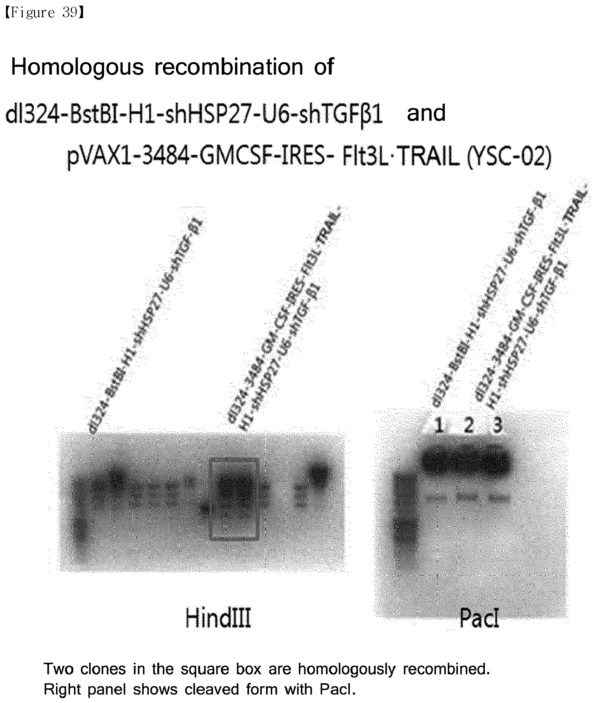

Through homologous recombination of the dl324-BstBI-ΔE3-U6-TGF-β2-H1-HSP27 or dl324-BstBI-ΔE3-H1-HSP27-U6-TGF-β1 obtained in Preparation Example 9 and the pVAX1-3484-CMVp-ΔE1B-GMCSF-IRES-Flt3L-TRAIL obtained in Preparation Example 6, dl324-3484-CMVp-ΔE1B-GMCSF-IRES-Flt3L-TRAIL-U6-shTGFp2-H1-shHSP27 (YSC-01) (FIGS. 36 and 38) or dl324-3484-CMV-ΔE1B-GMCSF-IRES-Flt3L-TRAIL-H1-shHSP27-U6-shTGFβ1 (YSC-02) was constructed (FIGS. 37 and 39).

DNA obtained from a bacterial clone in which homologous recombination had been confirmed was cleaved with PacI, and introduced into a 293A (a subclone of the 293 human embryonic kidney cell line) cell line for transfection, thereby producing an adenovirus. The purified adenovirus obtained by proliferation in the 293 cell line, isolation according to a CsCl concentration gradient by ultracentrifugation and dialysis was analyzed using a standard plaque assay kit developed by Qbiogene (Carlsbad, CA, USA) to estimate a titer. The final virus titer was 1x10¹² to 5x10¹².

### Preparation Example 20: Construction of GM-CSF, Flt3L-TRAIL, shTGF-β and shHSP25-loaded oncolytic adenovirus (mouse) (dl324-3484-CMVp-ΔE1B-mGMCSF-IRES-Flt3L-TRAIL-H1-shHSP25-U6-mshTGFβ1)

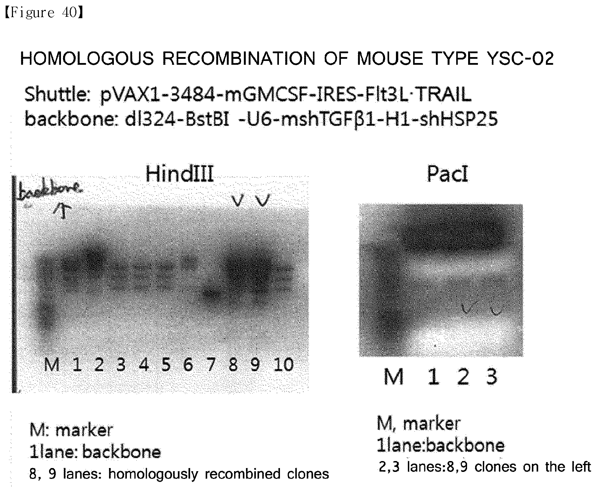

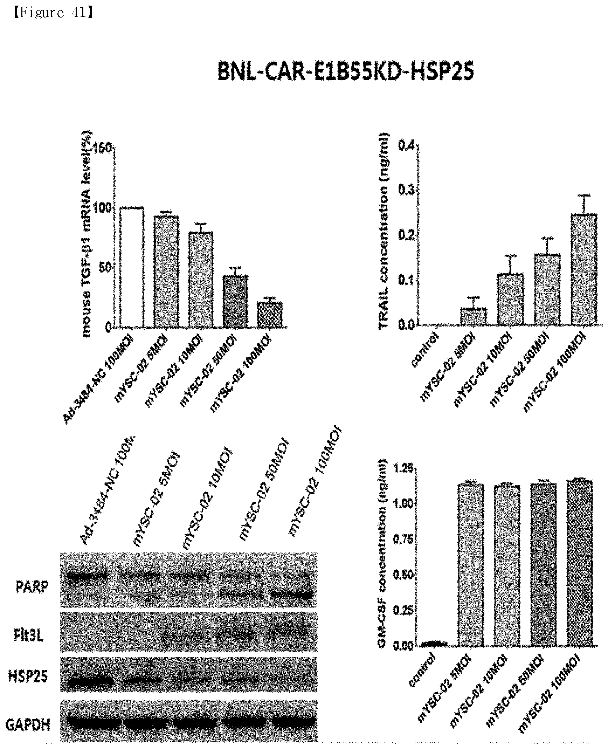

For homologous recombination of the dl324-BstBI-H1-mshHSP25-U6-mshTGFβ1 obtained in Preparation Example 10 and the pVAX1-3484-CMVp-ΔE1B-mGMCSF-Flt3L-TRAIL obtained in Preparation Example 6, a shuttle vector pVAX1-3484-CMVp-ΔE1B-mGMCSF-F1t3L-TRAIL was cleaved with PmeI, and backbone DNA dl324-BstBI-H1-shHSP25-U6-mshTGFβ1 was cleaved with Bsp1191, followed by confirming recombination (FIGS. 40 and 41).

Through the homologous recombination described above, dl324-3484-CMVp-ΔE1B-mGMCSF-IRES-Flt3L-TRAIL-H1-shHSP25-U6-mshTGFβ1 was constructed.

### Example 1: Antitumor effect of tumor-selective replication-competent virus that expresses each or both of shTGF-β and shHSP27 on in vivo nude mouse

1 x 107 cells/100 µl of cells of a human MDAMB-231-Her2 cell line were injected into an abdominal wall, and infected through intratumoral injection of each of adenoviruses (control groups: control virus, oncolytic NC, one type of oncolytic shTGF-β1 of Preparation Example 3, and one type of oncolytic shHSP27 of Preparation Example 4) and two types (shTGF-β1+shHSP27) of Preparation Example 7 three times every two days (1x10⁹ pfu/50 µl) when a tumor size reached 60 mm³. Six mice per experimental group were used.

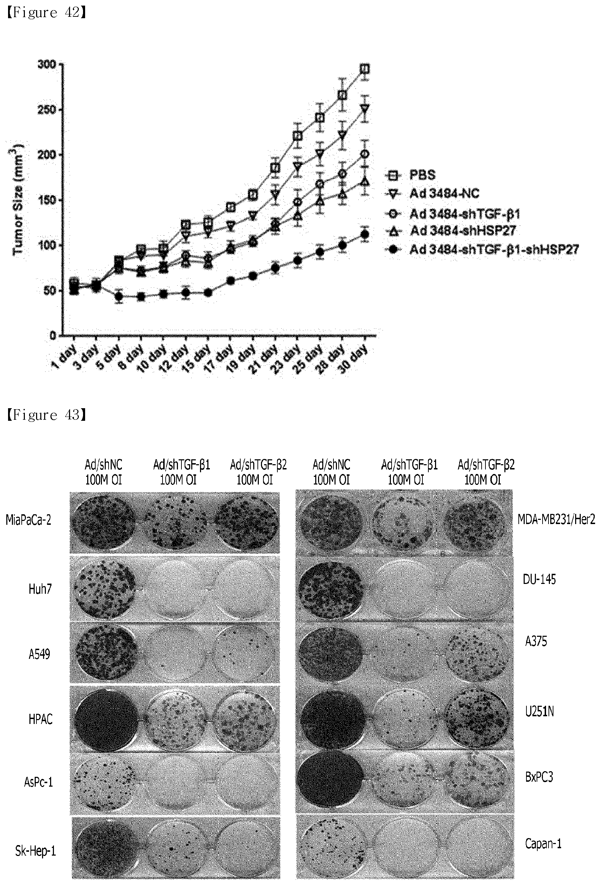

As a result, the oncolytic adenovirus/shTGF-β1+shHSP27 of Preparation Example 7 into which two genes were injected exhibited the most superior antitumor effect (FIG. 42).

### Example 2: Comparison in survival potential according to TGF-β isotype

A cell line that overexpresses Her2 in pancreatic cancer cell lines MiaPaCa-2, HPAC, BxPC3, AsPc-1 and Capan-1, hepatocellular carcinoma cell lines SK-Hep1 and Huh7, a lung cancer cell line A549, a prostatic cancer cell line DU-145, a melanoma cell line A375, a glioma cell line U251N, or a breast cancer cell line MDAMB231 was plated on a 6-well plate at 2x10⁵ cells per well, infected with defective adenovirus/negative control (NC), adenovirus/shTGF-β1 of Preparation Example 3, or adenovirus/shTGF-β2 at 100 MOI, after two days, the cells were detached and dispensed into 6 wells at 1 x 10⁵ cells/well, and then the medium was replaced with a 5% FBS-containing medium every two days. After 10 days, the culture medium was removed, and the cells were fixed with 4% paraformaldehyde and stained with crystal violet to observe the cells.

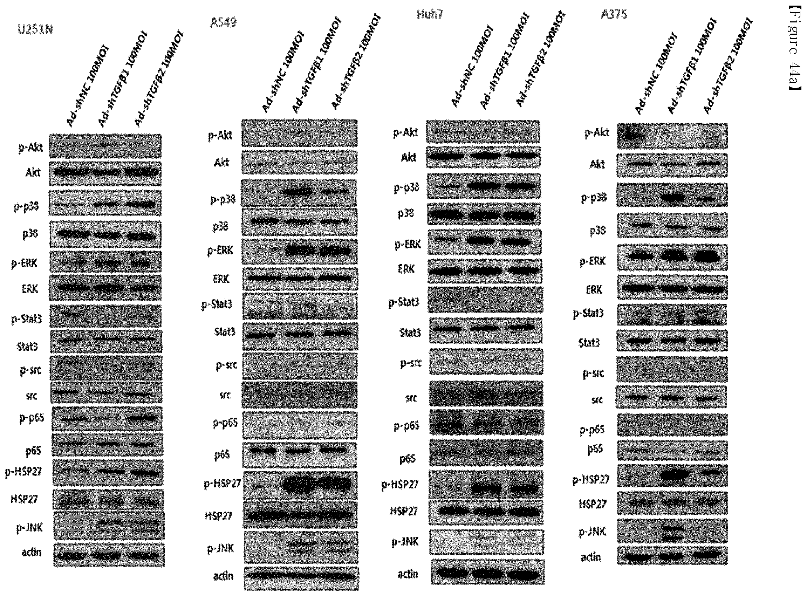

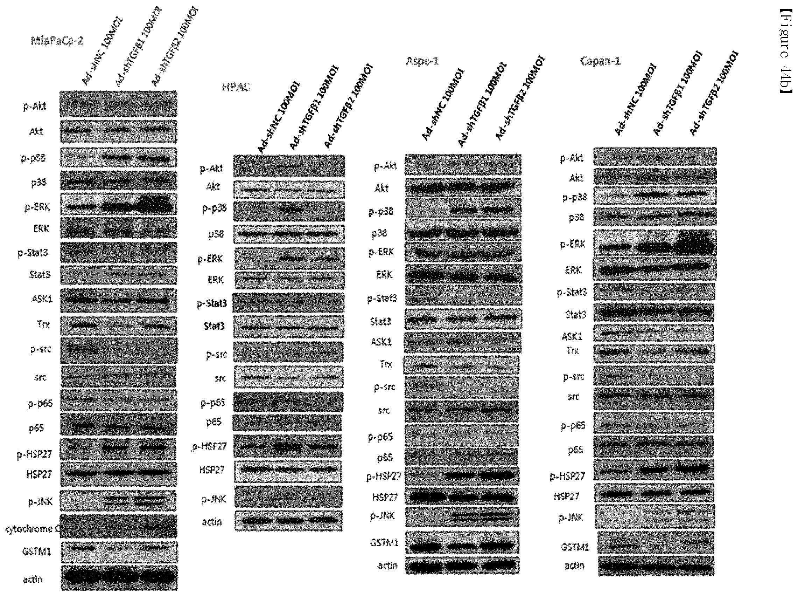

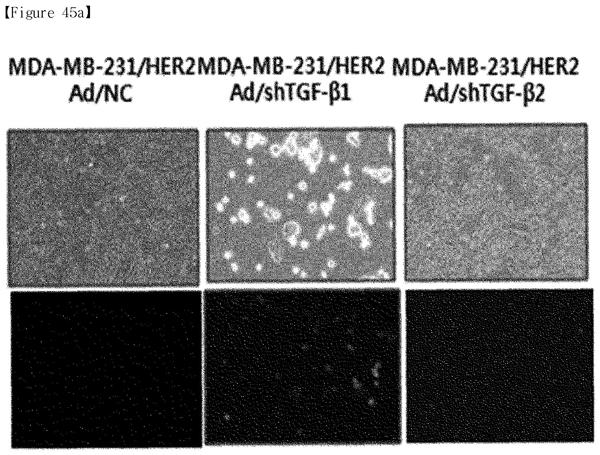

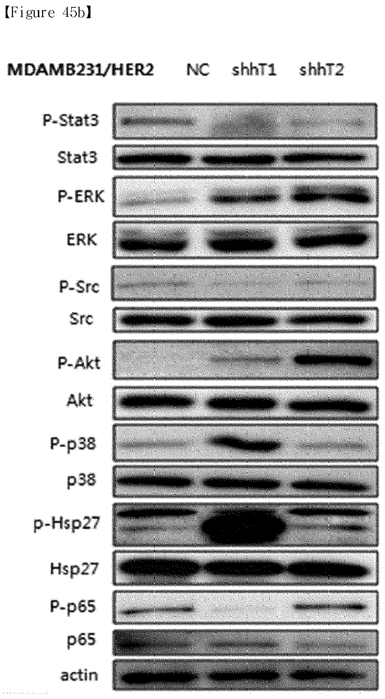

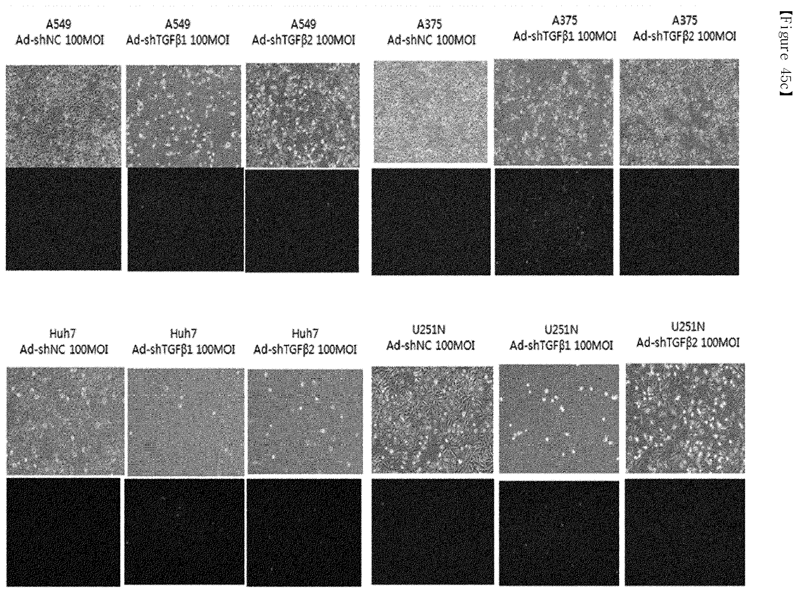

As a result, in most cancer cell lines, an effect of decreasing a survival rate due to a decrease in TGF-β1 was superior to that due to a decrease in TGF-β2, and in most pancreatic cancer cell lines except Capan-1, an effect of decreasing TGF-β was relatively insignificant (FIG. 43). As a result of investigating the effect of decreasing the survival rate of cells with a survival-associated signal and p38 and HSP27 phosphorylation, it can be seen that the survival rate was significantly decreased and the p38 and HSP27 phosphorylation was increased (FIG. 44). In addition, particularly, the effect of decreasing TGF-β1 was also caused by excessive secretion of intracellular ROS, leading to cell death (FIG. 45), and phosphorylation of enzymes involved in cell survival, such as pp65 or pStat3 was decreased upon TGF-β reduction (FIG. 44).

### Example 3: Confirmation of effect of two-type gene delivery system on various types of cancer cell lines

To examine whether, in addition to shTGF-β1, shHSP27 is needed for MDAMB231-Her2, cells were infected with defective adenovirus/negative control (NC), one type of Preparation Example 3 (adenovirus/shTGF-β1), or two types of Preparation Example 7 (adenovirus/shTGFβ1-shHSP27) at 100 MOI, and then western blotting was performed.

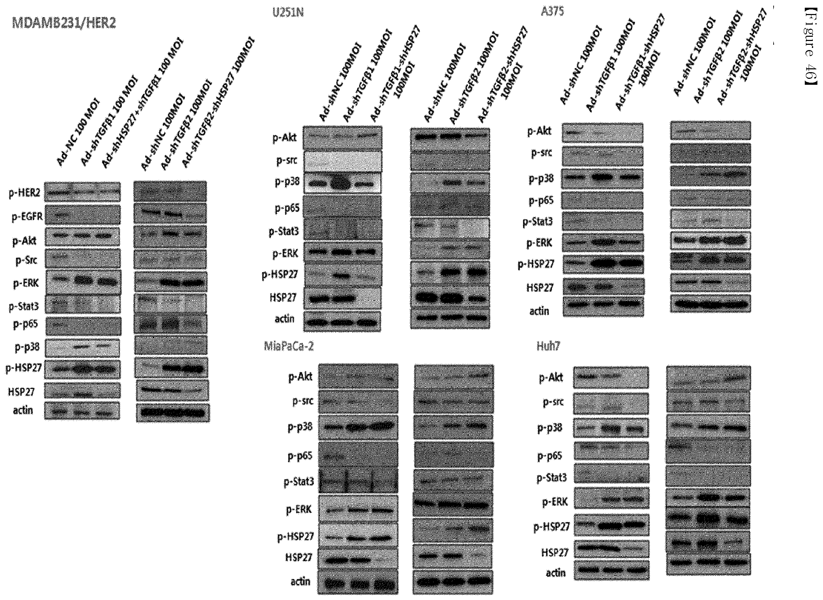

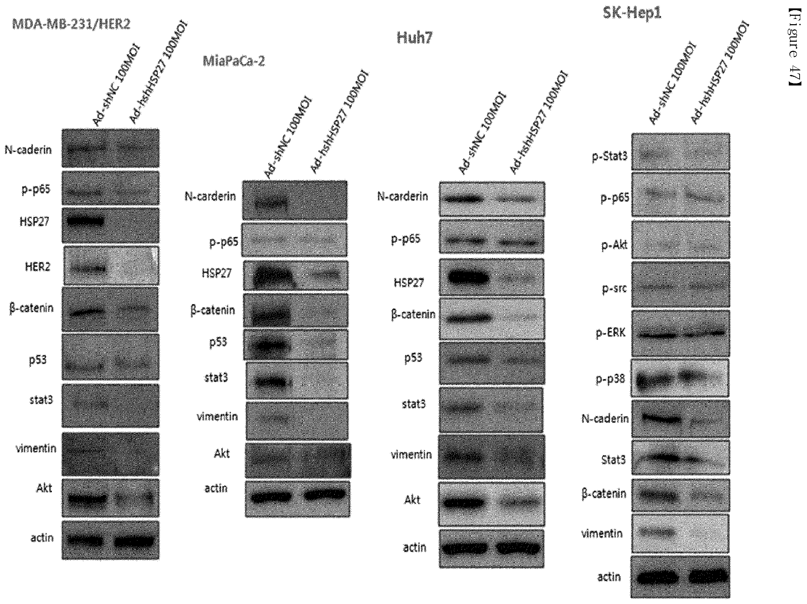

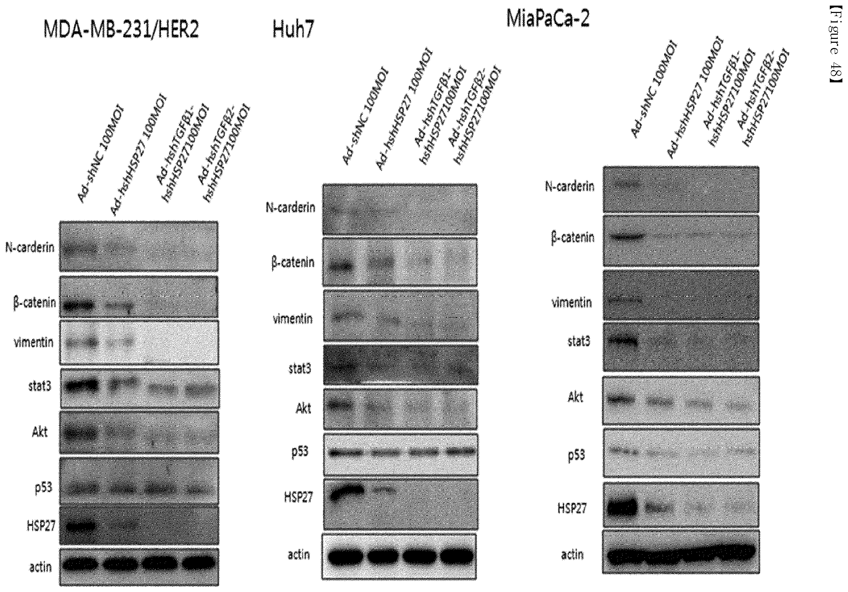

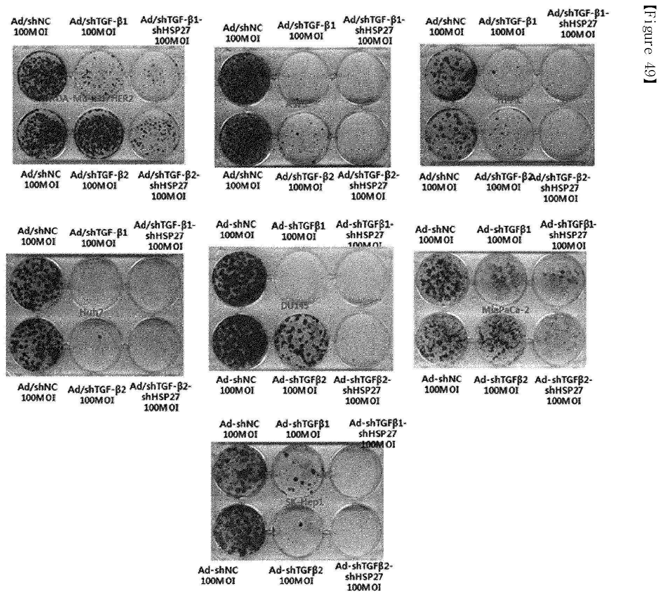

As a result, it was confirmed that, while a decrease in Akt phosphorylation did not occur, other than this, pSTAT3, pSrc and pEGFR were further decreased (FIG. 46), N-cadherin, β-catenin, stat3, vimentin and Akt, which are involved in cancer progression were considerably decreased when only HSP27 was decreased (FIG. 47). In addition, in a simultaneous decrease in TGF-β1 or -β2 and HSP27, an effect of decreasing proteins involved in progression was further maximized (FIG. 48). As a result of comparison in the effect of decreasing a survival rate by a single decrease in TGF-β1 or -β2 or simultaneous decrease in TGF-β1 or -β2/HSP27 through a clonogenic assay, the decrease in survival rate by the simultaneous decrease in two substances was significantly exhibited (FIG. 49).

### Example 4: Confirmation of effects of three-type gene delivery system (adding TRAIL to Example 3) on various types of cancer cell lines

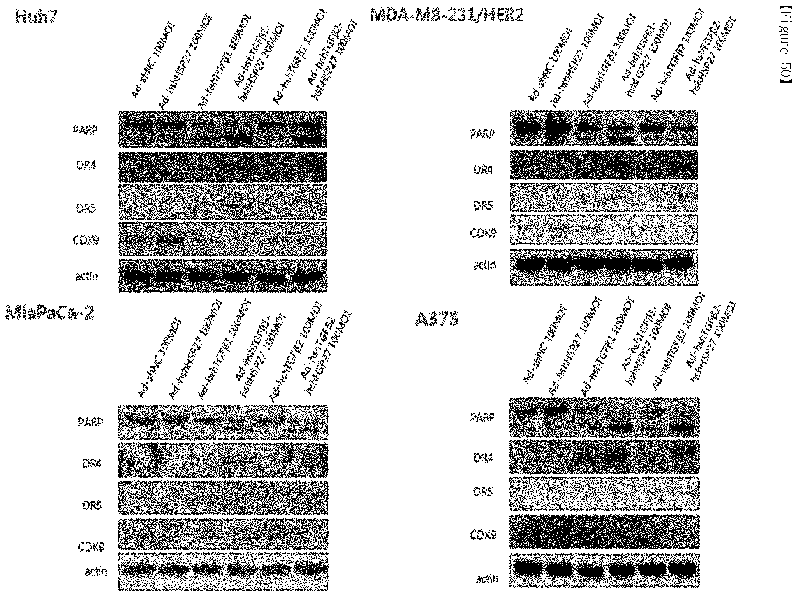

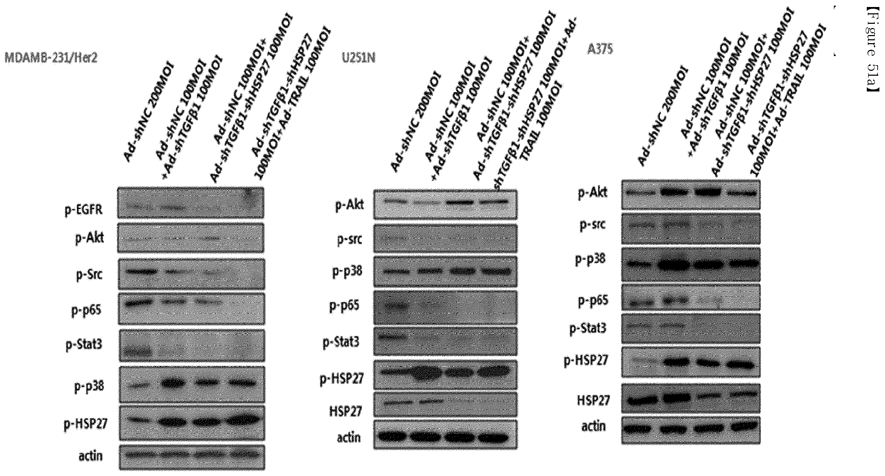

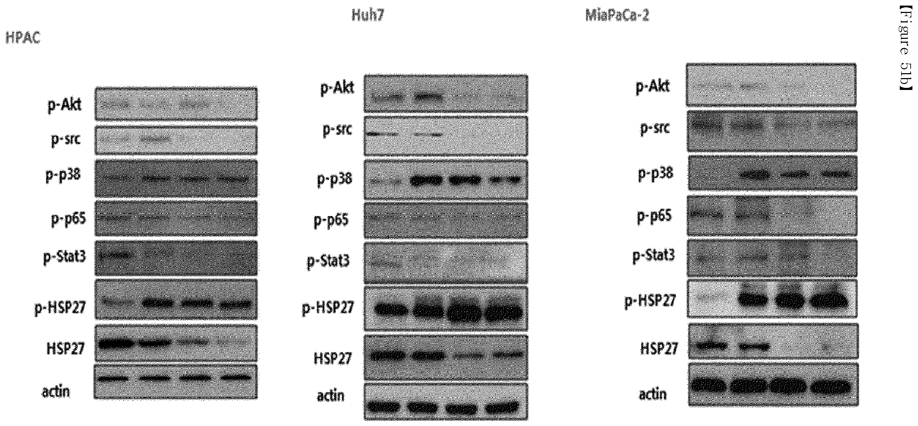

One specific matter is the possibility that increased cytotoxicity by TRAIL is increased such that, when TGF-β and HSP27 are simultaneously decreased, TRAIL receptors such as DR4 and DR5 are increased, and CDK9 which contributes to TRAIL resistance is decreased. FIG. 50 supports this possibility. Actually, in the case of TGF-β/HSP27 reduction and TRAIL expression, it was confirmed that survival-associated signals were further decreased (FIG. 51).

### Example 5: Confirmation of insertion and expression of four types of genes loaded in YSC-01 and YSC-02

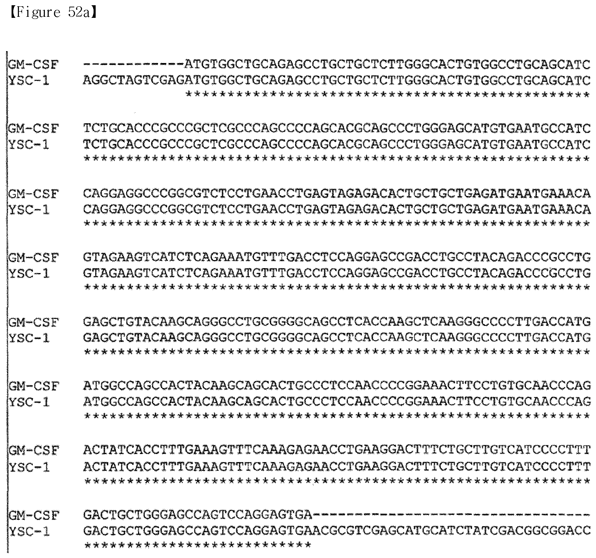

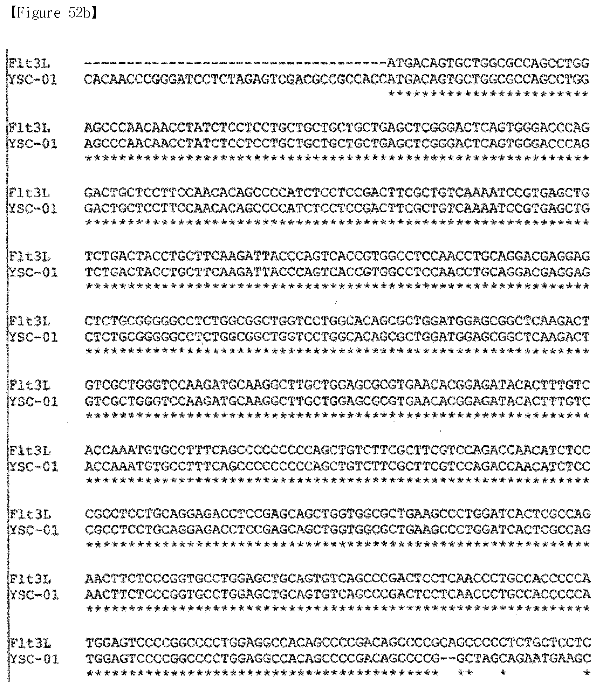

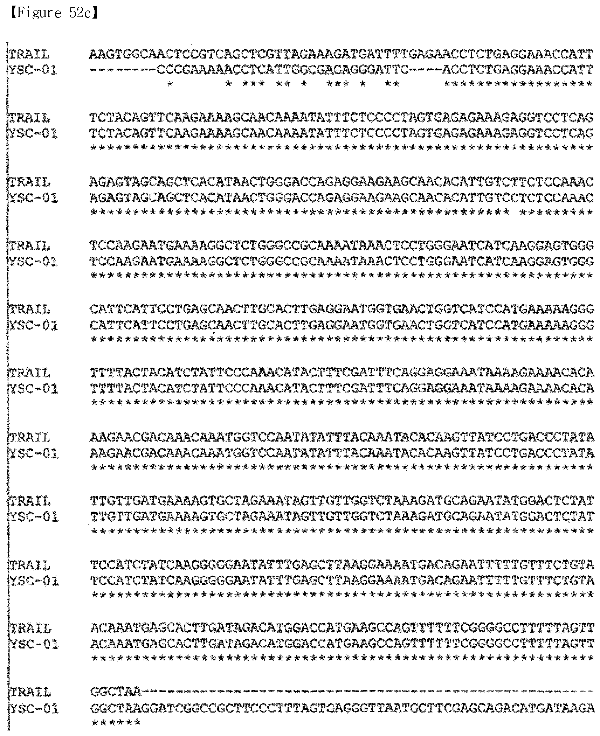

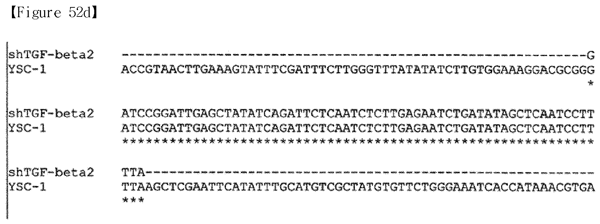

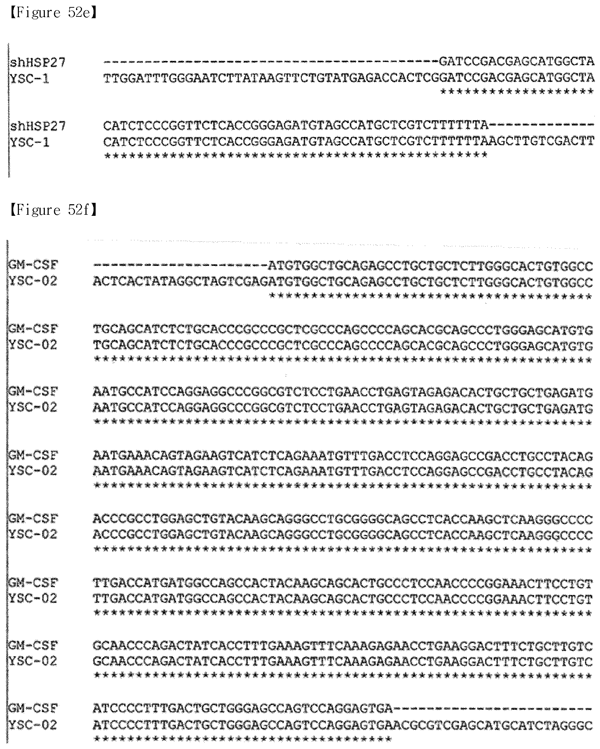

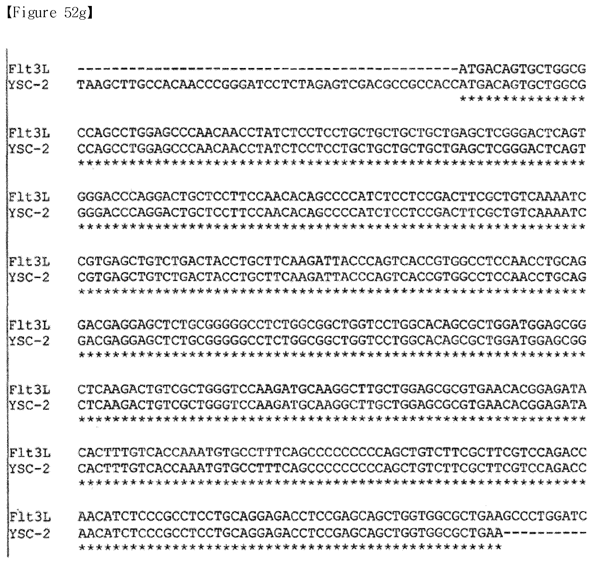

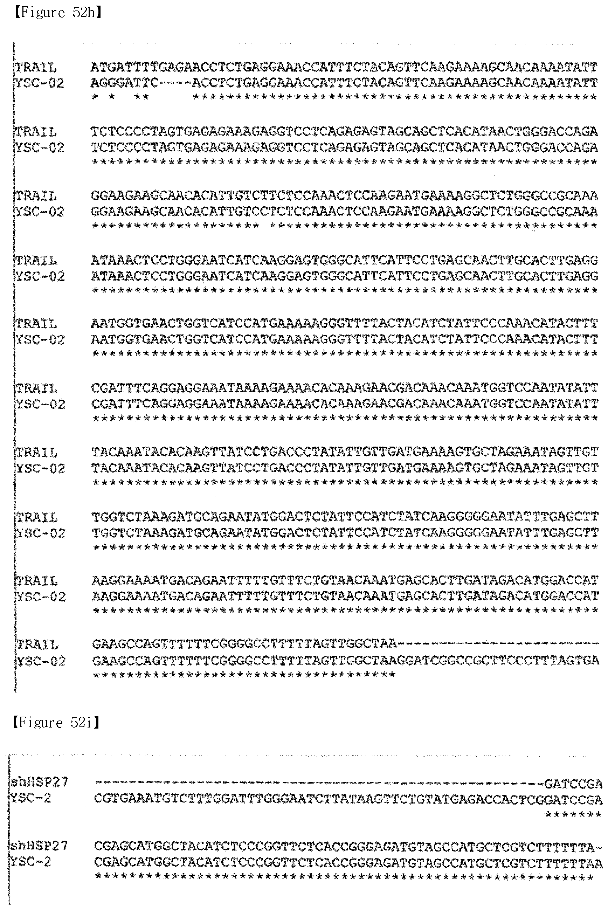

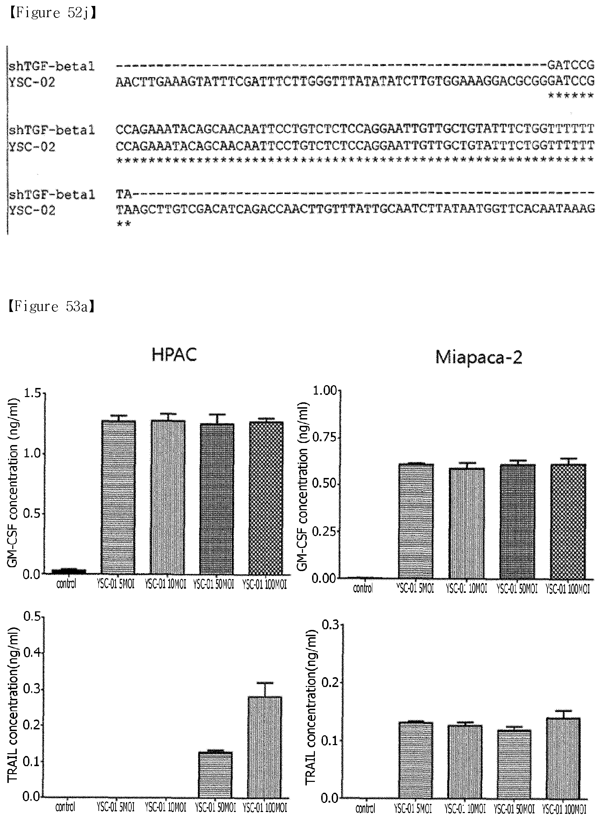

Normal insertion of four foreign genes into a virus was confirmed from viral DNA of YSC-01 and YSC-02 through sequencing of the viral DNA (FIG. 52).

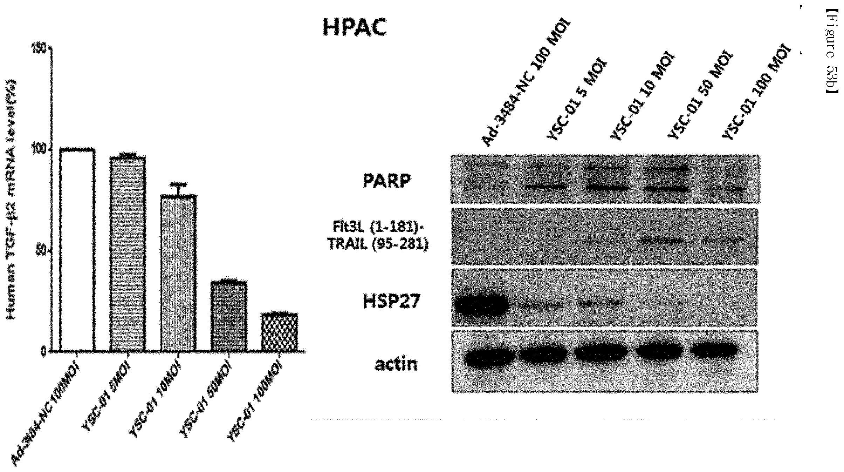

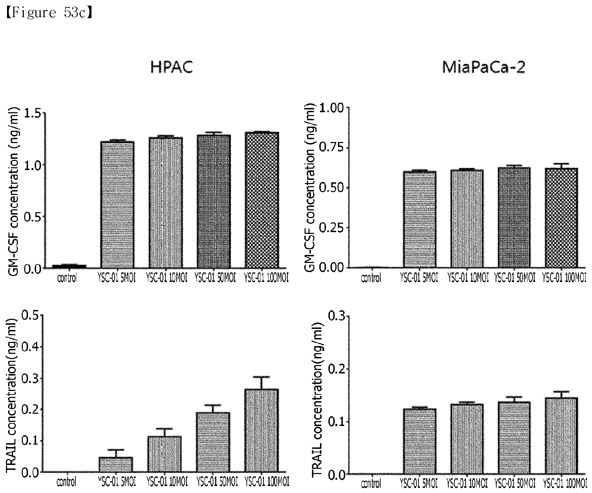

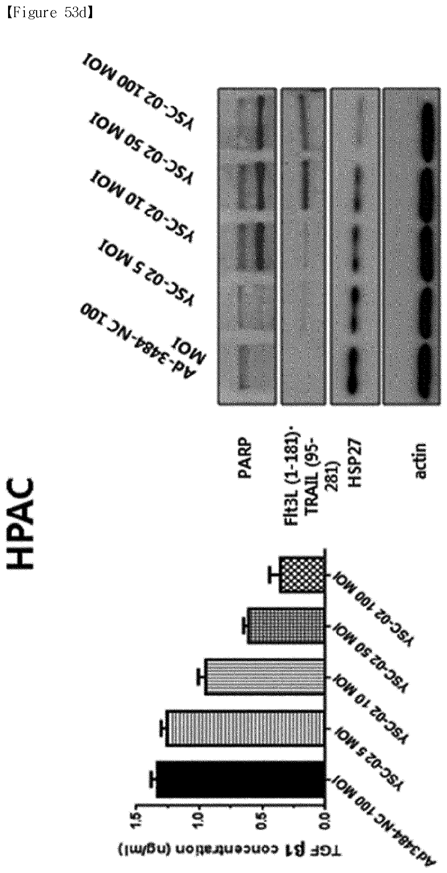

Pancreatic cancer cells HPACs and MiaPaCa-2 were seeded at 1x10⁵ cells/well of 6 well plate, infected with Ad-3484-NC (negative control oncolytic adenovirus) at 100 MOI, and infected with each of YSC-01 and 02 at 5, 10, 50 or 100 MOI, followed by culturing for 2 days, and then for the last 24 hours, levels of GM-CSF and TRAIL, which had been secreted into a serum-free medium, were measured. Secretion of GM-CSF, TRAIL and TGF-β1 was confirmed by ELISA, a decrease in mRNA of TGF-β2 was confirmed by real-time PCR, and Flt3L expression in F1t3L-TRAIL and a decrease in HSP27 expression were confirmed by western blotting (FIG. 53). Real time PCR (RT-PCR) was performed by dispensing the cells at 1x10⁵ cells/well of 6 well plate, infecting the cells with Ad-3484-NC at 100 MOI and with YSC-01 at 5, 10, 50 or 100 MOI, and isolating RNA two days after the infection. Primers for RT PCR to confirm the inhibition of human TGF-β2 were 5'-GCTGCCTACGTCCACTTTACAT-3' (SEQ ID NO: 27) as a forward primer and 5'-ATATAAGCTCAGGACCCTGCTG-3' (SEQ ID NO: 28) as a reverse primer. The RT PCR was carried out by mixing 0.2 µl of an RT enzyme mix (125X), 12.5 µl of RT-PCR Mix(2x), 0.5 µl of the forward primer (100 pM), 0.5 µl of the reverse primer (100 pM), 5 µl of RNA (10 ng/ µl), and 6.3 µl of nuclease-free water to a total volume of 25 µl using an AB power SYBR Green RNA-to-Ct 1 step kit, and repeating 40 cycles under the following reaction conditions: 10 min at 95 °C for enzyme activation; 15 sec at 95 °C for denaturation; and 1 min at 60 °C for annealing/extension.

### Example 6: In vitro tumor-selective anticancer activity confirmed through clonogenic assay for Ad-3484-GM-CSF-Flt3LTRAIL, YSC-01 and YSC-02

Clonogenic assays were performed for some cell lines including p53 mutant types such as MDA-MB231-Her2, MiaPaCa-2, A549, Huh7 and DU145, p53 normal-type cancer cell lines such as A375 and HPAC, normal cells lines such as pancreatic normal cells and Chang to confirm a survival rate.

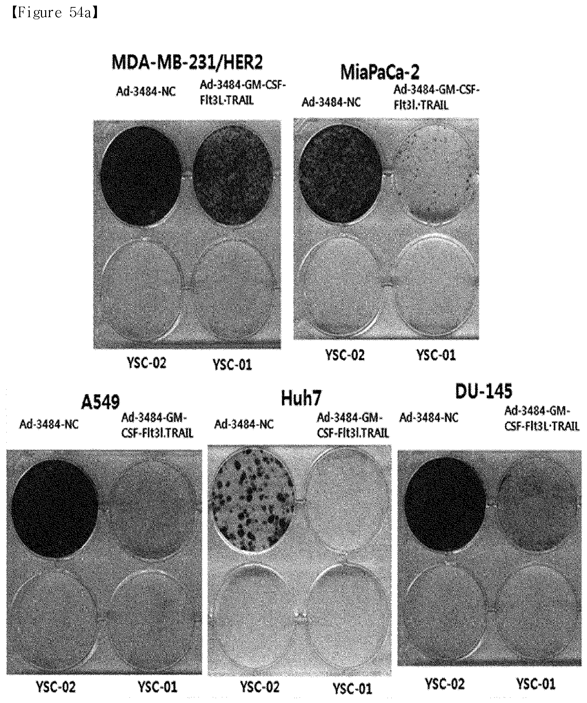

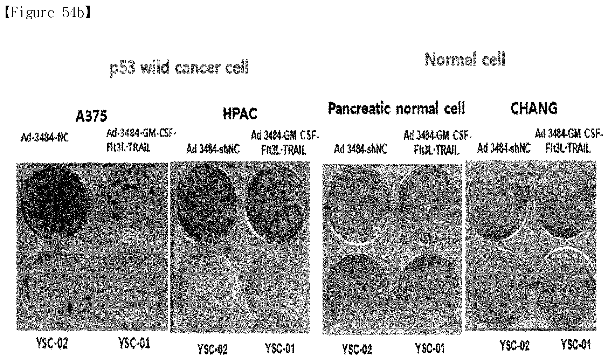

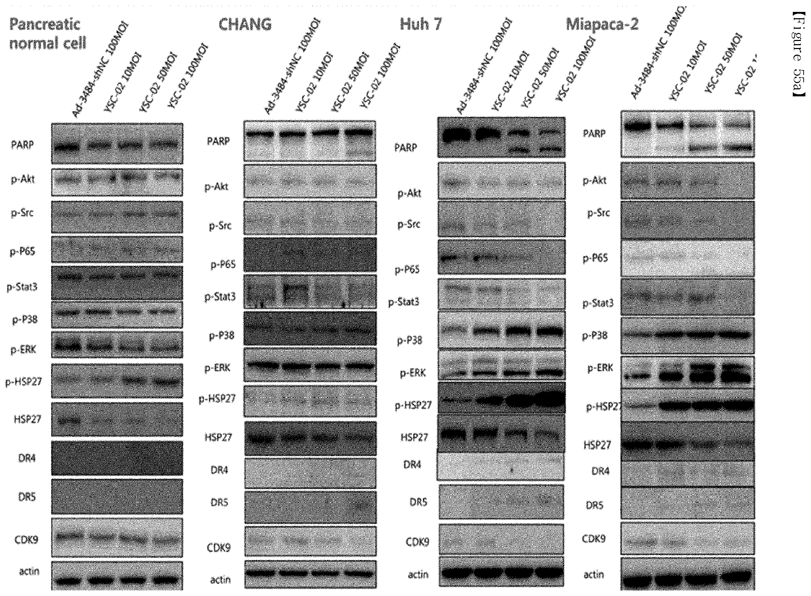

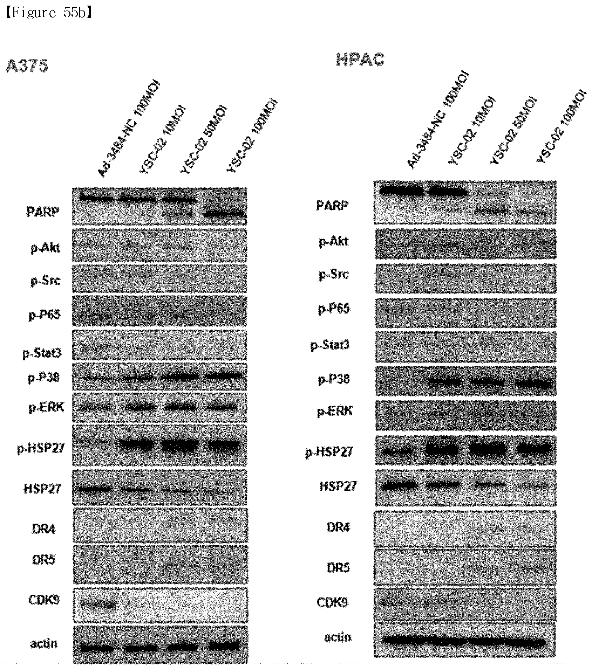

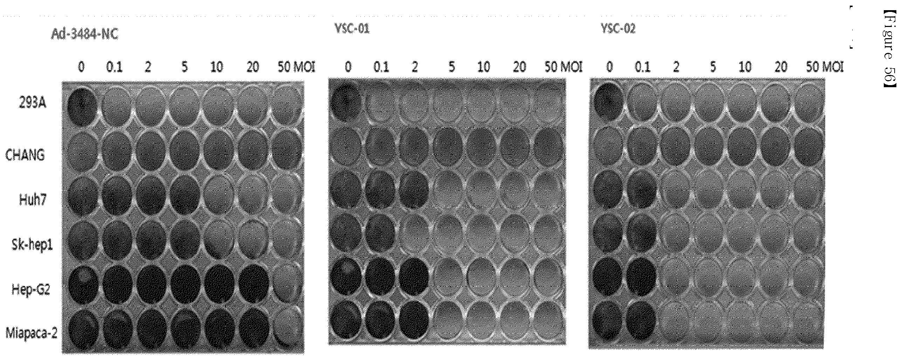

For an experiment, 4×10⁵ cells of each cell line were plated into a 60 mm dish, and the following day, infected with 1 MOI of Ad-3484-NC (negative control, oncolytic adenovirus), 1 MOI of Ad-3484-GM-CSF-Flt3L-TRAIL of Preparation Example 6, or 1 MOI of Ad 3484-GM-CSF-Flt3L-TRAIL-shTGFβ2-shHSP27 (YSC-01) of Preparation Example 19. The medium was replaced with 5% FBS-containing DMEM. The next day, the cells were dispensed into a 6-well plate at 1x10⁵ cells per well, and cultured until colonies were formed while the medium was changed every two days. At the suitable point of time in which colonies are formed (within 10 days), the medium was removed, the cells were fixed through treatment of 4% paraformaldehyde for 15 minutes at room temperature, stained with 0.05% crystal violet for 1 hour, and washed with water, followed by observing colonies stained in purple. As a result, it was confirmed through a clonogenic assay that YSC-01 and YSC-02 further degrade survival potential in a p53 normal-type cell line as well as a p53 mutant-type cell line, compared to the oncolytic adenovirus expressing GMCSF-Flt3L-TRAIL, described in Preparation Example 6 (FIG. 54A). However, since there was no effect of reducing survival potential in the normal cells, it was confirmed that tumor selectivity was also present (FIG. 54B). To confirm these experimental results, western blotting was performed, and as a result, compared to the normal cell lines, the oncolytic potential of YSC-02 was confirmed from a decrease in survival-related signals, and an increase (DR4 and DR5) or decrease (CDK9) in signals involved in TRAIL sensitivity regardless of a p53 type (FIGS. 55A and B). Moreover, as a result of an oncolytic assay for some types of hepatocellular carcinoma cell lines and pancreatic cancer cell lines (MiaPaCa-2), it was confirmed that the oncolytic activity induced by YSC-02 is relatively superior to that induced by YSC-01 (FIG. 56).

### Example 7: Antitumor effect induced by tumor-selective adenovirus coexpressing four types of genes in in vivo nude mouse

8 x 10⁶ cells/100 µl of cells of an MiaPaCa-2 cell line were injected into an abdominal wall, and infected through intratumoral injection of each of an adenovirus (negative control group: Ad-3484-NC), two types of Preparation Example 6 (Ad-3484-GM-CSF-Flt3L-TRAIL), and four types of Preparation Example 19 (Ad-3484-GM-CSF-Flt3L-TRAIL-shTGFβ2-shHSP27 (YSC-01) and Ad-3484-GM-CSF-Flt3L-TRAIL-shHSP27-shTGFβ1 (YSC-02)) three times every two days (1x10⁹pfu/50 µl) when a tumor size reached 60 mm³. Six mice per experimental group were used.

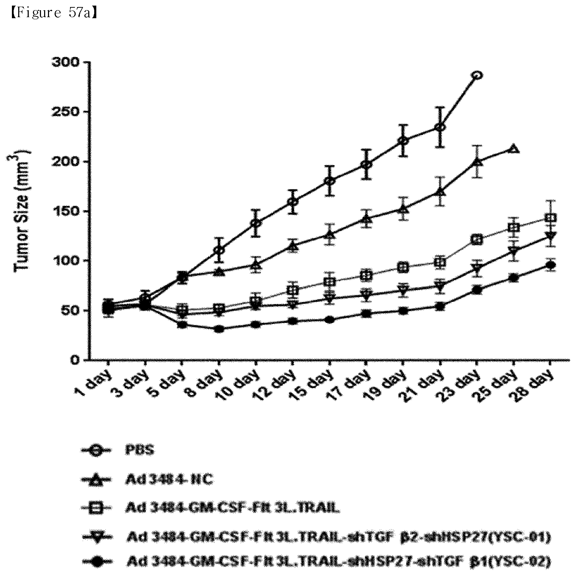

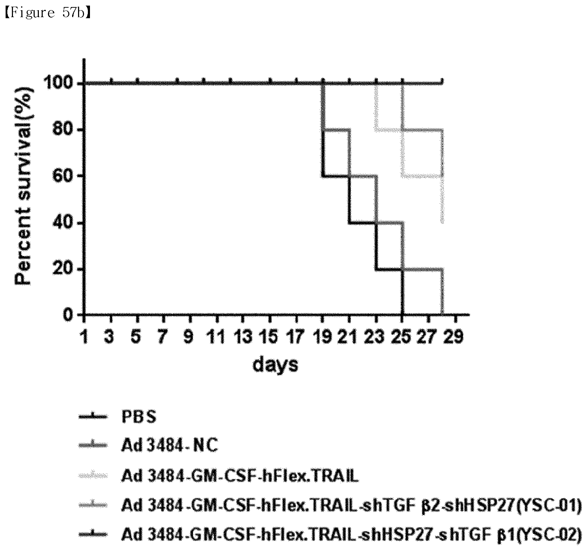

As a result, the four genes-inserted YSC-02 of Preparation Example 19 exhibited the highest antitumor effect, and the YSC-01 of Preparation Example 19 exhibited the second highest antitumor effect (FIG. 57A). In addition, the survival rate of a mouse was also highest in the case of YSC-02 (FIG. 57B).

### Example 8: Confirmation of antitumor effect induced by tumor-selective adenovirus coexpressing four types of genes in mouse with in vivo immunity

To compare the antitumor effect by murine-type YSC-02 with several comparative groups, an immunocompetent mouse model was first prepared. That is, a murine hepatocellular carcinoma cell line (BNL-CAR-E1B55K-HSP25) capable of infecting and replicating an adenovirus in an immunocompetent mouse (Balb/c) was constructed.

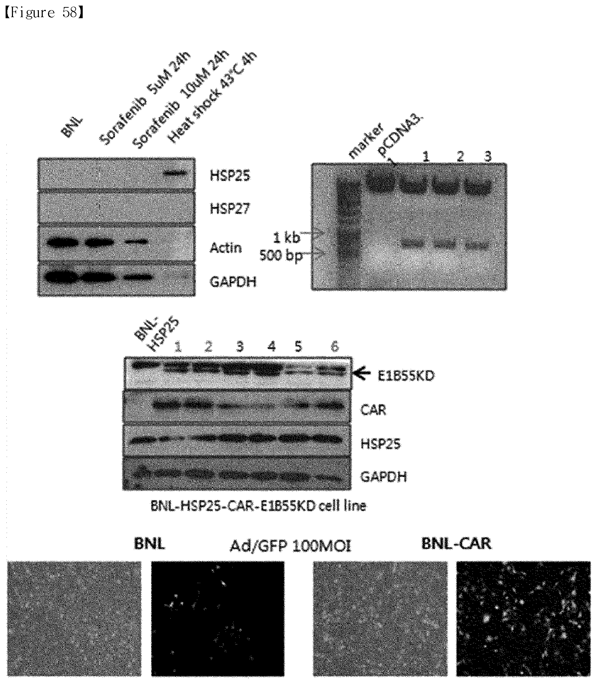

To this end, first, heat shock was applied to a BNL 1ME A.7R.1 (BNL) murine hepatocellular carcinoma cell line for 4 hours at 43°C, and the cells were cultured for 24 hours at 37°C, and then an experiment to confirm HSP25 and HSP27 expression was performed. Afterward, mRNA was extracted to synthesize cDNA, and PCR was performed using PCR primers to measure a size, followed by cloning. The primers used herein include mouse HSP25 sense :(Bam HI) 5'-cgc ggatcc atg acc gag cgc cgc gtg cc-3' (SEQ ID NO: 29) and anti-sense: (XhoI) 5'-ccg ctcgag ctacttggctccagactgtt-3' (SEQ ID NO: 30). The cloned DNA fragment was cleaved with BamHI/XhoI and inserted into pCDNA3.1 cleaved with BamHI/XhoI, thereby obtaining pcDNA3.1-HSP25. BNL cells were transfected with pcDNA3.1-HSP25, and selection was carried out using hygromycin B (250 µg/ml) to obtain HSP25-expressing clones. To produce an E1B55KD or CAR-expressing retrovirus, a pLNCX vector was used. For E1B55KD cloning, by adding HpaI and ClaI enzyme sites, E1B55KD was extracted from pcDNA3.1-E1B55KD by PCR and inserted into a pLNCX vector. For CAR cloning, by adding HindIII and ClaI enzyme sites, CAR was extracted from pCDNA3.1-CAR by PCR and inserted into a pLNCX vector. The selected BNL-HSP25 cells were coinfected with a retrovirus expressing CAR or E1B55KD, and then selected using G418 (1 mg/ml), thereby obtaining a BNL-CAR-Elb55KD-HSP25 cell line (FIG. 58).

1 x 107 cells/100 µl of cells of the BNL-CAR-E1B55KD-HSP25 cell line were injected into an abdominal wall, and when a tumor size reached 60 mm³, infected with an adenovirus (negative control group: Ad-3484-NC), one type of Preparation Example 1 (Ad-3484-mGM-CSF), one type of Preparation Example 2 (Ad-3484-Flt3L-TRAIL), two types of Preparation Example 6 (Ad-3484-mGMCSF-Flt3L-TRAIL), three types of Preparation Example 12 and Preparation Example 14 (Ad-3484-mGMCSF-Flt3L-TRAIL-shHSP25, and Ad-3484-mGMCSF-Flt3L-TRAIL-mshTGFβ1), four types of Preparation Example 20 (Ad-3484-mGMCSF-Flt3L-TRAIL-shHSP25-mshTGFβ1) and murine-type oncolytic virus 4m (GMCSF+DCN+shFoxp3+shTGFβ2) of Preparation Example 9 [hereinafter, referred to as GX-03] disclosed in Korean Unexamined Patent Application Publication No. 2015-0044507 as a comparative control group through intratumoral injection three times every two days (1x10⁹ pfu/50 µl). Six mice per experimental group were used.

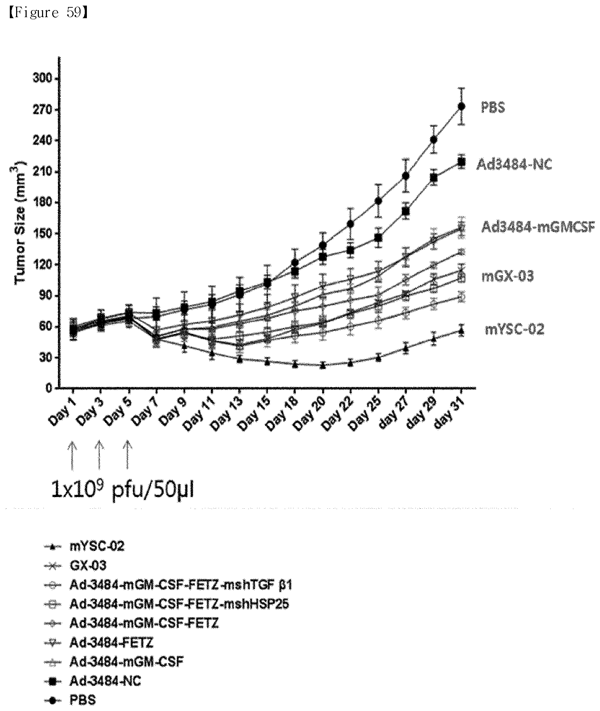

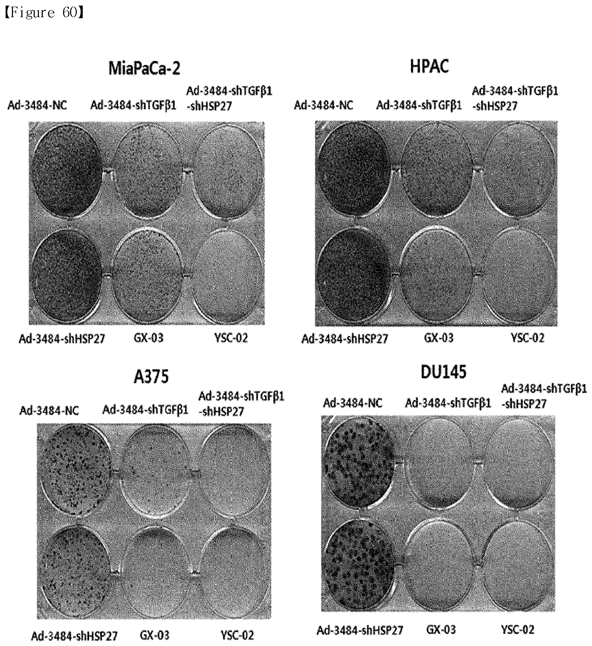

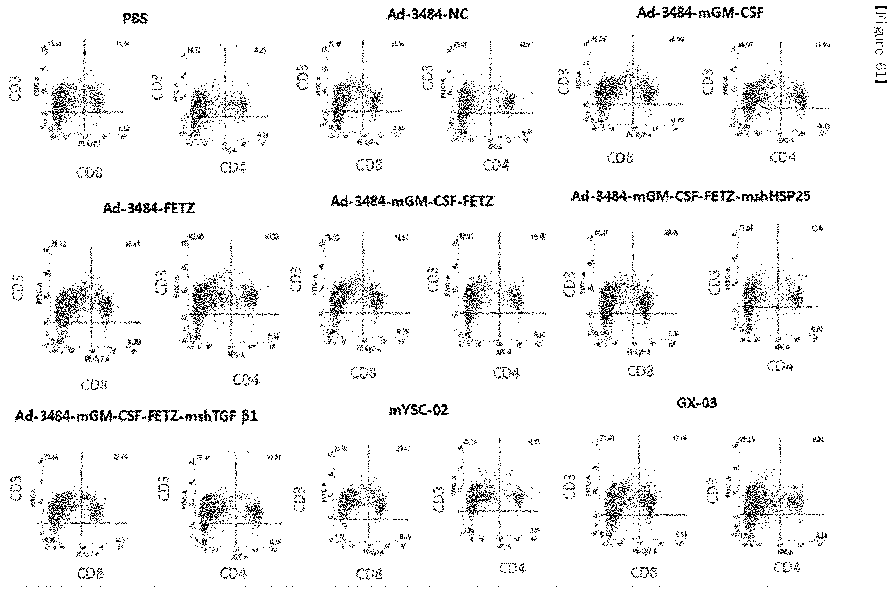

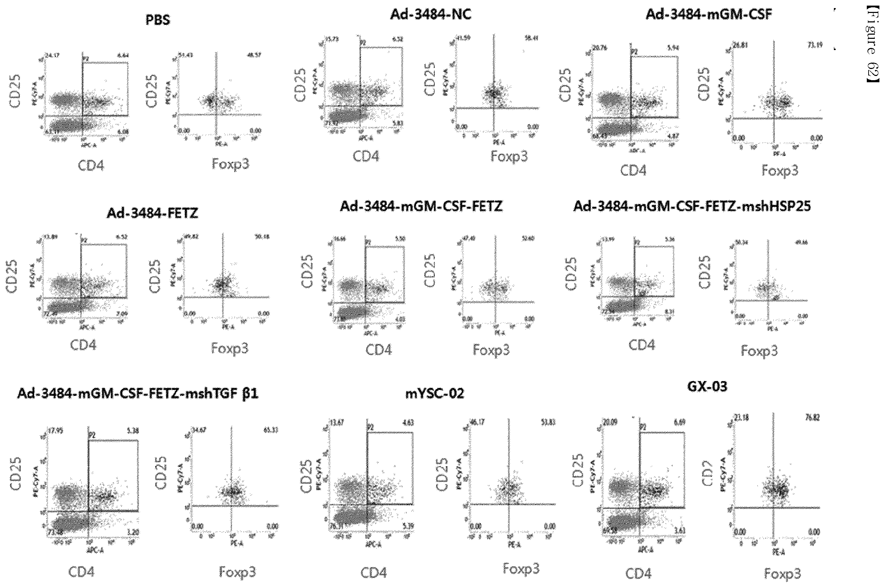

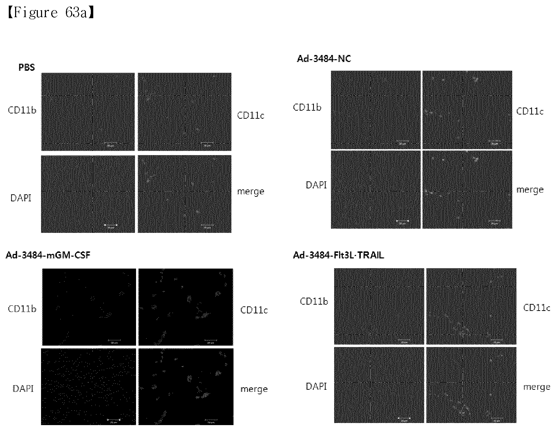

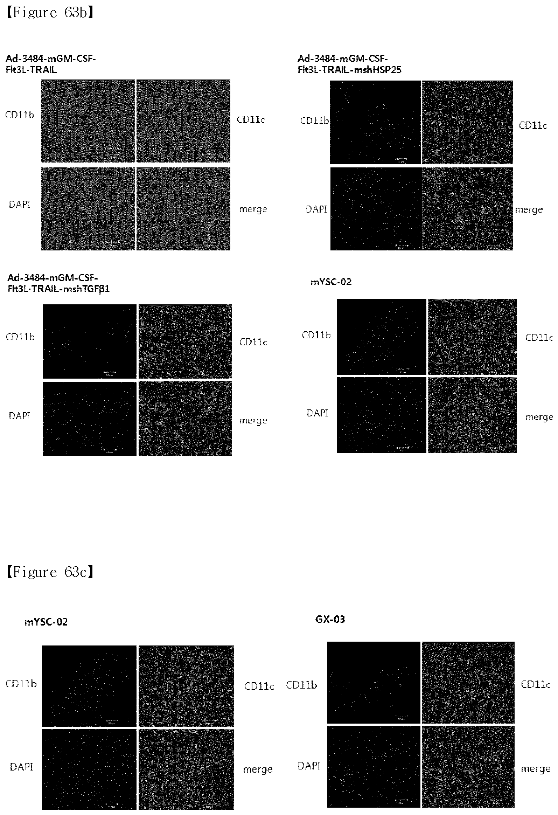

As a result, murine type YSC-02 including 4 types of genes exhibited the highest antitumor effect, and an adenovirus having three types of genes containing TGF-β1 shRNA exhibited the second highest antitumor effect, which was higher than GX-03 (FIG. 61). GX-03 exhibited a lower antitumor effect than three other types. Therefore, it was proved that YSC-02 was considerably superior to GX-03. In addition, YSC-02 exhibited a much higher antitumor effect than GX-03 as well as GM-CSF alone or F1t3L-TRAIL alone (FIG. 59) and exhibited a relatively very high effect of reducing apoptotic and survival potentials in vitro, compared to GX-03 as well as shTGF-β or shHSP27 alone and a shTGFβ-shHSP27 complex (FIG. 60). Most of all, in FIG. 55, compared to several types of comparative viruses including GX-03, in YSC-02 infection, T cell activity was overall increased after spleen cell isolation (FIG. 61), but Treg cells were not increased (FIG. 62), and DCs infiltrated into tumor tissue were clearly increased (FIG. 63).
## Claims

1. A gene delivery system for coexpressing a granulocyte-macrophage stimulating factor (GM-CSF), Flt3L-TRAIL, shTGF-β and shHSP, comprising:
a GM-CSF gene; a F1t3L-TRAIL fusion gene; shRNA inhibiting TGF-β expression (shTGF-β) and shRNA inhibiting HSP expression (shHSP).

2. The gene delivery system of claim 1, wherein the GM-CSF gene is represented by SEQ ID NO: 1 or SEQ ID NO: 2.

3. The gene delivery system of claim 1, wherein the F1t3L-TRAIL fusion gene is represented by SEQ ID NO: 3.

4. The gene delivery system of claim 1, wherein the shTGF-β is shTGF-β1 or shTGF-

5. The gene delivery system of claim 4, wherein the shTGF-β1 is represented by SEQ ID NO: 4 or SEQ ID NO: 5 or wherein the shTGF-β2 is represented by SEQ ID NO: 6 or SEQ ID NO: 7.

6. The gene delivery system of claim 1, wherein the shHSP is shHSP25 or shHSP27, preferably wherein the shHSP25 is represented by SEQ ID NO: 8 or preferably wherein the shHSP27 is represented by SEQ ID NO: 9.

7. The gene delivery system of claim 1, wherein the gene delivery system is a plasmid, a recombinant adenovirus vector, an adeno-associated virus (AAV), a retrovirus, a lentivirus, a herpes simplex virus, a vaccinia virus, a liposome or a niosome.

8. The gene delivery system of claim 7, wherein the gene delivery system is a recombinant adenovirus vector.

9. A pharmaceutical composition, which comprises the gene delivery system of any one of claims 1 to 8 for use in tumor treatment.

10. A pharmaceutical composition, which comprises a GM-CSF gene; a F1t3L-TRAIL fusion gene; shRNA inhibiting TGF-β expression (shTGF-β) and shRNA (shHSP) inhibiting HSP expression for use in tumor treatment, preferably wherein the GM-CSF gene is represented by SEQ ID NO: 1 or SEQ ID NO: 2.

11. The pharmaceutical composition of claim 10, wherein the F1t3L-TRAIL fusion gene is represented by SEQ ID NO: 3.

12. The pharmaceutical composition of claim 10, wherein the shTGF-β is shTGF-β1 or shTGF-β2, preferably wherein the shTGF-β1 is represented by SEQ ID NO: 4 or SEQ ID NO: 5 or preferably wherein the shTGF-β2 is represented by SEQ ID NO: 6 or SEQ ID NO: 7.

13. The pharmaceutical composition of claim 10, wherein the shHSP is shHSP25 or shHSP27, preferably wherein the shHSP25 is represented by SEQ ID NO: 8 or preferably wherein the shHSP27 is represented by SEQ ID NO: 9.

14. The pharmaceutical composition of claim 9 or 10, wherein the composition improves immune activity and immunogenicity.

15. The pharmaceutical composition of claim 10, wherein the gene or shRNA is introduced by one or more gene delivery systems.

## Patentansprüche

1. Ein Genverabreichungssystem zur Coexpression eines Granulozyten-Makrophagenstimulierenden Faktors (GM-CSF), Flt3L-TRAIL, shTGF-β und shHSP, umfassend:
ein GM-CSF-Gen; ein Flt3L-TRAIL-Fusionsgen; eine shRNA, die die TGF-β-Expression hemmt (shTGF-β), und eine shRNA, die die HSP-Expression hemmt (shHSP).

2. Das Genverabreichungssystem nach Anspruch 1, wobei das GM-CSF-Gen durch SEQ ID NO: 1 oder SEQ ID NO: 2 dargestellt wird.

3. Das Genverabreichungssystem nach Anspruch 1, wobei das F1t3L-TRAIL Fusionsgen durch SEQ ID NO: 3 dargestellt wird.

4. Das Genverabreichungssystem nach Anspruch 1, wobei das shTGF-β shTGF-β1 oder shTGF-β2 ist.

5. Das Genverabreichungssystem nach Anspruch 4, wobei das shTGF-β1 durch SEQ ID NO: 4 oder SEQ ID NO: 5 dargestellt wird oder wobei das shTGF-β2 durch SEQ ID NO: 6 oder SEQ ID NO: 7 dargestellt wird.

6. Das Genverabreichungssystem nach Anspruch 1, wobei das shHSP shHSP25 oder shHSP27 ist, vorzugsweise wobei shHSP25 durch SEQ ID NO: 8 dargestellt ist oder vorzugsweise wobei shHSP27 durch SEQ ID NO: 9 dargestellt ist.

7. Das Genverabreichungssystem nach Anspruch 1, wobei das Genverabreichungssystem ein Plasmid, ein rekombinanter Adenovirus-Vektor, ein Adeno-assoziiertes Virus (AAV), ein Retrovirus, ein Lentivirus, ein Herpes-simplex-Virus, ein Vacciniavirus, ein Liposom oder ein Niosom ist.

8. Das Genverabreichungssystem nach Anspruch 7, wobei das Genverabreichungssystem ein rekombinanter Adenovirus-Vektor ist.

9. Eine pharmazeutische Zusammensetzung, die das Genverabreichungssystem nach irgendeinem der Ansprüche 1 bis 8 zur Verwendung bei der Tumorbehandlung umfasst.

10. Eine pharmazeutische Zusammensetzung, umfassend ein GM-CSF-Gen; ein Flt3L-TRAIL-Fusionsgen; eine shRNA, die die TGF-β-Expression hemmt (shTGF-β) und eine shRNA (shHSP), die die HSP-Expression zur Verwendung in der Tumorbehandlung hemmt, wobei das GM-CSF-Gen vorzugsweise durch SEQ ID NO: 1 oder SEQ ID NO: 2 dargestellt wird.

11. Die pharmazeutische Zusammensetzung nach Anspruch 10, wobei das Flt3L-TRAlL Fusionsgen durch SEQ ID NO: 3 dargestellt wird.

12. Die pharmazeutische Zusammensetzung nach Anspruch 10, wobei shTGF-β shTGF-β1 oder shTGF-β2 ist, vorzugsweise wobei shTGF-β1 durch SEQ ID NO: 4 oder SEQ ID NO: 5 dargestellt ist oder vorzugsweise wobei shTGF-β2 durch SEQ ID NO: 6 oder SEQ ID NO: 7 dargestellt ist.

13. Die pharmazeutische Zusammensetzung nach Anspruch 10, wobei shHSP shHSP25 oder shHSP27 ist, vorzugsweise wobei shHSP25 durch SEQ ID NO: 8 dargestellt ist oder vorzugsweise wobei die shHSP27 durch SEQ ID NO: 9 dargestellt ist.

14. Die pharmazeutische Zusammensetzung nach Anspruch 9 oder 10, wobei die Zusammensetzung die Immunaktivität und Immunogenität verbessert.

15. Die pharmazeutische Zusammensetzung nach Anspruch 10, wobei das Gen oder die shRNA durch ein oder mehrere Genverabreichungssysteme eingeführt wird.

## Revendications

1. Système de délivrance de gènes pour la coexpression d'un facteur stimulant les granulocytes et les macrophages (GM-CSF), Flt3L-TRAIL, shTGF-β et shHSP, comprenant:
un gène GM-CSF;
un gène de fusion Flt3L-TRAIL;
un ARNsi inhibant l'expression du TGF-β (shTGF-β) et un ARNsi inhibant l'expression de la HSP (shHSP).

2. Système de transfert de gènes selon la revendication 1, dans lequel le gène du PPC génétiquement modifié est représenté par la SEQ ID n° 1 ou la SEQ ID n° 2.

3. Système de transfert de gènes selon la revendication 1, dans lequel le gène de fusion Flt3L-TRAIL est représenté par la SEQ ID NO: 3.

4. Système de transfert de gènes selon la revendication 1, dans lequel le shTGF-β est shTGF-β1 ou shTGF-β2.

5. Système de transfert de gènes selon la revendication 4, dans lequel le shTGF-β1 est représenté par la SEQ ID NO: 4 ou la SEQ ID NO: 5 ou dans lequel le shTGF-β2 est représenté par la SEQ ID NO: 6 ou la SEQ ID NO: 7.

6. Système de transfert de gènes selon la revendication 1, dans lequel le shHSP est shHSP25 ou shHSP27, de préférence dans lequel le shHSP25 est représenté par la SEQ ID NO: 8 ou de préférence dans lequel le shHSP27 est représenté par la SEQ ID NO: 9.

7. Système de transfert de gènes selon la revendication 1, dans lequel le système de transfert de gènes est un plasmide, un vecteur adénoviral recombinant, un virus adéno-associé (AAV), un rétrovirus, un lentivirus, un virus herpès simplex, un virus de la vaccine, un liposome ou un niosome.

8. Système de transfert de gènes selon la revendication 7, dans lequel le système de transfert de gènes est un vecteur adénovirus recombinant.

9. Composition pharmaceutique, qui comprend le système de délivrance de gènes de l'une quelconque des revendications 1 à 8, pour utilisation dans le traitement des tumeurs.

10. Composition pharmaceutique, qui comprend un gène de GM-CSF; un gène de fusion Flt3L-TRAIL; un ARNsh inhibant l'expression de TGF-β (shTGF-β) et un ARNsh (shHSP) inhibant l'expression de HSP pour une utilisation dans le traitement des tumeurs, de préférence dans laquelle le gène de GM-CSF est représenté par SEQ ID NO: 1 ou SEQ ID NO: 2.

11. Composition pharmaceutique selon la revendication 10, dans laquelle le gène de fusion Flt3L-TRAIL est représenté par la SEQ ID NO: 3.

12. Composition pharmaceutique selon la revendication 10, dans laquelle le shTGF-β est shTGF-β1 ou shTGF-β2, de préférence dans laquelle le shTGF-β1 est représenté par la SEQ ID NO: 4 ou la SEQ ID NO: 5 ou de préférence dans laquelle le shTGF-β2 est représenté par la SEQ ID NO: 6 ou la SEQ ID NO: 7.

13. Composition pharmaceutique selon la revendication 10, dans laquelle le shHSP est shHSP25 ou shHSP27, de préférence dans laquelle le shHSP25 est représenté par la SEQ ID NO: 8 ou de préférence dans laquelle le shHSP27 est représenté par la SEQ ID NO: 9.

14. Composition pharmaceutique selon les revendications 9 ou 10, dans laquelle la composition améliore l'activité immunitaire et l'immunogénicité.

15. Composition pharmaceutique selon la revendication 10, dans laquelle le gène ou l'ARNsh est introduit par un ou plusieurs systèmes de livraison de gènes.
